# EUROPEAN PATENT SPECIFICATION

(11) **EP 1 268 842 B1**
(45) Date of publication and mention of the grant of the patent: **21.06.2006**
(21) Application number: 01918516.4
(22) Date of filing: 08.03.2001
(51) Int. Cl.: C12Q 1/00, C12Q 1/02, C12Q 1/68, C12N 1/14, C12N 1/16, C12N 1/18

(54) **IMPROVED REVERSE N-HYBRID SCREENING METHOD**
VERBESSERTES VERFAHREN FÜR REVERSES N-HYBRID-SCREENING
METHODE DE CRIBLAGE INVERSE A N HYBRIDES AMELIOREE

(30) Priority: 08.03.2000 AU PQ613100; 23.03.2000 AU PQ643700; 11.04.2000 AU PQ683000; 06.11.2000 AU PR125600
(43) Date of publication of application: 02.01.2003
(73) Proprietor: Phylogica Limited, Northbridge, W.A. 6003 (AU)
(72) Inventor: HOPKINS, Richard, North Perth, W.A. 6006 (AU); SEREBRIISKII, Ilya, Philadelphia, PA 19111 (US); WATT, Paul, Michael, Mount Claremont, W.A. 6010 (AU); GOLEMIS, Erica, Oreland, PA 19075 (US)
(74) Representative: Dzieglewska, Hanna Eva
(86) International application number: PCT/US2001/007669
(87) International publication number: WO 2001/066787

(56) References cited:
- WO-A-97/38127
- WO-A-99/14319
- WO-A1-99/14319
- WO-A1-99/35282
- US-A- 5 965 368
- LICITRA E J ET AL: "A THREE-HYBRID SYSTEM FOR DETECTING SMALL LIGAND-PROTEIN RECEPTOR INTERACTIONS" PROCEEDINGS OF THE NATIONAL ACADEMY OF SCIENCES OF USA, NATIONAL ACADEMY OF SCIENCE. WASHINGTON, US, vol. 93, 1 November 1996 (1996-11-01), pages 12817-12821, XP002038709 ISSN: 0027-8424

## Description

### FIELD OF THE INVENTION

The present invention relates generally to an improved reverse n-hybrid screening method for identifying antagonists or inhibitors of biological interactions, wherein multiple reporter genes are employed to distinguish antagonists or inhibitors of one interaction from those antagonists or inhibitors of other interactions, such as, for example, wherein each of said interactions involves one or more identical interacting partners. The present invention provides the means by which a wide range of peptide-based therapeutic, prophylactic and diagnostic reagents may be developed.

### GENERAL

Those skilled in the art will be aware that the invention described herein is subject to variations and modifications other than those specifically described. It is to be understood that the invention described herein includes all such variations and modifications. The invention also includes all such steps, features, compositions and compounds referred to or indicated in this specification, individually or collectively, and any and all combinations of any two or more of said steps or features.

Throughout this specification, unless the context requires otherwise the word "comprise", and variations such as "comprises" and "comprising", will be understood to imply the inclusion of a stated integer or step or group of integers or steps but not the exclusion of any other integer or step or group of integers or steps. The present invention is not to be limited in scope by the specific embodiments described herein, which are intended for the purposes of exemplification only. Functionally-equivalent products, compositions and methods are clearly within the scope of the invention, as described herein.

Bibliographic details of the publications referred to by author in this specification are collected at the end of the description. Reference herein to prior art, including any one or more prior art documents, is not to be taken as an acknowledgment, or suggestion, that said prior art is common general knowledge in Australia or forms a part of the common general knowledge in Australia.

As used herein, the term "derived from" shall be taken to indicate that a particular integer or group of integers has originated from the species specified, but has not necessarily been obtained directly from the specified source.

This specification contains nucleotide sequence information prepared using the program Patentln Version 3.0, presented herein after the claims. Each nucleotide sequence is identified in the sequence listing by the numeric indicator <210> followed by the sequence identifier [e.g. <210>1, <210>2, etc]. The length, type of sequence [DNA, protein (PRT), etc] and source organism for each nucleotide sequence are indicated by information provided in the numeric indicator fields <211>, <212> and <213>, respectively. Nucleotide sequences referred to in the specification are defined by the term "SEQ ID NO:", followed by the sequence identifier [e.g. SEQ ID NO: 1 refers to the sequence in the sequence listing designated as <400>1].

The designation of nucleotide residues referred to herein are those recommended by the IUPAC-IUB Biochemical Nomenclature Commission, wherein A represents Adenine, C represents Cytosine, G represents Guanine, T represents thymidine, Y represents a pyrimidine residue, R represents a purine residue, M represents Adenine or Cytosine, K represents Guanine or Thymidine, S represents Guanine or Cytosine, W represents Adenine or Thymidine, H represents a nucleotide other than Guanine, B represents a nucleotide other than Adenine, V represents a nucleobde other than Thymidine, D represents a nucleotide other than Cytosine and N represents any nucleotide residue.

### BACKGROUND OF THE INVENTION

Biological interactions, such as, for example, a protein-protein interaction or protein-nucleic interaction, are involved in a wide variety of processes occurring in living cells. For example, agonism or antagonism of a receptor by a specific ligand, such as, for example, a drug, hormone, second messenger molecule, etc., may effect one or more of a variety of biological processes, including gene expression, cellular differentiation, growth, enzyme activity, metabolite flow, or metabolite partitioning between cellular compartments. Protein-protein interactions, DNA-protein interactions, and RNA-protein interactions, are well known for their effects in regulating such biological processes in both prokaryotic and eukaryotic cells, in addition to being critical for DNA replication, and, in the case of RNA viruses, RNA replication.

Undesirable or inappropriate gene expression and/or cellular differentiation, cellular growth and metabolism may be attributable, at least in many cases, to biological interactions involving the binding and/or activity of proteinaceous molecules, such as, for example, transcription factors, peptide hormones, receptor molecules, or enzymes. In one example, several genes activated by chromosomal translocations in lymphoid malignancies code for transcription factors, for example MYC, LYL-1 and SCL, which appear to function via protein-protein interactions. In normal cells, these proteins are in an appropriate equilibrium with their interaction partners which is disturbed as a consequence of oncogene activation and is thought to result in transcription of target genes normally expressed in other cells or lineages. These transcription factors may also substitute for, or antagonize, the function of closely related endogenous proteins to perturb gene expression essential for normal growth control.

Peptides present potential therapeutic and prophylactic agents for many human and animal diseases, biochemical disorders and adverse drug effects, because they can interact with other molecules highly specifically. For example, mimetic peptides have been reported to inhibit protein interactions and/or enzymatic functions. More specific examples include a nonapeptide derived from the ribonucleotide reductase of herpes simplex virus linked to an enterotoxin subunit for delivery into cells via its receptor. The peptide conjugate is found to inhibit herpes simplex type I replication in quiescent Vero cells (Marcello *et al*. 1994). Using detailed knowledge of the PCNA-interaction domain of p21^{WAF1}, a peptide was designed which effectively blocked the interaction. This 20-mer peptide bound with sufficient affinity to block SV40 replication (Warbrick *et al.* 1996). A 20-mer peptide sequence derived from p16 was found to interact with cdk4 and cdk6 and inhibited pRB phosphorylation and cell cycle progression (Fahraeus 1996). Peptides have even been shown to function as inhibitors in animal models. For examples, a peptide targeting the ICE protease was shown to be a potent protective inhibitor against liver apoptosis induced by TNF-α in the mouse (Rouquet et *al.* 1996). RNA-protein interactions, such as, for example, the TAT/TAR interaction of HIV, or the interaction of RNA with the protein subunits of telomerase, present attractive targets for therapy.

Conventional forward n-hybrid screens have proven useful for identifying proteins that specifically interact with a target protein of interest in a cell. In such assays, the protein of interest is generally expressed as a fusion protein with the DNA binding domain (DBD) of a transcription factor, and a transcription activator domain (AD) is expressed separately as a fusion with each member of an array of peptides. When the appropriate association between binding partners occurs in the cell, a functional transcription factor is reconstituted, and expression of a reporter gene placed under the control of the reconstituted transcription factor occurs. The cell expressing the appropriate binding partner can then be isolated, facilitating identification of the binding partner. Significantly, the selection system used is a positive selection, wherein expression of the reporter gene is enhanced, by the interaction between the binding partners.

Conversely, reverse n-hybrid screening technologies provide a direct means of isolating antagonists or "peptide inhibitors" of a given RNA-protein, DNA-protein, or protein-protein interaction. Such screens differ from canonical forward n-hybrid screens, by providing a selection against the interaction. This is achieved, for example, by utilizing a counter selectable reporter gene that encodes a lethal product when expressed in the cell, or alternatively, encodes an enzyme that converts a non-toxic substrate to a toxic product. Counter selectable reporter genes suitable for this purposes include, for example, the yeast URA3 structural gene which is lethal to yeast cells when expressed in the presence of 5-fluororotic acid (5-FOA); the yeast *CYH2* gene which is lethal when expressed in the presence of the drug cycloheximide; and the yeast *LYS2* gene which is lethal in the presence of the drug α-aminoadipate (α-AA). Reverse n-hybrid screens employing such counter selectable reporter genes are described in detail in Intemational Patent Application No. PCT/AU99100018 (WO 99/35282), which particularly describes a high-throughput reverse n-hybrid screening method for screening random peptide (i.e. aptamer) libraries *in vivo.*

The utility of reverse two hybrid approaches for high-throughput screening of small molecule drug libraries has also been described recently by Huang and Schreiber (1997) and by Young *et al*.(1998), albeit not with respect to the screening of aptamer libraries.

Many distinct biological interactions in complex organisms employ overlapping subsets of interacting partners, or partners that are organized into large protein families that are highly conserved at the amino acid sequence level, particularly in regions that are required for protein binding activity or nucleic acid binding activity. For example, the GTPases Ras and Krev-1 are 56% identical and are known to interact with an overlapping set of protein partners, albeit at different affinities, namely, Raf to which Ras preferentially binds, Krit 1 to which Krev 1 preferentially binds, and the Rai guanine dissociation stimulator protein (RaIGDS), to which both proteins bind (Serebdiskii et a1.1999). Similarly, the transcription factor SCL, which is expressed in malignant lymphoid cells, interacts with LM01, LM02, DRG, mSin3A, and E47 proteins (Mahajan et a/,1996).

Despite the utility of reverse n-hybrid screens for identifying potentially useful therapeutic molecules that antagonize a simple interaction in a cell, such screens may not be capable of distinguishing between multiple interactions in the cell that involve binding partners having the same or closely related partner-binding domains, such as those described supra. Known reverse two hybrid or reverse three hybrid screens do not provide a means for preferentially selecting for one interaction between two or more binding partners over another protein-protein interaction involving one or more of the same binding partners, or closely related binding partners. Accordingly, existing technologies have the disadvantage of detecting false positives in the screening process, necessitating additional time and effort to resolve true binding partners.

To attempt to overcome problems with the identification of false positives WO99/14319 describes a method that uses multiple reporter molecules to determine the ability of a test protein to interact with a first protein but not a second protein. While the method described is useful for identifying specific protein interactions it is of little use in identifying an inhibitor (e.g., an inhibitory peptide) that distinguishes between multiple interactions (i.e. a selective inhibitor).

US 5,965,368 describes a similar method for identifying a protein capable of interacting with a second protein but not with a third protein. However, in this case the described method uses a counter-selectable marker. The described method specifically selects against interactions involving multiple binding partners making it unsuitable for determining an inhibitor that distinguishes between multiple interactions.

Moreover, existing reverse two hybrid screens and reverse three hybrid screens are not ideally suited to high throughput applications due to a lack of suitable shuttle vectors or host cell strains.

### SUMMARY OF THE INVENTION

In work leading up to the present invention, the inventors sought to develop reverse n-hybrid screens having enhanced specificity in their detection of interacting binding partners compared to conventional reverse n-hybrid screens, and more particularly, having the capacity to distinguish between interactions that involve one or more common binding partners.

As used herein, the term "interaction" shall be taken to refer to a direct or indirect physical association between two or more molecules or "partrters", wherein said association is involved in a cellular process or alternatively, is required for said cellular process to occur. The "association" may involve the formation of an induced magnetic field or paramagnetic field, covalent bond formation such as, for example, a disulfide bridge formation between polypeptide molecules, an ionic interaction such as, for example, occur in an ionic lattice, a hydrogen bond or alternatively, a van der Waals interaction such as, for example, a dipole-dipole interaction, dipole-induced-dipole interaction, induced-dipole-induced-dipole interaction or a repulsive interaction or any combination of the above forces of attraction.

For example, in a reverse two hybrid assay, the interaction between the proteinaceous binding partners may involve their direct physical association (i.e. with no intervening molecule). Similarly, in a reverse three hybrid assay, the interaction between some of the binding partners, particularly the interaction between the two baits, and the interaction between one of the baits and the prey, is a direct physical association. However, in a three hybrid assay, the constant bait and the prey are clearly in indirect association with each other, because the non-constant bait (e.g. an RNA-bait in the case of an RNA/protein interaction) forms a "bridge between these partners. Accordingly, for the purposes of the present invention, the term "interaction" clearly includes within its scope such direct and indirect associations between the various binding partners, and, as a consequence, does not omit any unstated binding partners.

As used herein, the term "n-hybrid" refers to the number of binding partners, other than the peptide inhibitor, that are involved in an interaction that is assayed. Accordingly, there are two binding partners in a conventional reverse two hybrid screen and three binding partners in a conventional reverse three hybrid screen, and so on.

Accordingly, one aspect of the present invention provides a method of identifying a peptidethat partially or completely inhibits a target interaction between two or more binding partners in a host cell but does not inhibit a non-target interaction between some but not all of said binding partners, said method comprising:
(i) expressing in a cellular host: (a) the binding partners of said target interaction such that they operably control the expression of one or more reporter genes in said cellular host, wherein said expression is partially or completely inhibited by disruption of said target interaction; (b) the binding partners of said non-target interaction such that they operably control the expression of one or more reporter genes in said cellular host, wherein said expression is partially or completely inhibited by disruption of said non-target interaction and wherein said reporter gene is distinct from the reporter gene(s) expressed under control of the target interaction; and (c) a candidate peptide; wherein one or more reporter genes operably under the control of the target interaction or the non-target interaction is a counter selectable reporter gene that confers conditional lethality on the cellular host, and wherein, to determine the minimum concentration of binding partner expression required for the counter-selectable reporter gene to be expressed at a level that causes cell death, the expression of one or more of said binding partners is titrated in response to galactose or copper by placing said expression under the control of a promoter comprising regulatory elements of the *GAL1* or *CUP1* promoters;
(ii) growing the cellular host under conditions sufficient to distinguish the expression of each reporter gene(s) at (a) from expression of the reporter gene(s) at (b); and
(iii) detecting those host cells wherein expression of the reporter gene(s) operably under control of the target interaction is(are) partially or completely inhibited and expression of the reporter gene(s) operably under control of the non-target interaction is(are) not inhibited, said detected cells expressing a peptide that partially or completely inhibits the target interaction.

This aspect of the invention clearly includes within its scope both reverse two hybrid and reverse three hybrid assays, or any reverse n-hybrid assay, by virtue of the term "interaction" as defined herein above. As will be known to those skilled in the art, the only difference between the reverse two hybrid format, and higher order formats, is the addition of further binding partners that may alter the relative physical associations between each of the binding partners, however the general principal for all reverse n-hybrid assay formats is the same.

The host cell may be any cell capable of supporting the expression of exogenous DNA, such as, for example, a bacterial cell, insect cell, yeast cell, mammalian cell or plant cell. Preferably, the cell is a bacterial cell, mammalian cell, or a yeast cell. preferably, a yeast cell will have the genotype *MATα, ura3, trp1, his3, cyh2*^{*R*}*, lexAop-URA3, lexoAop-CYH2, ade2.* Such a yeast strain may be constructed using spontaneous cycloheximide-resistant derivatives of YPH252 (Sikorski *et al.,* 1989) or EGY40 (Golemis and Brent, 1992; Gyuris *et al.* 1993).

Alternatively, the yeast strain can be PRT 473, a cycloheximide resistant derivative of strain SKY 473, of the genotype *(MAT-*a*, cyh*2^{R} *his*3*, trp*1*, lexAop-LEU*2*, lexAop-CYH2ade2::ZEO*^{R}, *lexAop-URA3his5::G418*^{R}*, clop-LYS).*

By "target interaction" is meant the interaction in the cellular host against which a specific antagonist or peptide inhibitor is sought.

A "non-target interaction° means an interaction that involves at least one common binding partner as defined herein above with the target interaction, however against which a specific antagonist is not being sought. Accordingly, it is an object of the present invention to exclude those peptides that antagonize both the target interaction and the non-target interaction in the cellular host, and to preferentially select those peptides that antagonize only the target interaction in the cellular host, or that antagonize the target interaction at a higher affinity than the antagonism of the non-target interaction.

The term "partial or complete inhibition" means that expression of the reporter gene under the control of the target interaction is reduced to a level that does not interfere with the capability to distinguish between the target interaction and the non-target interaction as defined herein above.

As used herein, the term "binding partner" shall be taken to mean protein or nucleic acid that binds to another protein or nucleic acid in a cell, including a complete protein or gene, or the dimerization region of a protein, or a *cis*-acting nucleotide sequence that binds to nucleic acid or protein. As will be known to those skilled in the art, at least two binding partners are involved in any biological interaction in a cell. The term "dimerization region" means that part of a protein that is in physical relation with a binding partner. The term "*cis*-acting nucleotide sequence" shall be taken to mean a nucleotide sequence that regulates gene expression by virtue of the binding of a repressor protein, or activator protein thereto, including the minimum nucleotide sequence that has the ability to bind said repressor protein or said activator protein.

Accordingly, it shall be clear to the skilled person that any reference herein to a common binding partner includes a reference to homologous proteins or genes that share one or more dimerization regions or *cis*-acting nucleotide sequences, respectively, with a known binding partner that is involved in an interaction against which the reverse hybrid screen of the invention is directed (i.e. the target interaction).

Thus, in cases of large protein families, or large multigene families, it is an object of the present invention to select a peptide that partially or completely inhibits a target interaction between two or more binding partners in a host cell but does not inhibit a non-target interaction between some but not all of said binding partners, such as, for example, any homologues of said binding partners that share common dimerization regions or *cis*-acting sequences, or alternatively, identical binding partners that themselves interact with a number of different binding partners.

In a particularly preferred embodiment of the invention, one or more of the binding partners of the non-target interaction is the same as a binding partner of the target interaction.

The invention is not to be limited by the nature of the target interaction being assayed, or the nature of the non-target interaction, because all formats are readily determined by those skilled in the art without undue experimentation based upon the disclosure contained herein. Preferably, one or more of the binding partners is comprises amino acids, such as, for example, a peptide, polypeptide, protein or enzyme molecule, or alternatively, comprises nucleic acid, such as DNA, RNA, or a DNA/RNA hybrid. More preferably, the target and/or non-target interactions are/is a protein/protein interaction or protein/nucleic acid interaction.

Preferably, the number of binding partners involved in each interaction is at least two. In a particularly preferred embodiment, present invention provides an improved reverse two hybrid assay, wherein:
(i) the binding partners of the target interaction consist of two fusion proteins, wherein (a) one fusion protein comprises the DNA binding domain of a transcription factor and an amino acid sequence that dimerizes with the other fusion protein of the target interaction; and (b) one fusion protein comprises the transcriptional activator domain of a transcription factor and an amino acid sequence that dimerizes with the other fusion protein of the target interaction, and wherein dimerization between said fusion proteins produces a protein complex that binds to the *cis*-acting sequence and activates expression of the reporter gene(s) when the target interaction occurs in the host cell; and
(ii) the binding partners of the non-target interaction consist of two fusion proteins, wherein (a) one fusion protein comprises the DNA binding domain of a transcription factor and an amino acid sequence that dimerizes with the other fusion protein of the non-target interaction; and (b) one fusion protein comprises the transcriptional activator domain of a transcription factor and an amino acid sequence that dimerizes with the other fusion protein of the non-target interaction, and wherein dimerization between said fusion proteins produces a protein complex that binds to the *cis*-acting sequence and activates expression of the reporter gene(s) when the non-target interaction occurs in the host cell.

This embodiment of the invention has versatility in so far as the nature of the binding partners of the target interaction and the non-target interaction are interchangeable, provided that the capability for distinguishing between the target interaction and the non-target interaction is retained.

For example, the fusion proteins of the target interaction and the non-target interaction comprising a transcription activator domain can be the same, or have the same activation properties. In such a case, the fusion proteins of the target and non-target interactions comprising DNA binding domains will be different, as will the *cis*-acting sequences to which they bind. Alternatively, the proteins of the target or non-target interaction that comprise DNA binding binding domains may be the same, but fused to different DNA binding domains that bind to different *cis*-acting elements upstream of the reporter gene(s).

Alternatively, the fusion proteins of the target and non-target interactions comprising DNA binding domains can be the same, or have the same binding affinities. In such a case, the *cis*-acting sequences of the target and non-target interactions will also be the same or functionally equivalent, however the fusion proteins of the target interaction and the non-target interaction comprising a transcription activator domain will be different.

As will be known to the skilled person, reverse n-hybrid screens require the operable connection of the interaction being assayed to the expression of a reporter gene in the cell, wherein expression of the reporter is selected against to facilitate the detection of an antagonist of the interaction. The present invention develops this technology further by providing for the selection against the target interaction, concomitant with selection for or against the non-target interaction.

Particularly preferred target interactions susceptible to the inventive method described herein include those interactions between SCL or the dimerization region of SCL and protein selected from the group consisting of: LMO1. LMO2, DRG, mSin3A, E47, a dimerization region of LMO1, a dimerization region of LMO2, a dimerization region of DRG, a dimerization region of mSin3A, and a dimerization region of E47.

Alternatively, the inventive method is capable of detecting the interaction between LMO2 orthe dimerization region of LMO2 and a protein selected from the group consisting of: SCL, LMO1, DRG, mSin3A, and E47, a dimerization region of SCL, a dimerization region of LMO1, a dimerization region of DRG, a dimerization region of mSin3A, and a dimerization region of E47.

In a particularly preferred embodiment, the interaction is between the dimerization region of SCL and the dimerization region of LMO2 or the dimerization region of E47 or the dimerization region of LMO1.

In an alternative preferred embodiment, the interaction is between the dimerization region of LMO2 and the dimerization region of E47 or the dimerization region of mSin3A.

In a further preferred embodiment, the interaction is between the dimerization region of E47 and the dimerization region of mSin3A.

Preferably, the transcriptional activator domain is the GAL4 activator domain.

Preferably, the DNA binding domain is selected from the group consisting of: *LexA* operator binding domain, GAL4 DNA binding domain; and *cl* operator binding domain.

As exemplified herein, the present inventors show that a reverse two hybrid screen of the invention is capable of distinguishing between interactions in the same cell involving (i) SCL and LMO2; and (ii) SCL and the LMO1, mSin3A, or E47 proteins. In additional to distinguishing between these interactions, the method applies reverse n-hybrid screening procedures to identify peptide aptamers that block such interactions, and, as a consequence, have potential utility as therapeutic or diagnostic reagents.

In a further example, the inventive method described herein distinguishes effectively between interactions in the same cell between an RNA molecule and a protein. Accordingly, the present invention is equally applicable to the detection of inhibitors of both protein and nucleic acid interactions in cells.

The improved reverse two hybrid or reverse three hybrid screens described herein require the expression of the following integers in a single cell:
(i) the binding partners of a target interaction such that their interaction enhances the expression of one or more reporter genes in said cell and said expression is partially or completely inhibited by disruption of said target interaction;
(ii) the bind ing partners of a non-target interaction such that their interaction enhances the expression of one or more reporter genes in said cell and said expression is partially or completely inhibited by disruption of said non-target interaction, wherein said reporter gene(s) is(are) distinct from the reporter gene(s) expressed under control of the target interaction; and
(iii) a candidate peptide being tested for inhibitory activity;
wherein one or more reporter genes operably under the control of the target interaction or the non-target interaction is a counter selectable reporter gene that confers conditional lethality on the cellular host, and wherein, to determine the minimum concentration of binding partner expression required for the counter-selectable reporter gene to be expressed at a level that causes cell death, the expression of one or more of said binding partners is titrated in response to galactose or copper by placing said expression under the control of a promoter comprising regulatory elements of the *GAL1* or *CUP1* promoters.

As will be understood by the skilled person, the term "expressing" includes transcription of a gene to produce mRNA with or without any post-transcriptional processing of the primary transcript, such as, for example, mRNA splicing or the addition of a polyadenylate sequence, to produce translatable mRNA. Such " further includes transcription followed by translation of the mRNA, to produce a peptide or protein, with or without any post-translational modification of the protein required to modulate its activity, such as, for example glycosylation, processing, splicing, or transport.

It will be apparent to those skilled in the art from the description herein that the inventive method is equally applicable to the high throughput screening of a panel of candidate peptides for inhibitory activity. For example, a library of clones expressing candidate peptides may be produced and each member of said library introduced into cells in which the target and non-target interactions are capable of being assayed. Those cells in which the target interaction is partially or completely inhibited selected, wherein each selected cell has a clone that inhibits the target interaction in the cell.

Accordingly, as used herein, the term "expressing a candidate peptide", clearly encompasses the expressing of several members of a library of clones wherein each member expresses a single candidate peptide.

As used herein, the term "library" is a set of diverse nucleotide sequences encoding a set of amino acid sequences, wherein said nucleotide sequences are preferably contained within a suitable vector, cosmid, bacteriophage or virus vector molecule which is suitable for maintenance and/or replication in a cellular host The term "library" includes a random synthetic peptide library, in which the extent of diversity between the amino acid sequences or nucleotide sequences is numerous, and a limited peptide library in which there is a lesser degree of diversity between said sequences. The term "library" further encompasses diverse amino acid sequences derived from a cellular source, such as, for example, genome fragments obtained for example by shearing or partial digestion of genomic DNA using restriction endonucleases, amongst other approaches. A "peptide library" further includes cells, virus particles and bacteriophage particles comprising the individual amino acid sequences or nucleotide sequences of the diverse set

Preferred libraries according to this embodiment of the invention are "representative libraries", comprising a set of amino acid sequences or nucleotide sequences encoding same, which includes all possible combinations of amino acid or nucleotide sequences for a specified length of peptide or nucleic acid molecule, respectively.

The diversity of peptide libraries, in particular those derived from genomic sources, can be increased by means known to those skilled in the art, such as, for example, random or other mutagenesis. In one exemplification of this embodiment, peptide libraries derived from the expression of genomic DNA are amplified or propagated in bacterial strains which are defective in the epsilon (ε) subunit of DNA polymerase III (i.e. *dnaQ* and *mutD* alleles) and/or are defective in mismatch repair. *Escherichia coli* mutator strains possessing the *mutY* and/or *mutM* and/or *mutD* and/or *mutT* and/or *mutA* and/or *mutC* and/or *mutS* alleles are particularly useful for such applications. Bacterial strains carrying such mutations are readily available to those skilled in the art and are fully described for example, by Akiyama *et al* 1989; Fijalkowska and Schaaper, 1995; Frick *et al,* 1995; Lu *et al*, 1995; Maki and Sekiguchi, 1992; Miller, 1992; Miller and Michaels, 1996; Moriya and Gollman, 1992; Schaaper and Comacchio, 1992; Slupska *et al,* 1996; and Tajiri *et al*, 1995.

The expressed candidate peptide may comprise any amino acid sequence of at least about 1 to 60 amino acids in length and may be derived from the expression of nucleotide sequences which are prepared by any one of a variety of methods such as, for example, random synthetic generation, or using natural-occurring genomes as exemplified herein.

Preferably, the peptide is a 20-mer peptide. The use of larger fragments, particularly employing randomly sheared nucleic acid derived from bacterial, yeast or animal genomes, is not excluded.

Alternatively or in addition, the candidate peptide is expressed as a fusion protein with a nuclear targeting motif capable of facilitating targeting of said peptide to the nucleus of said host cell where transcription occurs, in particular the yeast-operable SV40 nuclear localization signal.

Alternatively, or in addition, the candidate peptide is expressed as a fusion protein with a peptide sequence capable of enhancing uptake of the peptide by an isolated cell such as, for example, when the subject peptide is synthesized *ex vivo* and added to isolated cells in culture. In a preferred embodiment, the peptide sequence capable of enhancing, increasing or assisting penetration or uptake is functional in insect cells or mammalian cells, for example the *Drosophila* penetratin targeting sequence.

The candidate peptide may also be expressed in a conformationally constrained form. Amino acid sequences which are expressed in a conformationally constrained form may be expressed within a second polypeptide as a fusion protein such that they are effectively "nested" in the secondary structure of the second polypeptide. Alternatively, the peptide, oligopeptide or polypeptide may be circularized within a loop of disulphide bonds to limit conformational diversity, such as, for example, by expressing the peptide within oxidized flanking cysteine residues. This may be particularly beneficial where the amino acid sequences are nested within a surface-exposed or functional site of a protein, such that they are accessible to the interaction of interest. For example, the peptide may be expressed within a thioredoxin (Trx) polypeptide loop.

The binding partners may be expressed from separate DNA molecules or vectors. Alternatively, they can be expressed from a single DNA molecule or vector. The only requirement for expression of the binding partners is that both partners are expressed in a cell at the same time as the candidate peptide being assayed. Additionally, said binding partners are expressed under conditions sufficient to modulate expression of the reporter genes to which they are operably connected in the cell.

The present invention clearly contemplates higher order interactions involving three or four or more binding partners of the target or non-target interaction.

The binding partners are preferably expressed as fusion proteins with a nuclear localization sequence to facilitate their transport to the site of transcription in a eukaryotic cell (i.e. the nucleus), such as, for example, the SV40 large T antigen nuclear localization signal.

A requirement for a suitable reporter gene is the capability of being expressed in a manner that is readily detected, such as by the phenotype said expression confers on the cell (for example, auxotrophy or prototrophy for a particular metabolite, or conditional lethality in the presence of a particular substrate), or alternatively, by expressing an enzyme activity, or a protein detectable by immunoassay or colorimetric detection, or fluorescence. As noted above, at least one of the reporter genes in the method is a counter-selectable reporter gene.

Suitable reporter genes include those encoding *Escherichia coli* β-galactosidase enzyme, the firefly luciferase protein (Ow *et al,* 1986; Thompson *et al,* 1991) the green fluorescent protein (Prasher *et al,* 1992; Chalfie *et al*, 1994; Inouye and Tsuji, 1994; Cormack *et al,* 1996; Haas *et al,* 1996; see also GenBank Accession No. U55762); and the red fluorescent proteins of *Discosoma* (Matz *et al.,* 1999) or *Propionibacterium freudenreichii,* (Wildt and Deuschle, 1999). Additionally, the *HIS*3 gene [Larson, R.C. *et al.* (1996), Condorelli, G.L. *et al.* (1996), Hsu, H.L., *et al.* (1991), Osada, *et al.* (1995)] and *LEU*2 gene (Mahajan, M.A. *et al.,* 1996) are also useful.

At least one reporter gene is a counter selectable reporter gene (i.e. confers conditional lethality on the cell). Other reporter genes may include genes that encode fluorescent proteins, and a combination of counter-selectable reporter genes and genes encoding fluorescent proteins may be used. Both types of reporter genes have suitability for high throughput applications, where large numbers of samples are screened in batches. Fluorescence can be readily assayed by fluorometry or fluorescence activated cell sorting (FACS), techniques known to those skilled in the art.

Wherein a reporter gene operably under the control of the target interaction or the non-target interaction encodes a fluorescent protein, detection of reporter gene expression is determined by identifying those cells that fluoresce when said reporter gene is expressed. Alternatively, inhibition of reporter gene expression is determined by identifying those cells that do not fluoresce or have reduced fluorescence compared to when said reporter gene is expressed.

Preferred reporter genes encoding fluorescent proteins include the *gfp* gene, the *cobA* gene, and fragments or variants of said *gfp* gene or *cobA* gene that encode a fluorescent proteins.

Wherein a reporter gene operably under the control of the target interaction or the non-target interaction is a counter selectable reporter gene that confers conditional lethality on the cell, detection of reporter gene expression is determined by identifying those cells that cease to grow or die when said reporter gene is expressed under conditions that confer lethality. Alternatively, inhibition of reporter gene expression is determined by identifying those cells that do not cease to grow compared to when said reporter gene is expressed, or do not die, under conditions that confer lethality.

Preferred counter selectable reporter genes are those genes that encode a polypeptide capable of converting a non - toxic substrate to a toxic product, in particular a counter selectable reporter gene is selected from the group consisting of: *CAN, URA3, CYH2, and LYS2.*

In a particularly preferred embodiment, the combination of *URA3* and *CYH2* genes operably under the control of the target interaction is preferred. Alternatively or in addition, use of the *LYS2* gene operably under control of the non-target interaction is particularly preferred.

In an alternative embodiment, multiple reporter genes are operably under the control of the target interaction, wherein said reporter genes comprise at least one counter selectable reporter gene and at least one gene encoding a fluorescent protein such that said detecting comprises identifying those cells grow or survive and do not fluoresce or have reduced fluorescence when said reporter gene is not expressed compared to when said reporter gene is expressed.

Similarly, multiple reporter genes can be placed operably under the control of the non-target interaction which genes include at least one counter selectable reporter gene and at least one gene encoding a fluorescent protein.

Persons skilled in the art will be aware of how to utilize genetic sequences encoding such reporter molecules in performing the invention described herein, without undue experimentation. For example, the coding sequence of the gene encoding such a reporter molecule may be modified for use in the cell line of interest (e.g. human cells, yeast cells) in accordance with known codon usage preferences. Additionally the translational efficiency of mRNA derived from non-eukaryotic sources may be improved by mutating the corresponding gene sequence or otherwise introducing to said gene sequence a Kozak consensus translation initiation site (Kozak, 1987).

To link reporter gene expression to the target interaction, or conversely, to the non-target interaction, the reporter gene should include one or more *cis*-acting sequences to which one of the binding partners to said interaction can bind or dock. Such features are known to those skilled in the art.

Preferably, the *cis*-acting sequence of the target interaction or the non-target interaction is selected from the group consisting of: LexA operator, GAL4 binding site, and *cl* operator. In accordance with this embodiment of the invention, it is preferred for a binding partner that includes a DNA binding domain to be capable of binding to said *cis*-acting sequence, in which case said DNA binding domain will be selected from the group consisting of: *LexA* operator binding domain, GAL4 DNA binding domain; and *cl* operator binding domain.

It will be known to those skilled in the art that the expression of an integer in a cell requires nucleic acid to be placed operably in connection with a promoter sequence that is operable in the cell.

Reference herein to a "promoter" is to be taken in its broadest context and includes the transcriptional regulatory sequences of a classical genomic gene, including the TATA box which is required for accurate transcription initiation in eukaryotic cells, with or without a CCAAT box sequence and additional regulatory elements (i.e. upstream activating sequences, enhancers and silencers). Promoters may also be lacking a TATA box motif, however comprise one or more "initiator elements" or, as in the case of yeast-derived promoter sequences, comprise one or more "upstream activator sequences" or "UAS" elements. For expression in prokaryotic cells such as, for example, bacteria, the promoter should at least contain the -35 box and -10 box sequences.

A promoter is usually, positioned upstream or 5' of a structural gene, the expression of which it regulates. Furthermore, the regulatory elements comprising a promoter are usually positioned within about 2 kb of the start site of transcription of the gene.

In the present context, the term "promoter" is also used to describe a synthetic or fusion molecule, or derivative which confers, activates or enhances expression of the subject reporter molecule in a cell.

Promoters may contain additional copies of one or more specific regulatory elements, to further enhance expression of the gene and/or to alter the spatial expression and/or temporal expression. For example, regulatory elements which facilitate the enhanced expression of a gene by galactose or glucose or copper may be placed adjacent to a heterologous promoter sequence driving expression of the gene. In the method of the invention, promoters comprising regulatory elements of the *GAL1* or *CUP1* promoters are used for titration of the expression of one or more binding partners in response to galactose or copper, respectively, in the culture medium in which the host cell is grown.

Placing a gene operably under the control of a promoter sequence means positioning the said gene such that its expression is controlled by the promoter sequence. Promoters are generally positioned 5' (upstream) to the genes that they control. In the construction of heterologous promoter/structural gene combinations it is generally preferred to position the promoter at a distance from the gene transcription start site that is approximately the same as the distance between that promoter and the gene it controls in its natural setting, i.e., the gene from which the promoter is derived. As is known in the art, some variation in this distance can be accommodated without loss of promoter function. Similarly, the preferred positioning of a regulatory sequence element with respect to a heterologous gene to be placed under its control is defined by the positioning of the element in its natural setting, i.e., the genes from which it is derived. Again, as is known in the art, some variation in this distance can also occur.

Examples of promoters suitable for use in regulating the expression of the reporter molecule and/or candidate peptide and/or a proteinaceous binding partner include viral, fungal, yeast, insect, animal and plant promoters, especially those that can confer expression in a eukaryotic cell, such as, for example, a yeast cell or a mammalian cell.

Particularly preferred promoters according to the present invention include those naturally-occurring and synthetic promoters which contain binding sites for transcription factors, more preferably for helix-loop-helbc (HLH) transcription factors, zinc finger proteins, leucine zipper proteins and the like. Preferred promoters may also be synthetic sequences comprising one or more upstream operator sequences such as, for example, LexA operator sequences or activating sequences derived from any of the promoters referred to herein such as, for example, *GAL4* DNA binding sites.

Those skilled in the art will recognise that the choice of promoter will depend upon the nature of the cell being transformed and the molecule to be expressed. Such persons will be readily capable of determining functional combinations of minimum promoter sequences and operators for cell types in which the inventive method is performed.

Whilst the invention is preferably performed in yeast cells, the inventors clearly contemplate modifications wherein the invention is performed entirely in bacterial or mammalian cells, utilizing appropriate promoters which are operable therein to drive expression of the various assay components in such cells. Such embodiments are within the ken of those skilled in the art.

In a particularly preferred embodiment, the promoter is a yeast promoter, mammalian promoter, a bacterial or bacteriophage promoter, selected from the group consisting of: *MYC, GAL1, CUP1, PGK1, ADH1, ADH2, PHO4, PHO5, HIS4, HIS5, TEF1, PRB1, GUT1, SPO13, CMV, SV40, LAC, TEF, EM7, SV40,* and *T7*.promoter sequences.

For expression in mammalian cells, it is preferred that the promoter is the *CMV* promoter sequence, more preferably the *CMV-IE* promoter or alternatively, the *SV40* promoter and, in particular the *SV40* late promoter sequence. These promoters, and other promoter sequences suitable for expression of genes in mammalian cells are known in the art.

Examples of mammalian cells contemplated herein to be suitable for expression include COS, VERO, HeLa, mouse C127, Chinese hamster ovary (CHO), WI-38, baby hamster kidney (BHK) or MDCK cell lines, amongst others. Such cell lines are readily available to those skilled in the art.

The prerequisite for producing intact polypeptides in bacterial cells and, in particular, in *Escherichia coli* cells, is the use of a strong promoter with an effective ribosome binding site, such as, for example, a Shine-Dalgamo sequence, which may be incorporated into expression vectors carrying the first and second nucleotide sequences, or other genetic constructs used in performing the various alternative embodiments of the invention. Typical promoters suitable for expression in bacterial cells such as, for example, *E*. *coli* include, but are not limited to, the *lacz* promoter, temperature-sensitive λ_{L} or λ_{R} promoters, T7 promoter or the IPTG-inducible *tac* promoter. A number of other vector systems for expressing nucleic acid in *E.coli* are well-known in the art and are described for example in Ausubel *et al* (1987) or Sambrook *et al* (1989). Numerous sources of genetic sequences suitable for expression in bacteria are also publicly available in various vector constructs, such as for example, pKC30 (λ_{L}: Shimatake and Rosenberg, 1981), pKK173-3 (*tac:* Amann and Brosius, 1985), pET-3 (T7: Studier and Moffat, 1986) or the pQE series of expression vectors (Qiagen, CA), amongst others.

Wherein the promoter is intended to regulate expression of the reporter molecule, it is particularly preferred that said promoter include one or more recognition sequences for the binding of a DNA binding domain derived from a transcription factor, for example a *GAL4* binding site or *LexA* operator sequence.

In accordance with the inventive method, host cells expressing the above components are grown under conditions sufficient to enable the binding partners of the target interaction, at least, to associate. The binding partners of the non-target interaction may also associate under such conditions. However, the formation of the target interaction and the non-target interaction are distinguished by virtue of the operable connection of the target interaction and the non - target interaction to distinct reporter genes, which can be assayed separately or simultaneously, depending upon the reporter genes used. For example, distinct counter selectable reporter genes can be used for assaying the target and non-target interactions, in which case those interactions can be distinguished by survival or growth of cells on particular substrates. As exemplified herein, the present inventors show that it is possible to distinguish between a target interaction operably linked to both *URA3* and *CYH2* genes, and a non-target interaction linked to the *LYS2* gene. In this embodiment, cells in which the target interaction but not the non-target interaction is inhibited are detectable, because they are resistant to fluororotic acid (5-FOA) and cycloheximide, and do not require lysine for growth and/or are sensitive to growth on media containing α-aminoadipate (α-AA). Conversely, cells in which the target interaction but not the non-target interaction activates reporter gene expression are detectable, because they are sensitive to fluororotic acid (5-FOA) and cycloheximide, and require exogenous lysine for growth and/or are resistant to growth on media containing α-aminoadipate (α-AA). Similarly, the present inventors have shown that it is possible to distinguish between target interaction and non-target interactions operably linked to distinct fluorescent protein-encoding reporter genes, by virtue of detecting the different emission wavelengths of the expressed proteins. However, the method of the invention requires that at least one reporter gene under the control of the target or non - target interaction is a counter-selectable reporter gene.

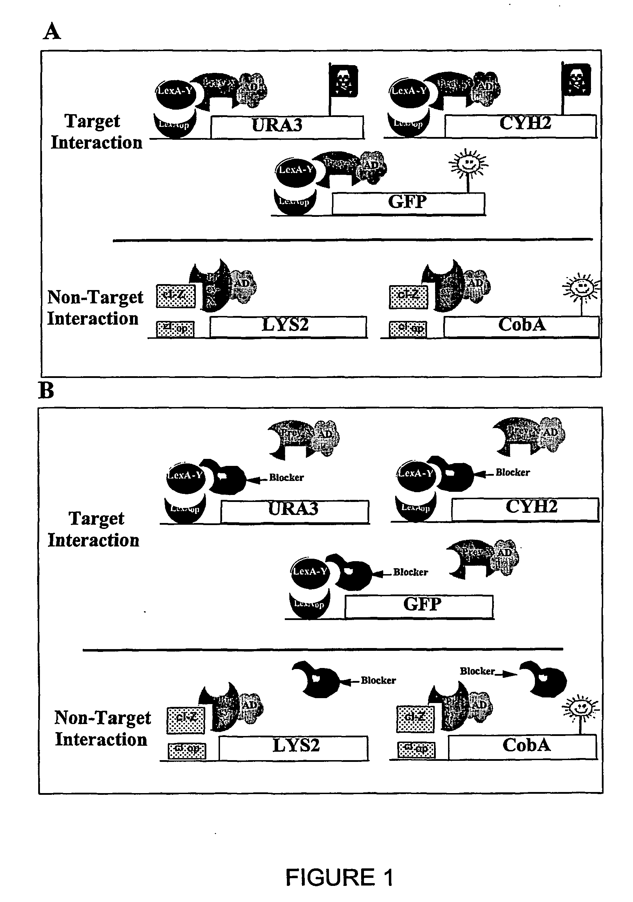

Since the exemplified reporter genes for the non-target interaction pairs are LYS2 and CobA (Figures 1-4), specificity of the inhibition achieved by blocking is determined by replica plating clones from the primary screen to media lacking lysine and checking for the presence of red fluorescence under excitation with UV light, indicating no inhibition of the target interaction. The 'interaction mating' technique of Finley and Brent (1994) can be used to rapidly determine whether peptide inhibitors isolated from the above screen are specific for the target interaction. However this approach is more time consuming since it requires rescue of the candidate peptide inhibitor plasmids from the diploid strain and re-transformation into a haploid strain of appropriate mating type that lacks the original bait plasmid, prior to mating to strains containing the specificity controls. An advantage of having an endogenous non -target bait linked to a different DNA binding domain is the ability to confirm specificity of the peptide aptamerfor a particular interaction directly, in the same strain in which the peptide inhibitor is expressed. This increase in efficiency is important for high through-put applications of the technology.

Preferably, the subject method further comprises selecting and growing the detected cells. Such selection will be based upon the detection of the reporter gene(s) used, and can be readily performed using art-recognized procedures. Similarly, culture methods for growing bacterial, yeast, or mammalian cells are well-known in the art.

Preferably, nucleic acid encoding the candidate peptide, one or more reporter genes, or one or more binding partners, is contained within a vector selected from the group consisting of: pBLOCK-3.0 (SEQ ID NO: 1); pBLOCK-3.2 (SEQ ID NO: 2); pBLOCK-3.4 (SEQ ID NO: 3); pBLOCK-3.6 (SEQ ID NO: 4); pBLOCK-3.8 (SEQ ID NO: 5); pBLOCK-3.9 (SEQ ID NO: 6); pBLOCK-3.10 (SEQ ID NO: 7); pBLOCK-3.11 (SEQ ID NO: 8); pBLOCK-4.0 (SEQ ID NO: 9); and pRT2 (SEQ ID NO: 10). Even more preferably, nucleic acid encoding the candidate peptide is contained within the vector set forth in any one of SEQ ID NOs: 1 to 9; nucleic acid encoding up to three of the binding partners is contained within pBLOCK-3.11 (SEQ ID NO: 8); and nucleic acid encoding two fluorescent reporter genes is contained within the vector pRT2 (SEQ ID NO: 10).

In an alternative embodiment of the present invention, the subject method comprises the further step of introducing into the cellular host one or more further nucleic acid molecules which encodes one or more polypeptide binding partners which are involved in the interaction, placed operably under the control of one or more suitable promoter sequence. Accordingly, the inventive method may be modified by introducing into the cellular host one or more nucleic acids selected from the group consisting of:
(i) nucleic acid encoding a binding partner of the target interaction in an expressible format;
(ii) nucleic acid encoding a binding partner of the non-target interaction in an expressible format;
(iii) nucleic acid encoding the candidate peptide in an expressible format;
(iv) nucleic acid comprising a *cis*-acting sequence and a reporter gene in an expressible format; and
(v) nucleic acid comprising a *cis*-acting sequence and a counter selectable reporter gene in an expressible format.

Methods for introducing the nucleic acid into the cellular host include mating those cells having one or more of said nucleic acids so as to combine sufficient nucleic acids into a single cell to select those host cells that grow or survive when the counter selectable reporter genes operably under control of the target interaction are expressed. Alternatively, standard transformation ortransfection procedures may be used to introduce individual genes into cells. In fact, any standard means may be used for their introduction, including cell mating, transformation ortransfection procedures.

In one embodiment, the method of the present invention is a method of identifying a peptide that partially or completely inhibits a target interaction between two or more binding partners in a yeast cell but does not inhibit a non - target interaction between some but not all of said binding partners, said method comprising:
(i) transforming a yeast cell with a vector selected from the group consisting of: pBLOCK-3.0 (SEQ ID NO:1); pBLOCK 3.2 (SEQ ID NO: 2); pBLOCK-3.4 (SEQ ID NO: 3); pBLOCK 3.6 (SEQ ID NO: 4); pBLOCK-3,8 (SEQ ID NO: 5); pBLOCK 3.9 (SEQ ID NO: 6); pBLOCK 3.10 (SEQ ID NO: 7); pBLOCK 3.11 (SEQ ID NO: 8); and pBLOCK-4.0 (SEQ ID NO: 9), wherein said vector further comprises nucleic acid encoding a candidate peptide being tested for inhibitory activity;
(ii) introducing to said transformed yeast cell nucleic acid encoding: (a) the binding partners of said target interaction such that they operably control the expression of one or more counter selectable reporter genes or fluorescent protein-encoding reporter genes in said cellular host, wherein said expression is partially or completely inhibited by disruption of said target interaction; and (b) the binding partners of said non-target interaction such that they operably control the expression of one or more counter selectable reporter genes or fluorescent protein-encoding reporter genes in said cellular host, wherein said expression is partially or completely inhibited by disruption of said non-target interaction and wherein said reporter gene is distinct from the reporter gene(s) expressed under control of the target interaction;
   wherein one or more reporter genes operably under the control of the target interaction or the non-target interaction is a counter selectable reporter gene that confers conditional lethality on the cellular host, and wherein, to determine the minimum concentration of binding partner expression required for the counter-selectable reporter gene to be expressed at a level that causes cell death, the expression of one or more of said binding partners is titrated in response to galactose or copper by placing said expression under the control of a promoter comprising regulatory elements of the *GAL1* or *CUP1* promoters:
(iii) selecting the recombinants;
(iv) growing the recombinants under conditions sufficient to distinguish the expression of each reporter gene(s) at (a) from expression of the reporter gene(s) at (b); and
(v) detecting those host cells wherein expression of the reporter gene(s) operably under control of the target interaction is(are) partially or completely inhibited and expression of the reporter gene(s) operably under control of the non-target interaction is(are) not inhibited, said detected cells expressing a peptide that partially or completely inhibits the target interaction.

Preferably, the fluorescent protein-encoding reporter genes are introduced to the cell by transforming the cell with the vector pRT2 (SEQ ID NO: 10)

Alternatively, or in addition, nucleic acid encoding one or more of the binding partners of the target interaction and/or the non-target interaction are introduced to the cell by transforming the cell with a derivative of a vector selected from the group consisting of pBLOCK 3.0 (SEQ ID NO:1); pBLOCK 3.2 (SEQ ID NO: 2); pBLOCK 3.4 (SEQ ID NO: 3); pBLOCK-3.6 (SEQ ID NO: 4); pBLOCK 3.8 (SEQ ID NO: 5); pBLOCK 3.9 (SEQ ID NO: 6); pBLOCK-3.10 (SEQ ID NO: 7); pBLOCK 3.11 (SEQ ID NO: 8); and pBLOCK-4.0 (SEQ ID NO: 9), wherein said derivative includes a nucleotide sequence encoding said binding partner.

In a preferred embodiment, nucleic acid encoding' up to three binding partners of the target interaction and/or the non target interaction are introduced to the cell by transforming the cell with
a derivative of the vector pBLOCK 3.11 (SEQ ID NO: 9), said derivative including nucleotide sequences encoding said binding partners.

In a further preferred embodiment, the subject method further comprises the step of isolating the third nucleic acid molecule from the host cell and sequencing the nucleic acid molecule and deriving the amino acid sequence encoded therefor. Synthetic peptides may be produced, based upon the derived amino acid sequence thus obtained. Techniques for such methods are described, for example by Ausubel *et al* (1987), amongst others. Those skilled in the art are well versed in such techniques.

In an alternative preferred embodiment, very specific peptides isolated from such screens are expressed, or alternatively, synthesized, prior to their delivery to a culture of eukaryotic cells. The effect of such a peptide on the expression of a range of genes, or on the development of a phenotype, is determined relative to cells lacking the peptide. For example the expression profile of the cell can be monitored using micro-array technology known to those skilled in the art. Accordingly, an inhibitory peptide identified using the improved reverse n-hybrid assay of the invention can serve as a dominant negative probe of a gene pathway. Such pathways are otherwise recalcitrant to analysis by standard genetic means. Additionally, the probes can facilitate the validation of candidate drug targets or the identification of new targets using surrogate markers.

A peptide may be identified by the method of the present invention, wherein said peptides have inhibitory activity against the target interaction, however possess reduced inhibitory activity or no inhibitory activity against the non-target interaction. A pharmaceutical composition may be prepared, comprising said inhibitory peptide and a pharmaceutically acceptable carrier and/or diluent.

Preferably, the peptide antagonizes or inhibits an interaction that produces one or more deleterious effects in eukaryotic cells, in particular human or animal cells. More preferably, the peptides antagonize or inhibit interactions that involve one or more oncoproteins.

Inhibitory peptides identified according to the present invention may be used in the prophylactic or therapeutic treatment of humans or animals. This may include their use in peptide therapy regimens such as, for example, in the treatment protocols for patients with leukemia and/or solid tumors. Their use in treatment protocols for said patients includes their administration as a means of blocking further cell division of the malignant cells, for example, targeting of the SCL oncoprotein to arrest the malignant cell division of the patient. The specific targeting of oncoproteins with pharmaceuticals comprising said peptides will reduce the side effects experienced by patients as compared to t hose experienced with conventional chemotherapy.

Such inhibitory peptides may also be used to treat other disorders resulting from deleterious expression of aberrant biological molecules that interfere with normal cellular functions.

Pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. Alternatively, injectable solutions may be delivered encapsulated in liposomes to assist their transport across cell membrane. Alternatively or in addition such preparations may contain constituents of self-assembling pore structures to facilitate transport across the cellular membrane. It must be stable under the conditions of manufacture and storage and must be preserved against the contaminating/destructive action of microorganisms such as, for example, bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as, for example, lecithin, by the maintenance of the required particle size in the case of dispersion and by the use of surfactants. Preventing the action of microorganisms in the compositions of the invention is achieved by adding antibacterial and/or antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and freeze-drying, to yield a powder of the active ingredient plus any additional desired ingredient from previously sterile-filtered solution thereof.

When the active ingredients are suitably protected they may be orally administered, for example, with an inert diluent or with an edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that a dosage unit form contains between about 0.1 ug and 20g of active compound.

The tablets, troches, pills, capsules and the like may also contain binding agents, such as, for example, gum, acacia, com starch or gelatin. They may also contain an excipient, such as, for example, dicalcium phosphate. They may also contain a disintegrating agent such as, for example, corn starch, potato starch, alginic acid and the like. They may also contain a lubricant such as, for example, magnesium stearate. They may also contain a sweetening agent such a sucrose, lactose or saccharin. They may also contain a flavoring agent such as, for example, peppermint, oil of wintergreen, or cherry flavoring.

When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparaben as preservatives, a dye and flavoring such as, for example, cheny or orange flavor. Of course, any material used in preparing any dosage unit form should be pharmaceutically pure and substantially non-toxic in the amounts employed. In addition, the active compound(s) may be incorporated into sustained-release preparations and formulations.

Forms suitable for topical application include for example, creams, lotions and gels.

Pharmaceutically acceptable carriers and/or diluents include any and all solvents, dispersion media, coatings, antibacterials and/or antifungals, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated, each unit containing a predetermined quantity of active material calculated to produce the desired therapeutic effect in association with the required pharmaceutical carrier. The dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form. A unit dosage form can, for example, contain the principal active compound in amounts ranging from 0.5 µg to about 2000 mg. Expressed in proportions, the active compound is generally present in from about 0.5 µg to about 2000 mg/ml of carrier. In the case of compositions containing supplementary active ingredients, the dosages are determined by reference to the usual dose and manner of administration of the said ingredients. The pharmaceutical composition may also comprise genetic molecules such as, for example, a vector capable of transfecting target cells where the vector carries a nucleic acid molecule capable of inhibiting such deleterious biological interactions. The vector may, for example, be a viral vector.

The method of the present invention may use a vector for expressing one or more peptides and/or binding partners in a prokaryotic or eukaryotic cell, said vector comprising the features of a vector selected from the group consisting of: pBLOCK-3.0 (SEQ ID NO: 1); pBLOCK-3.2 (SEQ ID NO: 2); pBLOCK-3.4 (SEQ ID NO: 3); pBLOCK-3.6 (SEQ ID NO: 4); pBLOCK-3.8 (SEQ ID NO: 5); pBLOCK-3.9 (SEQ ID NO: 6); pBLOCK-3.10 (SEQ ID NO: 7); pBLOCK-3.11 (SEQ ID NO: 8); and pBLOCK-4.0 (SEQ ID NO: 9). The specific characteristics of such shuttle vectors will be apparent to the skilled person from the detailed description of the invention and the accompanying drawings and Sequence Listing.

The method may further use a vector for expressing multiple fluorescent reporter genes in a yeast cell, said vector comprising:
(i) a green fluorescent protein expression cassette comprising the *gfp* gene operably under control of a chimeric yeast operable *LexAlGAL*1 promoter having multiple LexA operator sites; and
(ii) a red fluorescent protein expression cassette comprising the *cobA* gene operably under control of a chimeric *cl*/*GAL1* promoter having multiple *cl* operator sites.

The specific characteristics of such a vector will be apparent to the skilled person from the detailed description of the invention and the accompanying drawings and Sequence Listing.

It will be apparent from the description herein that the efficacy of any peptides identified by the reverse n-hybrid screens of the invention can be validated in several ways, and generally, by comparing the effect of the peptides on target cells of interest. For example, the exclusive rescue of yeast peptide library plasmids from positive blocking clones is rapidly achieved through selection in bacteria for the unique marker on the pBLOCK vector (e.g. blasticidin resistance). The DNA encoding the peptide can then be sequenced. For peptide libraries derived from sequenced natural genomes, it is not necessary to sequence the inhibitory peptide clone. Instead, the positive clones are merely transferred, such as by robot, onto master stocks, and the peptide-encoding DNA inserts amplified using PCR, to yield probes that can be hybridized to oligonucleotide micro arrays. Such arrays represent the whole genomes used to construct the library. The genomic location of a given candidate blocker can then be deduced by reference to the coordinates of the DNA micro array. The rescued DNA is then expressed in a suitable cell line to assess its effect on the phenotype or the expression profile of the cell. Such information is useful to confirm the inhibitory activity, therapeutic activity, or prophylactic activity, of the peptide *in vivo,* or to identify new binding partners, or drug targets in the same biochemical/signalling pathways as the partner of the target interaction.

Accordingly, a method for determining the effect of a peptide on a eukaryotic cell may comprise:
(i) isolating a nucleotide sequence encoding a peptide inhibitor identified by the method described herein;
(ii) transfecting said eukaryotic cell with the isolated nucleic acid; and
(iii) comparing the phenotype or expression pattern of the transfected eukaryotic cell to the phenotype or expression pattern of an otherwise isogenic non-transfected cell, wherein a different phenotype or expression pattern indicates that the peptide has an effect on the cell.

Preferably, such a method is performed on a mammalian cell.

The expression pattern of the transfected eukaryotic cell may be compared to the expression pattern of an otherwise isogenic non-transfected cell, by producing an array of protein or nucleic acid expressed by the transfected and non - transfected cells and comparing said protein or nucleic acid. Array technologies offer a considerable advantage in terms of providing rapid high throughput readouts of data, thereby facilitating the identification of novel binding partners for the inhibitory peptide, which binding partners may be nucleic acid or protein. Moreover, the novel binding partners may also be previously unknown binding partners for one or more of the binding partners of the target interaction assayed in accordance with the inventive method described herein.

Furthermore, when this method is performed on a diseased cell, such as, for example, a leukemia cell or other cancer cell, information derived from the modified expression pattern of the transfected cell, or the modified phenotype of said cell, is useful for designing new prophylactic or therapeutic drugs. In particular, the capability of a particular peptide to partially or completely reverse or correct the diseased phenotype (e.g. cancerous phenotype) can be determined.

Accordingly, a process for identifying a peptide for the prophylactic or therapeutic treatment of a mammal may comprise:
(i) isolating a nucleotide sequence encoding a peptide inhibitor identified by the method described herein;
(ii) transfecting said eukaryotic cell with the isolated nucleic acid;
(iii) comparing the phenotype or expression pattern of the transfected eukaryotic cell to the phenotype or expression pattern of an otherwise isogenic non-transfected cell, wherein a different phenotype or expression pattern indicates that the peptide has an effect on the cell; and
(iv) selecting a peptide that reverts the phenotype or expression profile of the transfected cell.

### BRIEF DESCRIPTION OF THE DRAWINGS

Figure 1 is a schematic representation of a generalized reverse two hybrid screen for peptides that specifically antagonize the interaction between two hypothetical proteins (designated X and Y in the Figure), however do not block the interaction between one of said hypothetical proteins (designated X in the figure) and another hypothetical protein (designated Z in the figure).
   Panel A shows the expected expression profile and selection characteristics of the cell in the absence of antagonism of the target X/Y interaction and the non-target X/Z interaction. The "prey" (designated "Prey-X" plus "AD" in the figure) for both the target interaction (top two rows) and the non-target interaction (lower row) comprises protein X expressed as a fusion protein with the activation domain (AD) of a transcription factor. The "bait" for the target interaction (top two rows) comprises a fusion protein between protein Y and the DNA binding domain of the LexA protein (designated "LexA-Y" in the figure). The reporter genes for the target interaction (top two rows) consist of chimeric counter selectable reporter genes *URA3* and *CYH2*, and the green fluorescent protein-encoding *gfp* gene ("GFP"; Prasher *et al.*, 1992; Chalfie *et al*., 1994; Inouye and Tsuji,1994) comprising one or more *LexA* operator sequences (LexAop) to which the bait can bind. The non-target bait comprises a fusion protein between protein Z and the cl repressor protein (stippled box labeled "cl-Z°). The reporter gene for the non-target interaction consists of chimeric LYS2 gene and chimeric *cobA* gene, each comprising one or more *cl* operator sequences (stippled boxes labeled "clop") to which the non-target bait can bind. The *cobA* gene encodes a red fluorescent protein (Wildt and Deuschle,1999). Black flags with skull and cross-bones indicate expression of the counter selectable reporter genes. Sun symbols indicate expression of the *gfp* and *cobA* reporter genes encoding green and red fluorescent proteins, respectively. Accordingly, the yeast strain is sensitive to 5-FOA and cycloheximide, due to the activating interaction between the target bait and prey fusion proteins bound to the LexA operator site(s) of the counter selectable reporter genes *URA3* and *CYH2;* and prototrophic for lysine biosynthesis, due to the activating interaction between the non-target bait and prey fusion proteins bound to the cl operator sites (clop) of the *LYS2* reporter gene which prototrophy can complement an auxotrophic *lys2* mutation in yeast. The strain can also express the *gfp* and *cobA* genes under the control of distinct upstream promoter elements, and, as a consequence, fluoresces at wavelengths for green light and/or red light Activation of these non-lethal reporter genes can be used to quantitate the strength of reporter gene activation using non-counterselectable conditions.
   Panel B shows the expected expression profile and selection characteristics of the cell in the presence of an inhibitory peptide (protein marked with arrow indicating "peptide inhibitor") that specifically blocks the X/Y target interaction (top two rows), but not the non-target X/Z interaction (lower row). Features of the reporter genes and binding partners are as described above. Sun symbols indicate expression of the *cobA* reporter gene encoding a red fluorescent protein. As shown, the inhibitory peptide (peptide inhibitor) prevents interaction between the bait of the target interaction (LexA-Y) and prey (Prey-X/AD), such that there is no expression in the cell of the *URA3* or *CYH2* counter selectable reporter genes, or the *gfp* reporter gene, of the target X/Y interaction. Accordingly, the yeast strain is made resistant to 5-FOA and cycloheximide, however no longer fluoresces green. Additionally, the inhibitory peptide (peptide inhibitor) fails to inhibit the interaction between the prey (Prey-X/AD) and the bait of the non-target interaction (cl-Z), and ,as a consequence, the cells remain prototrophic for lysine biosynthesis, due to the activating interaction between the non-target bait and prey fusion proteins bound to the cl operator sites (clop) of the *LYS2* reporter gene which prototrophy can complement an auxotrophic *lys2* mutation in yeast. The strain also continues to express the *cobA* gene, and, as a consequence, fluoresces red.

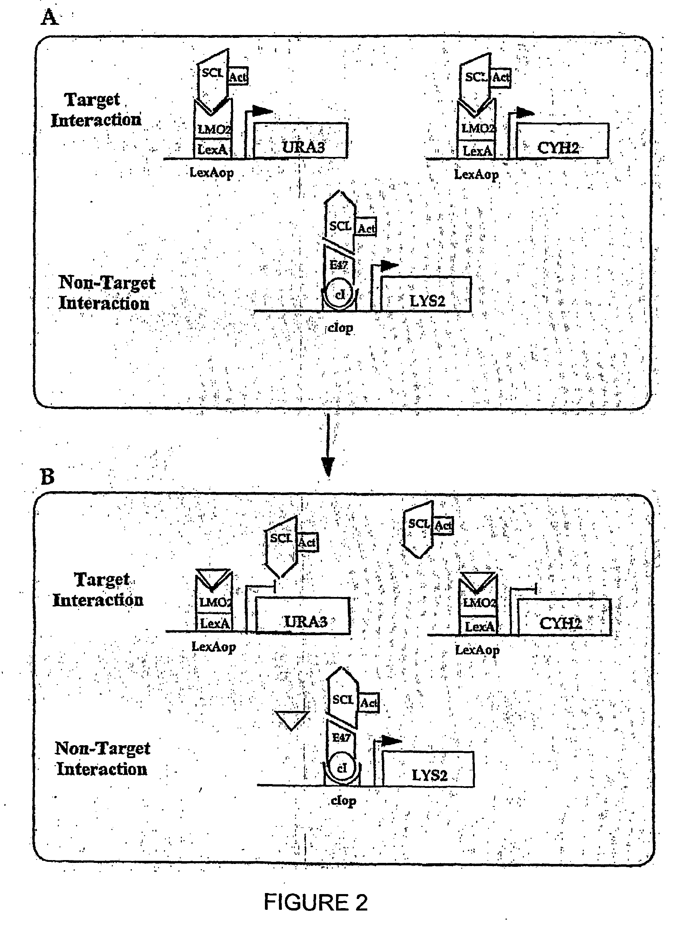

Figure 2 is a schematic representation of a reverse two hybrid screen for peptides that specifically antagonize the SCL/LMO2 interaction in yeast cells, however do not block the SCUE47 interaction.
   Panel A shows the expected expression profile and selection characteristics of the cell in the absence of antagonism of the target SCULM02 interaction and the non-target SCL/E47 interaction. The "prey" for both the target interaction (top row) and the non-target interaction (lower row) comprises SCL expressed as a fusion protein with the activation domain (Act) of a transcription factor (i.e. joined boxes marked SCL and Act). The "bait" for the target interaction (top row) comprises a fusion protein between LMO2 and the DNA binding domain of the LexA protein (i.e. joined boxes marked LMO2 and LexA). The reporter genes for the target interaction (top row) consist of chimeric *URA3* and *CYH2* genes comprising one or more *LexA* operator sequences to which the bait can bind. The non-target bait comprises a fusion protein between E47 (box marked E47) and the cl repressor protein (circle labeled cl). The reporter gene for the non-target interaction consists of a chimeric *LYS2* gene comprising one or more *cl* operator sequences to which the non-target bait can bind. Arrows indicate expression of the reporter genes. Accordingly, the yeast strain is sensitive to 5-FOA and cycloheximide, due to the activating interaction between the target bait and prey fusion proteins bound to the LexA operator site(s) of the counter selectable reporter genes *URA3* and *CYH2;* and prototrophic for lysine biosynthesis, due to the activating interaction between the non-target bait and prey fusion proteins bound to the cl operator sites (clop) of the LYS2 reporter gene which prototrophy can complement an auxotrophic *lys2* mutation in yeast.
   Panel B shows the expected expression profile and selection characteristics of the cell in the presence of an inhibitory peptide (Δ) that specifically blocks the SCL/LMO2 target interaction (top row), but not the non-target SCUE47 interaction (lower row). Features of the reporter genes and binding partners are as described above. Arrows indicate expression of the reporter genes. Blunt-ended lines (}) indicate an absence of detectable reporter gene expression. Accordingly, following introduction of an inhibitory peptide aptamer into the cell of panel A, the phenotype of the strain is converted to 5-FOA-resistant, and cycloheximide-resistant, due to inhibition of the activating interaction between the target bait bound to the LexA operator site(s) of the counter selectable reporter genes (*URA3* and *CYH2*) and the prey fusion protein; and prototrophic for lysine biosynthesis, due to the activating interaction between the non-target bait and prey fusion proteins bound to the cl operator sites (clop) of the *LYS2* reporter gene, which prototrophy can complement an auxotrophic *lys2* mutation in yeast.

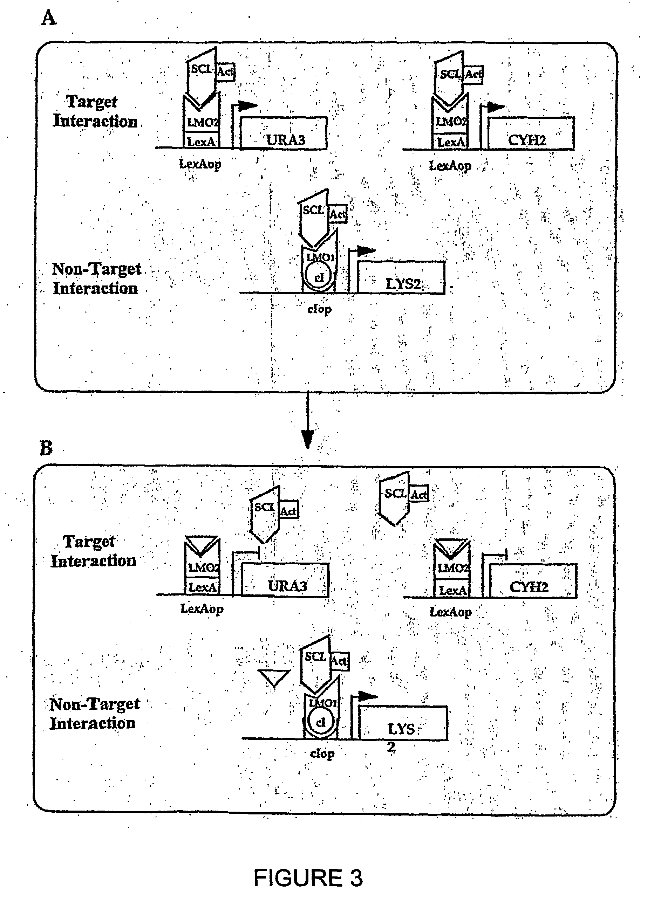

Figure 3 is a schematic representation of a reverse two hybrid screen for peptides that specifically antagonize the SCL/LMO2 interaction in yeast cells, however do not block the SCL/LMO1 interaction.
   Panel A shows the expected expression profile and selection characteristics of the cell in the absence of antagonism of the target SCULMO2 interaction and the non-target SCUE47 interaction. The "prey" for both the target interaction (top row) and the non-target interaction (lower row) is as described for Figure 1 *supra.* The "bait" and reporter genes for the target interaction (top row), and the reporter gene for the non-target interaction (lower row), are also as described for Figure 1 *supra.* The non-target bait comprises a fusion protein between LMO1 (box marked LMO1) and the cl repressor protein (circle labeled cl). Arrows indicate expression of the reporter genes. Accordingly, the yeast strain is sensitive to 5-FOA and cycloheximide, due to the activating interaction between the target bait and prey fusion proteins bound to the LexA operator site(s) of the counter selectable reporter genes *URA3* and *CYH2;* and prototrophic for lysine biosynthesis, due to the activating interaction between the non-target bait and prey fusion proteins bound to the cl operator sites (clop) of the *LYS2* reporter gene which prototrophy can complement an auxotrophic *lys2* mutation in yeast.
   Panel B shows the expected expression profile and selection characteristics of the cell in the presence of an inhibitory peptide (Δ) that specifically blocks the SCULM02 target interaction (top row), but not the non-target SCL/LMO1 interaction (lower row). Features of the reporter genes and binding partners are as described above and in the legend to Figure 1. Arrows indicate expression of the reporter genes. Blunt-ended lines (}) indicate an absence of detectable reporter gene expression. Accordingly, following introduction of an inhibitory peptide aptamer into the cell of panel A, the phenotype of the strain is converted to 5-FOA-resistant, and cycloheximide-resistant, due to inhibition of the activating interaction between the target bait bound to the LexA operator site(s) of the counter selectable reporter genes (*URA*3 and *CYH2*) and the prey fusion protein; and prototrophic for lysine biosynthesis, due to the activating interaction between the non-target bait and prey fusion proteins bound to the cl operator sites (clop) of the LYS2 reporter gene, which prototrophy can complement an auxotrophic *lys2* mutation in yeast.

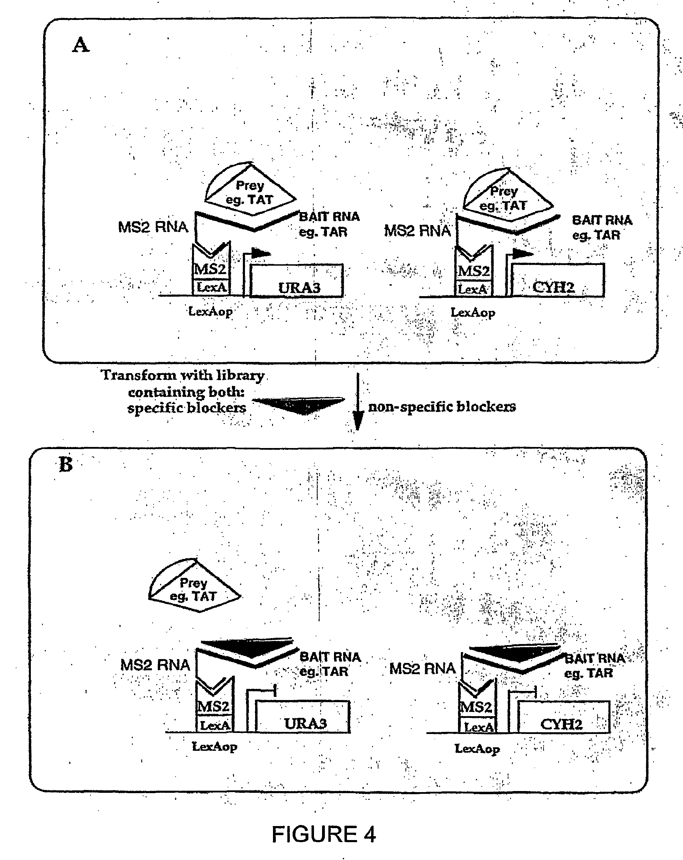

Figure 4 is a schematic representation of a reverse three hybrid screen for peptides that specifically inhibit the interaction between nucleic acid and protein in yeast cells.
   Panel A shows the expected expression profile and selection characteristics of the cell in the absence of antagonism of the target interaction. The non-target interaction is not indicated in the Figure. The "prey" for both the target interaction (and any non-target interaction, not indicated) is an RNA binding protein (TAT) expressed as a fusion protein with the activator domain of a transcription factor. The assay employs two "baits" for the target interaction comprising the following: (i) a hybrid MS2/TAR RNA molecule comprising at least the protein-binding regions of the MS2 coat protein-encoding RNA and TAR-encoding RNA that binds to TAT; and (ii) a fusion protein between MS2 and the DNA binding domain of the LexA protein (i.e. joined boxes marked MS2 and LexA). The reporter genes for the target interaction consist of chimeric *URA3* and *CYH2* genes comprising one or more LexA operator sequences to which the bait can bind. The non-target bait (not shown) comprises a fusion protein between any other protein to which the MS2/TAR hybrid RNA can bind, (either through the MS2-binding nucleotide sequence moiety or the TAT-binding sequence) and the cl repressor protein. The reporter gene for the non-target interaction (not shown) consists of a chimeric *LYS2* gene comprising one or more *cl* operator sequences to which the non-target bait can bind. Arrows indicate expression of the reporter genes. Accordingly, the yeast strain is sensitive to 5-FOA and cycloheximide, due to the interaction between the target baits (LexA/MS2 protein and MS2/TAR RNA) and the prey fusion protein (i.e. the TAT-activator fusion) at the LexA operator site(s), which interaction activates expression of the counter selectable reporter genes *URA3* and *CYH2*. Additionally, the strain is prototrophic for lysine biosynthesis, due to the interaction between the non-target bait and prey fusion proteins bound to the cl operator sites (not shown), which interaction activates the expression of the *LYS2* reporter gene.
   Panel B shows the expected expression profile and selection characteristics of the cell in the presence of an inhibitory peptide (▼) that specifically blocks the RNA/protein target interaction, but not the non-target RNA/protein interaction (not shown). Features of the reporter genes and binding partners are as described above. Blunt-ended lines (}) indicate an absence of detectable reporter gene expression. Accordingly, following introduction of an inhibitory peptide aptamer into the cell of panel A, the phenotype of the strain is converted to 5-FOA-resistant, and cycloheximide-resistant, due to inhibition of the activating interaction between the prey TAT-activator fusion protein and the target MS2/TAR bait RNA, said inhibition preventing expression of the counter selectable reporter genes (*URA3* and *CYH2*). The strain is also prototrophic for lysine biosynthesis, due to the continued activating interaction between the non-target baits and the prey fusion protein at the cl operator sites of the LYS2 reporter gene.

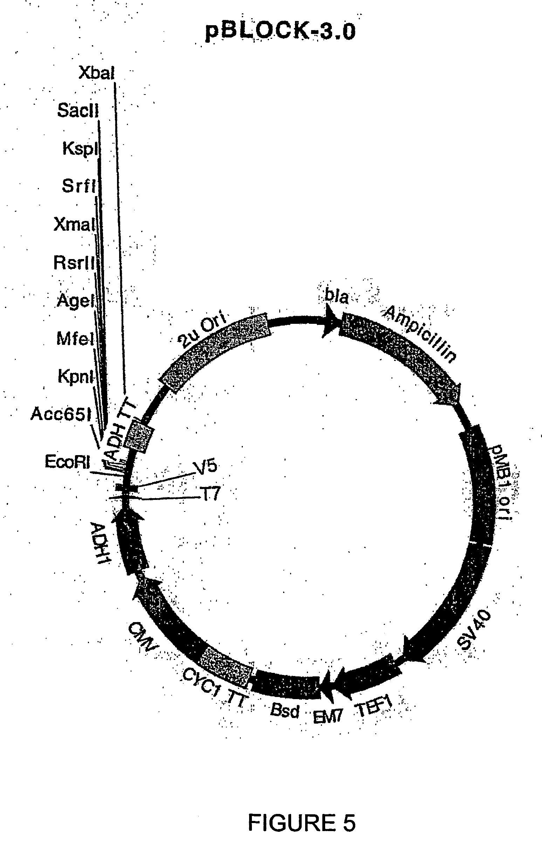

Figure 5 is a schematic representation of the vector pBLOCK-3.0 (SEQ ID NO:1) for expressing a peptide in a cell comprising:
   (i) an expression cassette comprising: (a) a multiple cloning site having the restriction sites *Eco*RI, *Acc*651, *Kpn*l*, Mfe*l, *Age*l, *Rsr*II, *Xma*l, *Srf*I, *Ksp*l, and *Sac*ll, for insertion of a nucleotide sequence encoding said peptide, wherein said multiple cloning site is adjacent to a sequence encoding the V5 epitope tag and SV40 nuclear localization sequence (not shown) such that a fusion polypeptide comprising said peptide and said epitope tag and nuclear localization sequence can be expressed; (b) the tandem promoters *T7, ADH1,* and *CMV*, for regulating expression of said fusion polypeptide in the cells of bacteria, yeast, and mammals, respectively; and (c) a terminator sequence (ADH TT) between said multiple cloning site and an Xbal site, and distal to said tandem promoters;
   (ii) a selectable marker gene (*Bsd*) for conferring resistance to basticidin in yeast and bacteria operably under the control of tandem yeast and bacterially-operable promoters *TEF1* and *EM7*, and placed upstream of the yeast-operable transcription terminator CYC1 (CYC TT);
   (iii) a selectable marker gene (ampicillin) for conferring resistance to the antibiotic ampicillin in bacteria operably under the control of the *bla* promoter,
   (iv) a bacterial origin of replication (pMB1 ori); and
   (v) a eukaryotic origin of replication (2 µ Ori).

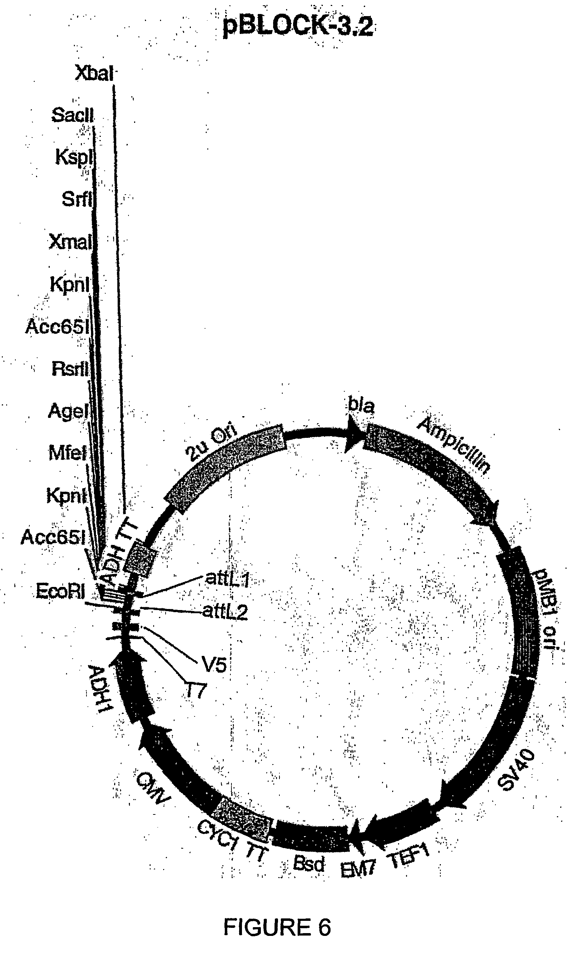

Figure 6 is a schematic representation of the vector pBLOCK-3.2 (SEQ ID NO: 2) for expressing a peptide in a cell comprising the features of pBLOCK-3.0 (SEQ ID NO: 1) as described in the legend to Figure 5, except that the multiple cloning site has the restriction sites *Eco*RI, *Acc*651, *Kpn*l, *Mfe*l*, Age*l, *Rsr*ll*, Acc*651*, Kpn*l*, Xma*l, *Srf*l*, Ksp*l, and *Sac*ll, and is flanked by bacteriophage λ integration sites (attL1 and attL2) adjacent to a sequence encoding the V5 epitope tag such that a fusion polypeptide comprising said peptide and said epitope tag is capable of being expressed and nucleic acid encoding the peptide (not encoding said fusion polypeptide) is subsequently able to be excised by homologous recombination about said integration sites.

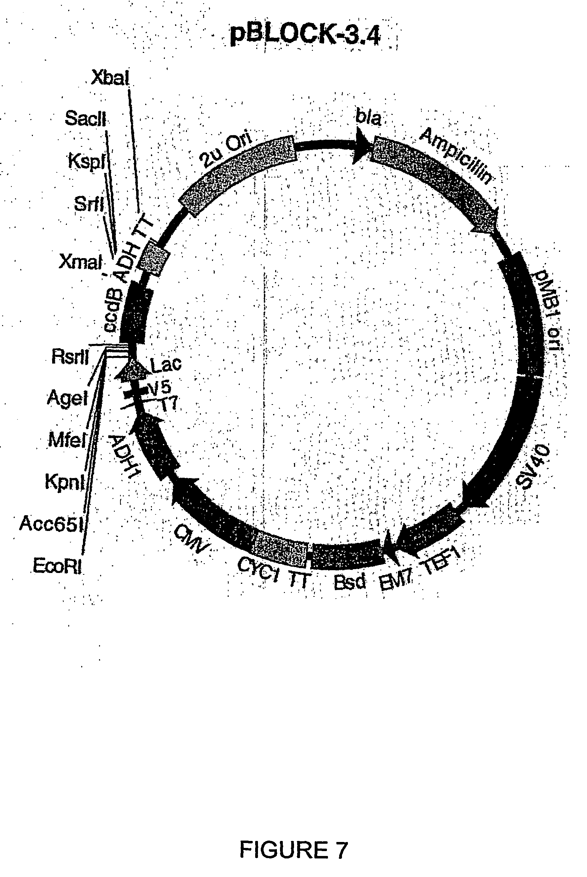

Figure 7 is a schematic representation of the vector pBLOCK-3.4 (SEQ ID NO: 3). for expressing a peptide in a cell comprising the features of pBLOCK-3.0 (SEQ ID NO:1) as described in the legend to Figure 5, except that the expression cassette comprises the counter selectable reporter gene *ccdB* cloned between the *Rsr*II and *Xma*I sites of the multiple cloning site, and in operable connection with a *lac* promoter placed upstream of the *Eco*RI-*Rsr*II portion of the multiple cloning site. Accordingly, the vector is conditionally lethal in bacterial cells, unless the chimeric *Lac-ccdB* gene is disrupted by insertion of nucleotide sequences encoding the peptide to be expressed, within the *EcoR*I-*Rsr*II portion of the multiple cloning site.

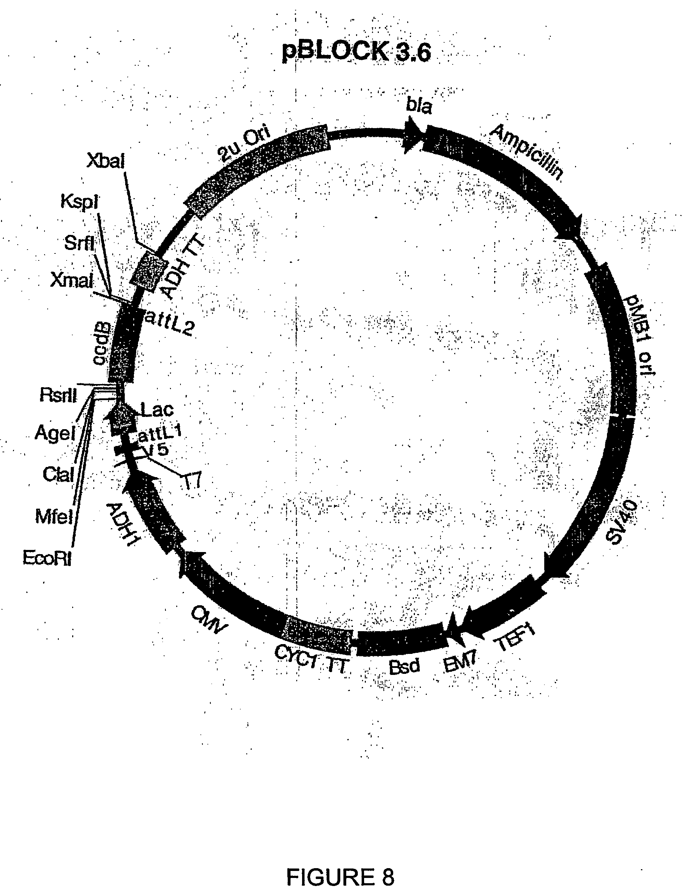

Figure 8 is a schematic representation of the vector pBLOCK-3.6 (SEQ ID NO: 4) for expressing a peptide in a cell, which comprises the features of pBLOCK-3.4 (SEQ ID NO: 3) as described in the legend to Figure 7, except that (i) the multiple cloning site for inserting the peptide-encoding sequence has the unique restriction sites *EcoR*I, *Mfe*l, *Cla*I, Agel and Rsrll; and (ii) the chimeric *lac*- ccdB gene is flanked by bacteriophage A integration sites (attL1 and attL2) between the sequence encoding the V5 epitope tag (V5) and the ADH terminator (ADSH TT) to facilitate excision.

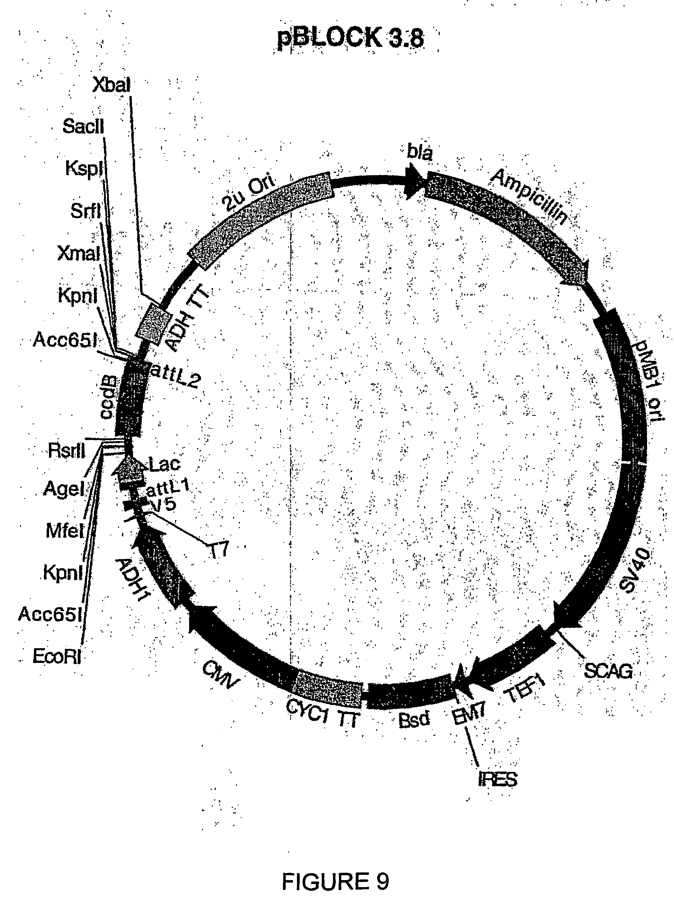

Figure 9 is a schematic representation of the vector pBLOCK-3.8 (SEQ ID NO: 5) for expressing a peptide in a cell, which vector comprises the features of pBLOCK-3.6 (SEQ ID NO: 4) as described in the legend to Figure 8, and further includes: (i) a single chain antibody-encoding gene (SCAG) placed operably under the control of the SV40 promoter, and (ii) an internal ribosome entry site sequence (IRES) placed between the EM7 promoter and the *Bsd* gene.

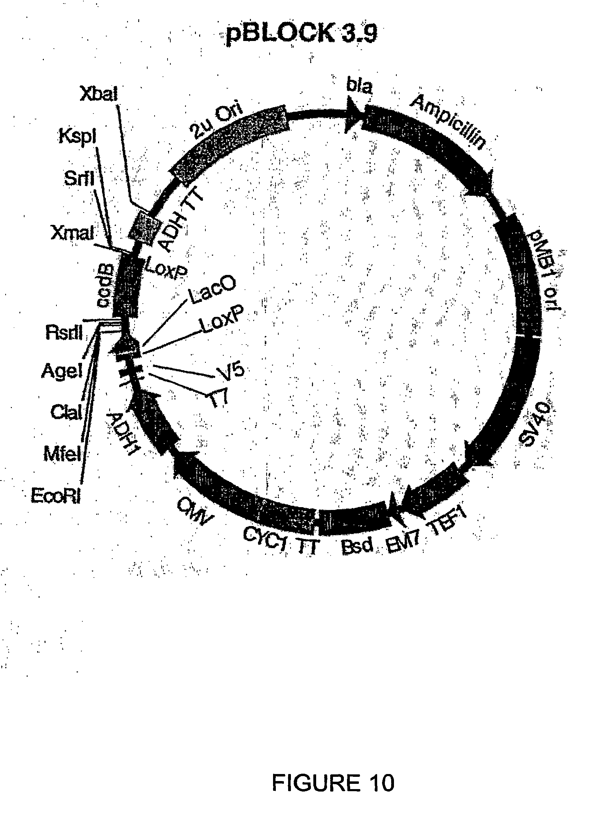

Figure 10 is a schematic representation of the vector pBLOCK-3.9 (SEQ ID NO: 6) for expressing a peptide in a cell, which vector comprises the features of pBLOCK-3.6 (SEQ ID NO: 4) as described in the legend to Figure 8, however the bacteriophage A integration sites (attL1 and attL2) of pBLOCK-3.6 are replaced by LoxP sites to facilitate Cre/*loxP*-mediated excision of the chimeric *lac-ccdB* gene.

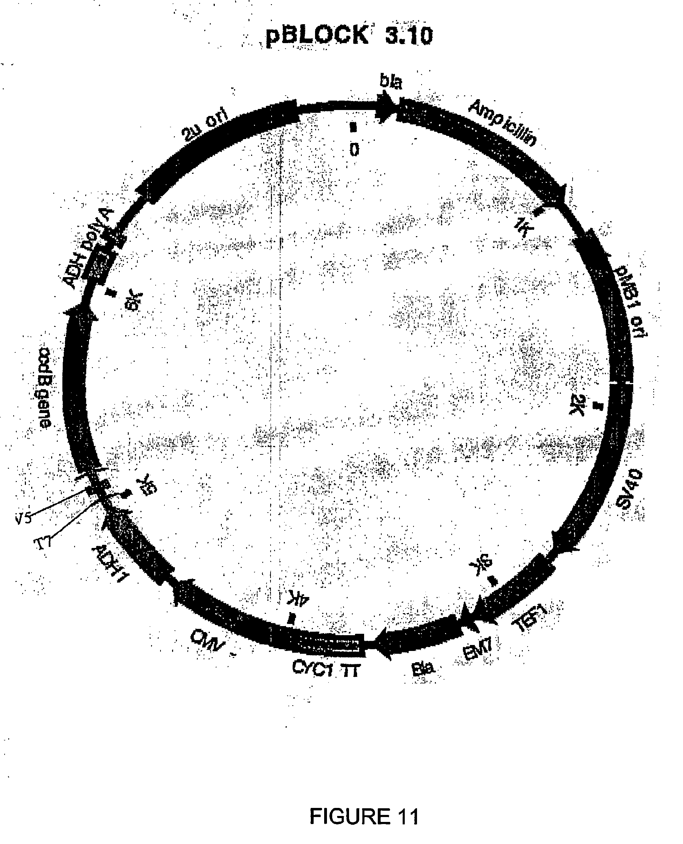

Figure 11 is a schematic representation of the vector pBLOCK-3.10 (SEQ ID NO: 7) for expressing a peptide in a cell, which vector comprises the features of pBLOCK-3.4 (SEQ ID NO: 3) as described in the legend to Figure 7.
Figure 92 is a schematic representation of the vector pBLOCK-3.11 (SEQ ID NO: 8) for simultaneously expressing up to three distinct peptides in the same cell and from the same vector, which vector comprises the features of pBLOCK-3.0 (SEQ ID NO:1) as described in the legend to Figure 5, however contains translation start ATG codons linked in-frame to sequences encoding the epitope and SV40 nuclear localization domains, in each of the three possible forward reading frames. More particularly, the epitope encoding sequences in reading frames 1-3 are *c-myc,* FLAG, and V5, respectively.
Figure 13 is a schematic representation of the vector pBLOCK-4.0 (SEQ ID NO: 9) for expressing a peptide in a cell, which vector comprises the features of pBLOCK-3.0 (SEQ ID NO:1) as described in the legend to Figure 5, except that the gene encoding blasticidin resistance (Bsd) is substituted for a sequence (G418) that encodes a protein conferring resistance to kanamycin in bacteria or resistance to gentamycin in eukaryotic cells.
Figure 14 is a schematic representation of the vector pRT2 (SEQ ID NO: 10) containing the following features:
   (i) a first fluorescent reporter gene cassette comprising the *gfp* gene encoding green fluorescent protein placed operably under control of a chimeric yeast operable *LexA*/*GAL1* promoter having 8 *LexA* operator sites, and upstream of the yeast *ADH1* terminator;
   (ii) a second fluorescent reporter gene cassette comprising the *cobA* gene encoding a red fluorescent protein placed operably under control of a chimeric *cl*/*GAL1* promoter having 3 cl operator sites;
   (iii) a wild-type yeast operable selectable marker gene (*ADE2*) for conferring adenine auxotrophy on cells expressing said gene;
   (iv) a selectable marker gene for conferring resistance to the antibiotic kanamycin in bacteria;
   (v) a bacterial origin of replication (colE1); and
   (vi) a eukaryotic origin of replication (2 µ Ori).

The present invention clearly extends to the use of the methods and vectors described herein to identify novel drugs, such as, for example, antibiotics or inhibitory agents. In fact, the present invention is particularly useful in drug screening protocols to identify candidate agonists and antagonists of any biological interaction. For example, bacterial expression systems may be used in high through-put screening for novel antibiotics or other inhibitory agents which target specific protein: DNA or protein: protein interactions. The pBLOCK series of vectors described herein are particularly useful in such applications.

### DETAILED DESCRIPTION OF THE PREFERRED EMBODIMENTS

This invention provides a modified reverse two hybrid screen compared to that described in Intemational Patent Application No. PCT/AU99/00018 (WO 99/35282), In particular, whilst the reverse two hybrid screen for peptides that inhibit a target interaction between two proteinaceous binding partners can be performed as described previously, an additional selection is made for peptides that do not inhibit a non-target interaction between one of said proteinaceous binding partners and another binding partner. This means that the bait and prey constructs, and the reporter gene constructs for assaying the target interaction can be any of those described previously. However, to assay the non-target interaction, the host cell must contain an additional "bait" construct and reporter gene construct other than that used to assay the target interaction, for assaying the non-target interaction.

Additionally, it is particularly preferred for dual reporter genes to be operably and separately connected to the target interaction in the host cell, to reduce background. For example, two counter selectable reporter genes can be used for the target interaction to eliminate the potential background of 5-FOA-resistant colonies that arise from mutations in the *URA3* reporter gene.

Preferably, the yeast strain is modified to allow the introduction of an additional plasmid expressing a candidate peptide being assayed for inhibitory activity against the target interaction but not the non-target interaction.

The development of new and improved reverse n-hybrid screens that are particularly useful for high-throughput screening of peptide aptamer inhibitors of protein-protein interactions is described below. The detailed description of the preferred embodiments presented herein is for the purposes of exemplification only and should not be construed as placing a limitation on the presently described invention.

### EXAMPLE 1

### Generalized scheme of improved reverse two hybrid screen

A generalized scheme for the reverse two hybrid screen of the invention is presented in Figure 1. In this embodiment, gene constructs for the target interacting partners (designated X and Y in Figure 1) are prepared, based on the vectors pJG4-5 and pGilda that have been described for use with the *lexA*-based two hybrid system (Gyuris *et al,* 1993; Ausubel,1989). The coding region of the prey, PROTEIN-X, is expressed as an activation domain fusion. The coding region of the target bait, PROTEIN-Y, is expressed as a LexA fusion. In this scheme, a non-target interaction between the prey PROTEIN-X and a non-target bait, PROTEIN-Z (designated Z in Figure 1) is also assayed. The non-target bait, PROTEIN-Z, is expressed as a cl-repressor fusion from pGMS13; a derivative of pGMS12 (Serebriiskii, 1999) that contains *HIS5* in place of the G418 selectable marker gene.

Alternative cl-bait vectors include pGKS8, pGMS12 (Serebriiskii, 1999) or pCIAuri (a derivative of the pAUR112 CEN/ARS vector (Panvera corporation), which contains a *GAL1* promoter Cl-repressor and *ADH1* terminator in place of the URA3 gene), can also be used. These vectors contain alternative markers allowing selection for Zeocin, G418, or Auriobasidin resistance, respectively.

The reporter genes for the target interaction between PROTEIN-X and PROTEIN-Y consist of two chimeric counter selectable reporter genes *URA3* and *CYH2,* and the green fluorescent protein-encoding *gfp* gene (Prasher *et a*/*.,* 1992; Chalfe *et al.*, 1994; Inouye and Tsuji, 1994), each comprising one or more *LexA* operator sequences to which the target bait can bind. It should be noted that the *CYH2* selection requires a cycloheximide resistant yeast background containing the *cyh2*^{*R*} allele. The reporter genes for the non-target interaction include the single chimeric counter selectable reporter gene *LYS2,* and the red fluorescent protein-encoding *cobA* gene (Wildt and Deuschle,1999), each comprising one or more *cl* operator sequences to which the non-target bait can bind.

Yeast cells are transformed with gene constructs expressing the prey, target bait, non-target bait, and all reporter gene constructs, and these features are then recombined into single cells by mass mating (Feilotter *et al,* 1994). The prey, and the target and non-target baits are all expressed from the CEN/ARS-based *GAL1* expression vectors, pGilda or pCIAuri. Clearly, this method is not restricted to the bait being expressed from such plasmid vectors, since the expression cassettes could be integrated into the genome without adverse effects.

Vectors marked with *TRP1, HlS3,* and *HIS5,* encoding the prey, target bait, and non-target bait are transformed into yeast strain PRT473; a cycloheximide resistant derivative of strain SKY473 having the genotype (*MAT-*a, *cyh2*^{R} *his*3*, trp*1*, lexAop-LEU2, lexAop-CYH2ade2::ZEO*^{R}*, lexAop-URA3his5::G418*^{R}, *clop*-*LYS*). The recombinants are screened in parallel for the interacting target and non-target partners.

Because the binding partners of the target interaction are expressed under control of the galactose-inducible *GAL1* promoter, it is possible to fine-tune their expression relative to the presence of 5-FOA and cycloheximide in the cell growth media. The minimum level of induction required to cause lethality is determined, as follows: The diploid SKY48/SKY473 strain containing the interacting target bait and prey fusion proteins is tested for growth on a panel of selection media, containing 2% (w/v) raffinose (which is neutral with respect to *GAL1* induction), pre-determined concentrations of cycloheximide and 5-FOA, and an amount of galactose in the range 0% (w/v) galactose to 2% (w/v) galactose. The minimum concentration of galactose required for complete lethality of this strain in the presence of 5-FOA and cycloheximide is then determined.

In the absence of any inhibition, the yeast strain is sensitive to the presence of 5-FOA and cycloheximide in growth medium following induction by galactose, due to the interaction between the target bait and prey fusion proteins bound to the LexA operator site(s) of the counter selectable reporter genes *URA3* and *CYH2*. The same interaction makes the yeast cell fluoresce green, due to the expression of the *gfp* gene. Additionally, interaction between the non-target bait and the prey makes the cell prototrophic for lysine biosynthesis, as a consequence of *LYS2* gene expression. Finally, the cell also fluoresces red as a consequence of the interaction between the non-target bait and prey fusion proteins activating *cobA* reporter gene expression. The phenotype of the cell in the absence of any inhibition is shown in Panel A of Figure 1.

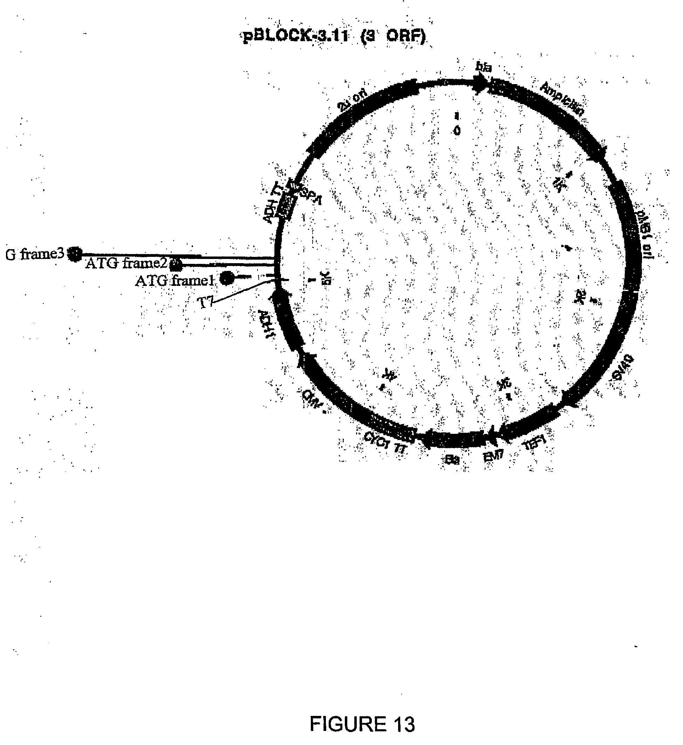

To screen for specific peptide inhibitors of the target interaction, a further gene construct, in particular any one of the pBLOCK-3 series of vectors shown in Figures 5-12, or the pBLOCK-4.0 vector shown in Figure 13, is modified to express a peptide aptamer. Preferably, a library of peptide aptamers is produced using such vectors. Individual recombinant DNAs of the library are separately introduced into PRT48; a cycloheximide resistant yeast strain derived from strain SKY48 (Serebriiskii, 1999), having the genotype: *(MATa, leu2, ade2, ura3, his3, his5 lys2 trp1, cyh2*^{*R*}*).* The transformants are frozen until required.

The library is introduced into the recombinant PRT 473 strains expressing the binding partners (see above) by mass mating. Diploid yeast cells arising from the mass mating of recombinants derived from strains PRT 473 and PRT 48 are selected on minimal media containing Blasticidin (pBLOCK-3 series of vectors) or G418 (pBLOCK-4.0 vector) and Zeocin, but lacking leucine, tryptophan and histidine, and stored frozen as glycerol stocks.

Screening of the library is then performed by plating the yeast strains, at 10-fold the original library complexity, on minimal media lacking leucine, tryptophan, and histidine, but containing blasticidin (derivatives of the pBLOCK-3.0 series of vectors) or G418 (pBLOCK-4.0 vector derivatives), 5-FOA and/or cycloheximide. Induction of gene expression is achieved by adding the appropriate amount of galactose as determined *supra* to the growth media.

Panel B of Figure 1 shows the expected expression profile and selection characteristics of the transformed yeast cells expressing peptide aptamers. Where the inhibitory peptide aptamer prevents interaction between the target bait and prey, no expression of the *URA3* or *CYH2* counter selectable reporter genes, or the *gfp* reporter gene, is detected following galactose induction. Accordingly, the yeast strain is made resistant to 5-FOA and cycloheximide, however no longer fluoresces green. On the other hand, cells that do not express an inhibitory peptide with respect to the target interaction will not survive on media containing 5-FOA and/or cycloheximide.

If the inhibitory peptide fails to inhibit the interaction between the prey and the non-target bait, the surviving cells remain prototrophic for lysine biosynthesis, due to the activating interaction between the non-target bait and prey fusion proteins bound to the cl operator sites of the *LYS2* reporter gene, which prototrophy can complement an auxotrophic *lys2* mutation in yeast. The strain will also continue to express the *cobA* gene, and, as a consequence, fluoresces red. On the other hand, if the inhibitor peptide also inhibits the non-target interaction, the surviving cells will not grow on media lacking lysine, and will not fluoresce red.

Accordingly, specificity of the inhibition of reporter gene expression is determined by replica plating colonies onto the identical media and media lacking lysine. Colonies that grow in the presence of 5-FOA and cycloheximide, however fail to grow on media lacking lysine express peptide inhibitors that inhibit both the target and non-target interactions. Colonies that grow in the presence of 5-FOA and cycloheximide, and on media lacking lysine express peptide inhibitors that specifically inhibit the target interaction.

### EXAMPLE 2

### Reverse two hybrid assay for peptide inhibitors of the SCL/LMO2 interaction

It will be apparent to the skilled worker how to vary the protocol provided in the preceding example, to assay for peptide inhibitors of a particular protein-protein interaction, using the reverse two hybrid screen of the invention. The following modifications are made to the preceding example.

Two parallel *reverse* two hybrid screens are performed, as shown in Figures 2 and 3. The first screen (Figure 2) identifies peptide inhibitors of a target interaction between the prey, SCL, and a target bait, LM02, that do not inhibit a non-target interaction between said prey and a non-target bait, E47. The interaction between SCL and E47 has been previously described in Mahajan *et al* (1996). The second screen (Figure 3) identifies peptide inhibitors of the same target interaction that do not inhibit another non-target interaction between the prey, SCL, and the non-target bait, LM01. Accordingly, both non-target interactions can be distinguished from the target interaction. Clones expressing peptide inhibitors of the target interaction alone, or the target interaction and one or both of the non-target interactions can also be identified.

The SCL prey is an existing activation domain/SCL fusion protein, containing the bHLH domain (residues 176-245) of SCL that have been implicated in the interaction of LMO2 (Wadman *et al*., 1994).

Three counter selectable reporter genes are employed in each two hybrid screen. The first two reporter genes, *URA3* and *CYH2*, are under the control of LexA operators and are therefore dependent on the target interaction. This dependence is because the target bait, LMO2, is expressed as a lexA fusion protein in both screens (Figure 2 and Figure 3). As with the previous example, these screens each exploit the toxicity of the URA3 gene product in the presence of the drug 5-fluoro-orotic acid (5-FOA), and the toxicity of the wild-type *CYH2* gene product in the presence of the drug cycloheximide. Accordingly, any activation of reporter gene expression arising from the target interaction between SCL and LMO2 is selected against in the presence of the drugs 5-FOA and cycloheximide, as described in the preceding example.

The counter selectable reporter genes for detecting non-target interactions (i.e. SCL/E47 and SCL/LMO1) is the LYS2 gene which is under the control of cl operator sequences (Figure 2 and Figure 3). Expression of the LYS2 reporter gene depends on binding of the non-target bait proteins, E47 (Figure 2) and LMO1 (Figure 3), which binding is achieved by expressing these non-target baits as fusion proteins with cl operator DNA-binding domains.

Absent any inhibition of SCL and LMO2 binding in either screen, and subsequent inhibition of *URA3* and *CYH2* reporter gene expression, the yeast strains are sensitive to 5-FOA and cycloheximide following induction by galactose. In contrast, when an inhibitory peptide aptamer prevents interaction between SCL and LM02, no expression of the URA3 or *CYH2* counter selectable reporter genes is detected following galactose induction, and, as a consequence, the yeast strain is made resistant to 5-FOA and cycloheximide. Cells that do not express an inhibitory peptide with respect to the target interaction do not survive on media containing 5-FOA and/or cycloheximide.

The cells of both screens that survive on 5-FOA and cydoheximide are assayed further for their lysine requirements, as described in the preceding example. Absent inhibition of the SCL/E47 interaction (Figure 2) or the SCL/LMO1 interaction (Figure 3), the cells are prototrophic for lysine. Accordingly, those cells that survive of 5-FOA and cycloheximide are also prototrophic for lysine biosynthesis, the interaction between SCL and E47 (Figure 2), or between SCL and LMO1 (Figure 3), is not inhibited, and the inhibitory peptide is specific for the target interaction between SCL and LMO2. On the other hand, in either of the parallel screens, the yeast strains that grow on 5-FOA and cycloheximide, but do not grow on media lacking lysine, express an inhibitory peptide that also inhibits the particular non-target interaction in question (i.e. the SCL/E47 interaction or the SCL/LMO1 interaction). Accordingly, it is possible to determine those peptide aptamers that inhibit one or two or three of the interactions assayed that involve SCL. Those skilled in the art will readily be able to assay additional interactions involving the SCL protein, by performing additional screens, each having a different non-target bait, in the manner described herein.

### EXAMPLE 3

### Reverse three hybrid screen for peptide inhibitors of an RNA/protein interaction

Reverse three hybrid screens are described in international Patent Application No. PCT/AU99/00018 (WO 99/35282).

In the reverse three hybrid method described herein, a constant bait construct is required that expresses a DNA binding domain, such as, for example, GAL4 or LexA, as an in-frame fusion with an RNA-binding protein, such as, for example, the coat protein MS2 (Invitrogen) which contains an RNA-binding cleft. Preferably, this construct is integrated (Invitrogen). More preferably, the construct is contained on a yeast plasmid containing the 2 micron or CEN/ARS origin of replication.

Additionally, a second RNA-bait construct is required that expresses a hybrid RNA comprising an RNA target and the ligand for the RNA-binding protein of the constant bait. For example, a hybrid RNA-encoding gene comprising MS2 RNA sequences can be used to bind to an MS2 coat protein domain of the constant bait Examples of such RNA-bait constructs include the vectors pRH3' and pRH5' (Invitrogen).

Additionally, a specific prey construct is also required, expressing the protein that interacts with the target RNA of interest, fused to a transcriptional activation domain. This prey construct is of a standard form, as for two hybrid screening, such as, for example, pJG4-5, or pYESTrp2, or pACT2. Preferably, the prey construct is pJG4-5. More preferably, the prey construct is pYESTrp2.

In other respects, the principles for three performing reverse hybrid screens are essentially as described herein for reverse two hybrid screens.

To screen for candidate peptide inhibitors of the RNA/protein interaction, a peptide library expressed from a vector such as the pBLOCK-3 series of vectors described herein (Figure 5-12) or pBLOCK-4.0 (Figure 13) can be used. The recombinant library is transformed into a yeast strain expressing the constant bait protein, the RNA bait and the prey protein in the manner described for the preceding examples.

A non-limiting example of the reverse three hybrid screen of the invention is present in Figure 4. In this screen, inhibitors of the target interaction between the TAT protein and RNA encoding TAR protein are identified, and selected preferentially over peptides that only bind to non-target interactions involving TAT protein, RNA encoding TAR protein, MS2 RNA, or MS2 protein.

The prey for both the target interaction (and any non-target interaction, not indicated) is an RNA binding protein (TAT) expressed as a fusion protein with an activator domain as described in the preceding examples. The RNA bait is a hybrid MS2/TAR RNA molecule comprising at least the RNA-binding cleft of the MS2 coat protein-encoding RNA and TAR-encoding RNA that binds to TAT. The constant bait is a fusion protein between MS2 protein and the DNA binding domain of the LexA protein. The reporter genes for the target interaction consist of chimeric *URA3* and *CYH2* genes comprising one or more LexA operator sequences to which the bait can bind, as described in the preceding examples.

The non-target bait comprises a fusion protein between any other protein to which the MS2/TAR hybrid RNA can bind, (either through the MS2-binding nucleotide sequence moiety or the TAT-binding sequence) and the cl repressor protein. The reporter gene for the non-target interaction consists of a chimeric LYS2 gene comprising one or more *cl* operator sequences to which the non-target bait can bind, as described in the preceding examples.

Expression of the *URA3* and *CYH2* counter selectable reporter genes requires the formation of a complex between the LexA operator and the LexA-MS2 fusion protein and the MS2-TAR hybrid RNA and the TAT-activator domain fusion protein. Accordingly, this interaction makes the cells susceptible to 5-FOA and cycloheximide following galactose induction of expression of the LexA-MS2 fusion protein and/or the TAT-activator domain fusion protein. Similarly, expression of the *LYS2* gene when a non-target interaction occurs in the cell makes the cell prototrophic for lysine. Accordingly, absent any inhibition of formation of the above complex, the yeast strain is sensitive to 5-FOA and cycloheximide, and prototrophic for lysine biosynthesis.

Inhibition of the target interaction between TAT protein and TAR-encoding RNA, will confer resistance to 5-FOA and cycloheximide on the cell. Such cells express a peptide that inhibits the target interaction. The peptide also inhibits a non-target interaction that normally activates *LYS2* reporter gene expression, when the cells require lysine for their growth. On the other hand, the peptide is specific for the target interaction when thew cells remain prototrophic for lysine.

### EXAMPLE 4

### Construction of new vectors for expressing candidate peptide inhibitors.

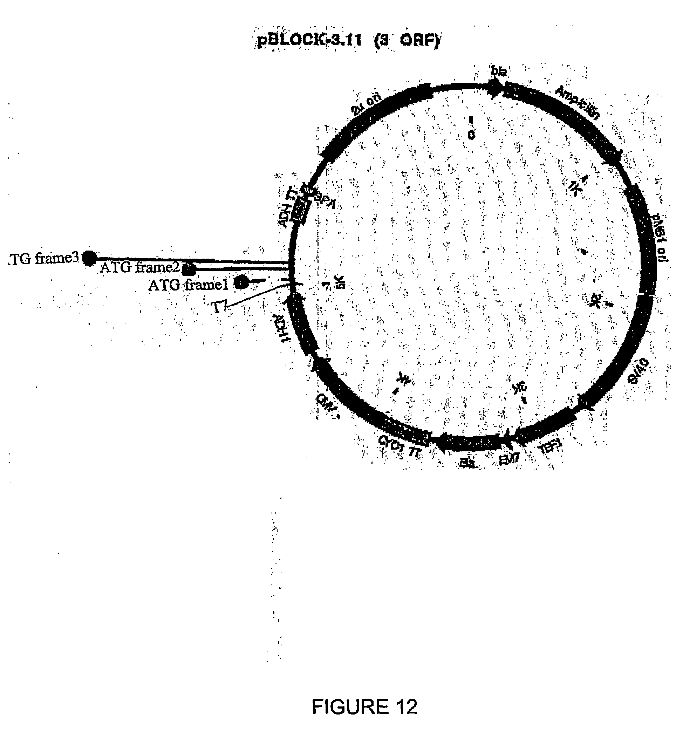

We have prepared a series of vectors suitable for expressing peptide aptamers to be screened in the reverse n-hybrid assays described herein. The pBLOCK-3 series of vectors described herein comprise the following vectors: pBLOCK 3.0 (Figure 5 and SEQ ID NO:1); pBLOCK-3.2 (Figure 6 and SEQ ID NO: 2); pBLOCK-3.4 (Figure 7 and SEQ ID NO: 3); pBLOCK-3.6 (Figure 8 and SEQ ID NO: 4); pBLOCK-3.8 (Figure 9 and SEQ ID NO: 5); pBLOCK-3.9 (Figure 10 and SEQ ID NO: 6); pBLOCK-3.10 (Figure 11 and SEQ ID NO: 7); and pBLOCK-3.11 (Figure 12 and SEQ ID NO: 8).

These vectors contain many features described for the vectors pBLOCK-1 and pBLOCK-2 as described in Intemational Patent Application No. PCT/AU99/00018 (WO 99/35282), including the tandem promoter features for expressing the candidate peptide in both prokaryotic and eukaryotic cells; and the presence of both *E.coli* and yeast replication origins to facilitate their use in both bacterial and yeast cells. As with pBLOCK-1 and pBLOCK-2, the constitutive strong ADH promoter drives expression of the candidate peptides in yeast, to allow high level expression that is independent of the carbon source. The SV40 nuclear localization sequence to direct the candidate peptide to the nucleus is also present. As with pBLOCK-1 and pBLOCK-2, the peptide is expressed as a fusion with an epitope tag (V5) to facilitate immune precipitation or Westem blotting of the expressed candidate peptide.

Additionally, the vectors described herein are small (approximately 6 kilobase pairs), to facilitate complex library construction.

All pBLOCK-3 series of vectors contain the Bsd gene conferring resistance to the drug Blasticidin in yeast and bacterial cells. The gene is flanked by both bacterial and yeast transcriptional control sequences enabling the rapid, specific rescue of library plasmids encoding candidate peptide inhibitors by transformation of *E.coli* and selection on rich media containing Blasticidin.

Particular derivatives of pBLOCK-3.0 incorporate a range of additional features, as described in the corresponding figure legends:
(i) A single chain antibody gene (SCAG) operably under control of the SV40 promoter (vector pBLOCK-3.8) allows detection of transfected eukaryotic cells using antibodies.
(ii) Site-specific recombinase sites (e.g. *frt, att, loxP, res*), flanking sequences encoding the candidate peptide in vectors pBLOCK-3.6, pBLOCK-3.8 and pBLOCK-3.9, facilitate rapid transfer of inserts from this vector into another vector via site specific recombination mediated by the corresponding recombinase enzymes (e.g. Flp recombinase, lambda integrase, Cre recombinase, and transposon resolvase, respectively).
(iii) positive selection genes encoding toxic products, in particular the *ccdB* gene operably under the control of the *lac* promoter (vectors pBLOCK-3.4, pBLOCK-3.6, pBLOCK-3.8, pBLOCK-3.9, pBLOCK-3.10, and pBLOCK-3.11) facilitates cloning and/or insert transfer by recombination.

Furthermore, the vector pBLOCK-4.0 (Figure 13 and SEQ ID NO: 9) is identical to the vector pBLOCK-3.4 except that the Bsd gene has been substituted with the *G418* gene that confers kanamycin resistance in bacteria and gentamycin resistance in eukaryotic cells.

The features of each plasmid are summarized in Table 1. Additional salient or unique features of each of these vectors will be apparent from the Sequence Listing and the drawings.

It is clear to those skilled in the art that the utility of the shuttle vectors such as those of the pBLOCK family are not restricted to reverse two hybrid screening. They are also useful for the cloning of a range of DNA molecules for a variety of purposes well known to those skilled in the art, including cDNA, genomic, PCR and oligonucleotide cloning (Ausubel *et al,* 1989). Minor modifications to the sequences of these pBLOCK vectors by means of small deletions, insertions and/or transitions or transversions, would not have a significant effect on the function of these vectors or to constitute an inventive step. Accordingly, all such equivalent vectors (i.e. variants or derivatives) may be used in the present invention.

**TABLE 1**

| **FEATURE** | **BLOCK.3.0** | **BLOCK 3.2** | **BLOCK 3.4** | **BLOCK 3.6** | **BLOCK 3.8** | **BLOCK 3.9** | **BLOCK 3.10** | **BLOCK 3.11** | **BLOCK 4.0** |
|---|---|---|---|---|---|---|---|---|---|
| ORIGINS OF REPLICATION | | | | | | | | | |
| **2 µm ori** | 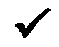 | 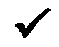 | 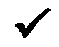 | 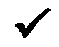 | 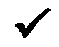 | 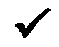 | 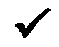 | 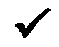 | 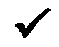 |
| **pMB1 ori** | 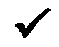 | 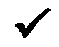 | 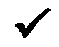 | 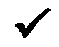 | 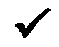 | 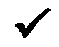 | 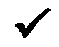 | 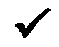 | 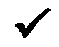 |
| **SELECTABLE MARKER GENE** | | | | | | | | | |
| ***bsd* gene** | 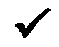 | 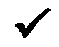 | 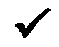 | 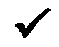 | 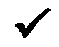 | 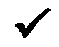 | 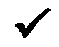 | 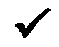 | |
| ***g418* gene** | | | | | | | | | 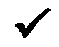 |
| **amp**^{**r**} **gene** | 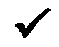 | | 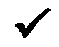 | 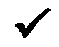 | 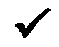 | 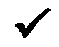 | 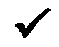 | 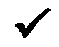 | 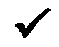 |
| **TANDEM PROMOTERS** | 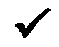 | 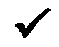 | 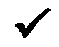 | 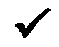 | 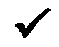 | 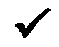 | 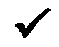 | 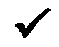 | 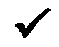 |
| **CMV/ADH/T7** | 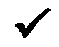 | 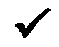 | 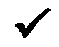 | 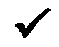 | 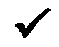 | 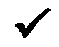 | 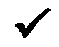 | 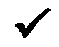 | 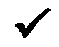 |
| **SV40/TEF/EM7** | 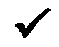 | 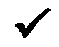 | 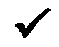 | 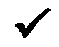 | 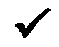 | 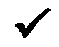 | 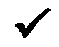 | 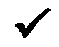 | 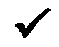 |
| **TOXIN GENES** | | | | | | | | | |
| ***lac-ccdB*** | | | 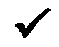 | 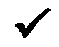 | 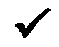 | 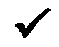 | 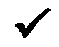 | 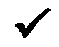 | 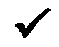 |
| **EPITOPE TAGS** | | | | | | | | | |
| **V5 epitope** | 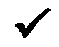 | 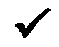 | 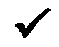 | 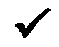 | 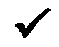 | 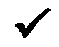 | 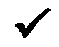 | 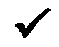 | 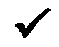 |
| **C-myc epitope** | | | | | | | | 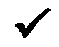 | |
| **RECOMBINASE SITES** | | | | | | | | | |
| ***attL1I attL2*** | | 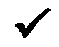 | | 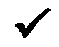 | 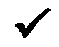 | | | | |
| ***LoxP* sites** | | | | | | 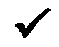 | | | |
| **SV40 NLS** | 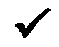 | 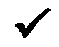 | 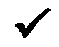 | 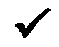 | 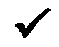 | 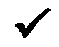 | 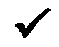 | 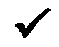 | 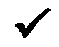 |
| **ADHTT** | 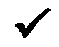 | 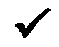 | 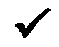 | 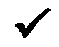 | 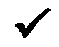 | 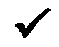 | 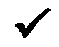 | 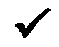 | 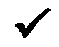 |
| **IRES** | | | | | 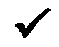 | | | | |
| **SCAG** | | | | | 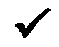 | | | | |

### EXAMPLE 5

### Construction of random peptide aptamer libraries

Conventional two hybrid libraries are fused to a transcriptional activation domain and are not suitable for reverse n-hybrid screens, because they do not allow the cloning of various types of peptide inhibitors wherein some members of the library activate transcription and thereby evade the screen, regardless of whether or not they block the interaction under target. Moreover, an additional selectable marker in the yeast vector, and an appropriate strain for its selection, are preferred.

Existing rational design approaches attempt to model candidate therapeutic peptides based on homologies in the databases to natural inhibitory peptides (e.g. Tiozzo *et al*., 1998). Such existing approaches focus on peptides/polypeptides which have previously been identified from their natural source due to their inhibitory properties. In contrast the screens described herein, empirically determine the most suitable peptides from a wide array of candidate peptides encoded in a genomic expression library. The presence of clones in all reading frames, using a vector such as pBLOCK-3.11, also allows the simultaneous screening of *random* peptides expressed in reading frames which do not occur in nature, together with a variety of *natural* peptide domains cloned in the appropriate reading frame.

Preferably, the Trx-presented random peptide libraries for the reverse two hybrid screen. The preparation of the random inserts constrained within the Trx loop is identical to that described by Colas (1996), except that the preferred vector is optimized for high through-put applications, such as, for example, a pBLOCK vector described herein.

To improve the proportion of appropriately folded domains in the repertoire available for screening, peptide libraries are constructed in the pBLOCK series of vectors described in the preceding example, using randomly selected sequences derived from a phylogenetically diverse compact genomes. To maximize the diversity of the pool, the relative concentration of DNA in the pool from larger genomes is increased in proportion to the total haploid genome size. Such libraries are constructed according to standard methodology (Ausubel, 1989) using the highest efficiency available competent cells, for example XL10-Gold (Stratagene), to ensure complexities greater than 10⁷ independent clones.

To maximize the diversity of the pool of potential inhibitory peptide aptamers, whilst taking advantage of the structural information present in naturally-evolved gene sequences, libraries of inhibitory peptides are preferably constructed from natural genome sources. This approach attempts to accelerate the evolutionary process, by artificially combining domains from different genomes which is unlikely to co-evolve. To achieve this end, genomic expression libraries from evolutionarily diverse organisms are produced, such as, for example, *Fugu rubripes* (Elgar, 1996a, 1996b), *Caenorhabditis elegans* (Plasterk, 1999), and *Saccharomyces cerevisiae.* Alternatively, libraries are constructed using pooled genomic DNA from these organisms, or microorganisms that have been characterized at the genetic level. While the potential diversity achieved by such an approach is theoretically less than that achieved by cloning and expressing DNA purified directly from the environment (Short, 1997), it offers several distinct advantages.

For example, true diversity and bias of the library is more easily approximated, allowing the operator to maximize domain diversity and minimize bias towards the genomes of dominant species.

Additionally, artificial pooling of DNA derived from distinct known organisms allows unique opportunities to survey diverse genomes. For example, the genomes of certain archaebacteria are simultaneously screened with those of obligate parasites, such as Mycoplasma, and/or diverse gram positive and/or gram negative organisms.

Additionally, the diversity of such domain libraries is increased further by mutation. For example, the plasmid library is amplified in bacterial strains that are deficient in mismatch repair (e.g. strains containing the *mutS* mutation), resulting in the generation of mutations.

### EXAMPLE 6

### LexA-responsive counter-selectable CYH2 reporter gene

A panel of constructs have been generated containing up to eight LexA operator DNA binding domains (DBD) embedded in a *GAL10*/*GAL1* basal promoter, configured to drive expression of a *CYH2* reporter gene in yeast. Immediately downstream from *CYH2,* a Zeocin resistance gene (ZEO) is inserted in the reverse orientation. Both yeast and bacterial promoters/transcriptional terminators direct expression of the ZEO gene. The constructs were amplified by PCR, using primers containing 3' tags homologous to the *ADE2* gene, thereby facilitating their targeted integration to gene constructs having the *ADE2* gene. Stable integrants were selected for Zeocin resistance and screened for red pigmentation (adenosine auxotrophy).

### EXAMPLE 7

### LexA-responsive counter-selectable URA3 reporter.

A panel of constructs have been generated containing up to eight *Lex*A operator DNA binding domains (DBD) embedded in a *GAL10*/*GAL1* basal promoter, configured to drive expression of a *URA3* reporter gene in yeast Immediately upstream from the *GAL10*/*1* promoter, the G418 gene is inserted. Expression of G418 is controlled by the *TEF1* promoter, which functions in both yeast and bacteria. The constructs were amplified using PCR primers containing 3' tags homologous to the *HIS5* gene and transformed into yeast. Stable integrants were selected for G418 resistance and screened for histidine auxotrophy.

There is a potential for inadvertent cell death upon counter selection, caused by non-specific activation of the basal promoters fused to reporter genes, via interaction with library products expressed from the pBLOCK3 series of vectors. The impact of such interactions is limited by employing different basal promoter-reporter gene fusions in the same two-hybrid system, since the likelihood of the same expressed library product activating distinct promoters is very low. Hence, the present invention contemplates the use of alternate basal promoters in place of GAL1/10, to drive the *URA3* counter-selectable reporter. *Accordingly, LexAop-URA3* reporter genes, containing either SP013 (Vidal *et al,* 1996a,b), or *CUP1-URA3* fusion genes are constructed. Embedded in these basal promoters are up to eight LexA operator sequences. Constructs are integrated in the SKY473 genome at the *HIS5* locus via a double crossover recombination event (Huang and Schreiber, 1997).

### EXAMPLE 8

### The dual fluorescent reporter gene vector pRT2

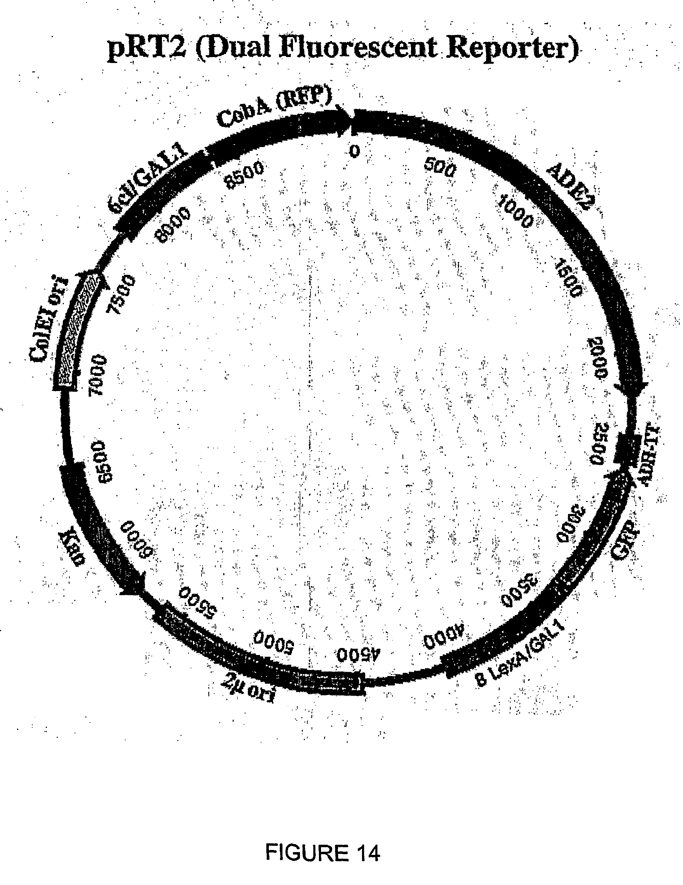

A novel dual fluorescent reporter gene vector was produced. This vector, designated pRT2 (Figure 14 and SEQ ID NO: 10) is derived from pRG64, which contains a *GAL10*/*GAL1* basal promoter with three cl binding sites operably connected to expression of the β-glucuronidase (Gluc) reporter gene.

To produce the intermediate vector pRT1, plasmid pRG64 was digested with *Hind*III, and the ends filled-in and re-ligated to generate a unique *Nhe* I site immediately downstream of the *URA3* counter selectable reporter gene. A fragment of 2,276 bp in length, containing a minimal complementing region of the yeast *ADE2* genomic locus, was amplified using PCR, and inserted at this *Nhe* I site. The *URA3* gene was deleted using *Not*l and *Bsa*l, and a DNA fragment containing a *GAL10*/*GAL1* basal promoter having six cl binding sites operably connected to expression of the *CobA* gene, was inserted into the same sites.

To generate pRT2 (Figure 14), plasmid pRT1 was cut with *BamH*I*,* immediately 5' of the *GAL-Gluc* reporter, and the ends filled-in, and the blunt-ended fragment re-ligated, to generate a *Cla*l site. The entire *GAL*-*Gluc* reporter was excised by digestion with *Cla*l*,* and a 1800 bp fragment, containing a *GAL10*/*GAL1* basal promoter having eight LexA binding sites operably connected to the expression of the CobA gene, was inserted into the *Cla*l site.

The vector contains dual fluorescent reporters allowing real-time assessment of reporter gene activation without the need for substrates. The vector also allows assessment of reporter gene activation via baits fused to LexA and/or cl DNA Binding Domains (DBD's) incorporating both fluorescent reporters, each dependent on distinct DBD's, in the one vector, eliminates confounding effects associated with variability in reporter gene expression due to variation in plasmid copy number observed when the reporters are expressed on different plasmids

The dual fluorescent reporter construct is transformed into yeast strains, that are auxotrophic for Adenine *(ADE2)* thereby selecting for maintenance of the vector. Transformation of the aforementioned strains with prey and baits, the latter fused to LexA and/or cl DBD's, allows for activation of the fluorescent reporters. In the case of a forward two-hybrid yeast screen, successful interaction between baits and preys would result in activation of the relevant fluorescent reporter, which is measured by fluorescent-based detection systems familiar to those skilled in the art. The converse is true for reverse hybrid screening where one is looking for down-regulation of reporter gene expression resulting from the inhibition of interactions between bait and prey proteins.

### EXAMPLE 9

### The dual colorimetric reporter gene vector pRT3

The dual reporter plasmid pRT3 is derived from pDR8, which contains a *GAL10*/*GAL1* basal promoter having three cl binding sites directing expression of the Gluc reporter gene, and a *GAL10*/*GAL1* basal promoter having eight LexA binding sites directing expression of a LacZ reporter gene.

The *URA3* selectable marker contained in pDR8 is exchanged for the *ADE2* gene by digesting the plasmid with *Hind*III, end-filling and re-ligating the blunt-ended fragment, to generate a unique *Nhe*l site. A DNA fragment containing the URA3 gene is excised following digestion with *Nhe*l and *Bsa*I, and a 2,276 bp PCR fragment, containing a minimal complementing region of the yeast *ADE2* genomic locus, is inserted at the same sites, generating pRT3.

### EXAMPLE 10

### Host Strains

The parent host strain SKY473 contains the cl-responsive LYS2 reporter gene. It has the genotype (*MAT-*a*, his*3, *trp*1, *lexAop-Leu*2, *clop-LYS*2).

Particular constructs described in the preceding examples are transformed into a cycloheximide resistant derivative of SKY 473 (*MAT-*a, *his*3*, trp*1, *lexAop-Leu*2, *clop-LYS2*, cyh2^{R}), generating the strain PRT 473 *(MAT-*a, *cyh*2^{R} *his3, trp*1, *lexAop-LEU*2, *lexAop-CYH2ade2::ZEO*^{R}, *lexAop-URA3his5.:G418*^{R}, *clop-LYS*).

Using the same starting strain, different gene constructs produce strain PRT 475 *(MAT-a, cyh2*^{*R*} *his3, trp1, ade2, lexAop-GAL1-LEU2, lexAop-GAL1*/*10-CYH2, lexAop-CUP1-URA3, and clop-GAL1-LYS2).*

Strain PRT 474 differs from strain PRT 475 only by the choice of repressed promoter, which drives URA3 expression. The *CUP1* promoter has the advantage of being copper inducible, thereby allowing titration of repression, by altering the concentration of copper in the medium. Methods for such titration of the degree of repression of the *CUP1* promoter are known to those skilled in the art (Schneider, 1991).

### EXAMPLE 11

### Adjusting selection threshold by titration of galactose.

The reverse two hybrid screening system of the present invention allows the primary adjustment of stringency threshold for any interacting partners expressed from the *GAL1* promoter. Since both the bait and prey interactors are expressed under the control of the *GAL1* promoter, the expression of these proteins in the cell is varied by modifying the level of galactose in the media between no galactose and 2% (w/v) galactose. The concentration of the neutral sugar, raffinose, is maintained at a constant 2%(w/v) concentration.

The galactose induction system facilitates determination of the minimum concentration of binding partner expression required for a counter selectable reporter gene to be expressed at a level that causes cell death, at a predetermined concentration of 5-FOA or cycloheximide. This overcomes the problem of auto-activation of some bait constructs, which causes cell death in the absence of an interacting partner.

The means for determining an appropriate concentration of galactose to induce expression of binding partners is described in Example 1 *supra.*

### EXAMPLE 12

### identifying high affinity peptide inhibitors using surface plasmon resonance

Surface plasmon resonance has rapidly become the benchmark technique for the physical measurement of biomolecular interactions. The instrument makes use of an evanescent wave phenomenon to produce a detectable signal from very subtle refractive index changes on the surface of a microchip, such as, for example, a biosensor chip. Accordingly, the binding, or dissociation, of even a few protein molecules to a partner protein molecule bound to the microchip surface is measured in real-time. This facilitates the derivation of real-time association and dissociation constants. Thus, plasmon resonance is ideally suited to study interactions of proteins identified in reverse n-hybrid screens.

Furthermore, the sensitivity of surface plasmon resonance instrumentation (e.g. BIAcore2000., Pharmacia), facilitates the identification of components in crude cell extracts that compete with the target interaction, assayed on a microchip surface. For example, SCL, purified as a chitin binding protein fusion using the vector pTYB1 (New England Biolabs) is used as starting material. In an adaptation of the screen described in Example 2, purified SCL is covalently coupled to the microchip surface, and purified LMO2, DRG, or E47, expressed as fusion proteins with glutathione S- transferase (GST) is passed over the chip surface, and the binding is monitored using the BlAcore2000. Yeast extracts that express peptide aptamers, or a vector control Trx polypeptide, are then passed across the chip, and the ability of each aptamer to inhibit the association of cognate partners with SCL is determined by a change in refractive index that is measured by the BlAcore2000. The change in refractive index is measured when the peptide inhibitor binds to SCL or to its partner protein or to both proteins. Since the signal produced is proportional to the size of the interacting protein, the difference between the binding of the cognate partners of SCL and the much smaller artificial aptamer, should it interact directly with SCL, can be detected.

The reverse two hybrid assay is repeated, using unrelated pairs of proteins to ensure specificity of the interaction.

Peptide aptamers that inhibit interactions with highest affinity and specificity are immunopurified *via* their Trx domain or other epitope domain, and tested for direct interactions with the binding partners. Their dissociation constants, and sequence are then determined.

The inhibitory effect of peptide aptamers is initially tested by expressing them constrained within the Trx polypeptide loop in cell lines. The 20mer peptides are synthesized as cyclic peptides fused to the *Dropsophila* penetratin motif. Such cyclization is achieved by including flanking cysteine residues in the synthetic peptides. This maintains high affinity, by conformationally constraining the peptides (Giebel *et al*.,1995). Fusion to the penetratin motif facilitates administration of the peptides to the medium of a cell, or the extracellular space. This approach has yielded several biologically active peptide inhibitors.

### EXAMPLE 13

### Preliminary ex-vivo validation of drug screening approach

Transfection assays are used to establish whether expression of peptides that inhibit the SCL/LMOS interaction *in vitro* also inhibit cellular proliferation in a dominant negative manner. Peptides are synthesized in a confonnationally constrained form as fusions with the *Drosophila* 'penetrating' targeting sequence, as described in the preceding example. Alternatively, or in addition, the peptides are expressed as a fusion with the VP22 protein of Herpes Simplex Virus Type-1 (Phelan et at., 1998). These cyclic peptides are subsequently administered directly to leukemia cells in culture to determine their ability to inhibit growth or proliferation of the cells.

In the embodiment described herein, the following methods are employed. Leukemia cells are grown in RPMI including 10% FCS, and transfected with plasmids expressing candidate peptide inhibitors by electroporation according to standard protocols. Transfected cells are purified via the single chain antibody to the hapten phOx expressed by the vector pHOOK-2 (Invitrogen) using magnetic beads. Purified transfectants are outgrown in the presence of G418. Standard methods to determine growth by uptake of ³H thymidine from media and clonogenic colony formation assays are routinely used. Enumeration of live and dead cells is performed using a set of probes from Molecular Probes (#L03224).

### Expression of peptide inhibitors in cell lines derived from leukemia patients: A model for inhibition of leukemia cell growth.

Plasmids from the pBLOCK vector series described herein allow the direct bioassay of candidate blocking peptides in mammalian cells. Clones are rescued from yeast cultures by the transformation of bacteria such as *E.coli* using yeast lysates and selection for antibiotic resistance. Selection of the pBLOCK plasmid amongst other plasmids present in the yeast cell is facilitated by ensuring that the antibiotic resistance marker is unique to that plasmid.

Alternatively, promising high affinity peptide inhibitors are cloned in the context of the thioredoxin loop into the mammalian expression vector pHOOK-2 (Invitrogen) under the control of the CMV promoter. This vector also contains a mammalian selectable marker (NEO) and constitutively expresses a single chain antibody to the hapten phOx. Alternatively, where the recipient plasmid contains appropriate flanking sequences for site-specific recombination, the plasmids (eg. pBLOCK-3.2, pBLOCK-3.6 and pBLOCK-3.8) allow the rapid transfer of inserts from the library into the desired expression plasmid using a recombinase reaction. In one preferred embodiment, this step is eliminated in vectors of the pBLOCK family which already contain genes encoding single chain antibodies for physical selection via their cognate epitopes/haptens. Preferably, the peptide inhibitor-expressing constructs are transfected into the following cell lines by lipofection or electroporation:
(i) Erythroleukemic K562 cells that express LM02, DRG, E47 and SCL;
(ii) the cell line PER-255 (Kees *et al*., 1989) that expresses DRG, E47, and SCL;
(iii) the cell line PER-117 (Kees *et al.,* 1987) that expresses SCL, DRG and E47; and
(iv) the cell line PER-550 (Kees et al, unpublished) that expresses LM02, SCL, E47 and DRG.

As a control, use of the T-cell line MOLT-4, or the B-cell line Raji , which do not express SCL, but express both DRG and E47 (Green *et al.*, 1991a,b), is contemplated.

Additionally, the expression vector alone is transfected into each cell line *supra* as a negative control.

Following transfection and growth for 24 hours, the transfected cell population is purified using magnetic beads coated with the phOx-coated hapten. Equal numbers of selected and control cells are cultured and grown in media containing G418. Cell numbers are measured after 24, 48, and 72 hours. Proliferation is determined by measuring ³H-thymidine uptake, and performing clonogenic colony formation assays, using standard procedures. Viability is assessed by enumerating live and dead cells using probes that are amendable to detection by flow cytometry or FACS.

### Introduction of peptides into leukemia cell lines

The peptides used in this experiment were previously selected *in vitro* for high affinity inhibition of the SCULMO2 interaction (Example 2). Inhibitory peptides, and control "scrambled" peptides of equivalent length, are synthesized as fusions with the *Drosophila* penetratin sequence, and having oxidizable flanking cysteine residues to facilitate their cyclization.

Peptide inhibition of SCL-dependent growth and target gene activation is determined by adding the interacting peptides (and a non-interacting control) directly to cultures of K562, PER-255, PER-550, PER-117, MOLT-4 and Raji cells, and monitoring the effects over 72 hours as described above. Peptides that inhibit interactions *in vivo* block SCL-dependent growth. In contrast, the control peptide, or peptides having an affinity for a non-expressed partner protein, have no effect on SCL-dependent growth.

### EXAMPLE 14

### Design of peptides that inhibit oncoprotein interactions.

Consensus alignments of sequence information derived from promising peptide inhibitors of the SCL/LMO2 interaction assist in the elucidation of potential naturally-occurring binding partners for a nuclear oncoprotein. In particular, the aligned sequences define consensus motifs for high affinity binding partners.

In one approach, cDNA libraries constructed from T-ALL cell lines *supra* are screened using oligonucleotides that encode consensus interaction motif(s), to identify a cDNA encoding a naturally-occurring oncoprotein having the consensus interaction motif(s).

Alternatively, libraries constructed from the pooled genomes of sequenced organisms may be screened and the sequences of the positive hybridizing clones aligned. Whilst the alignment of sequences derived from a screen reveals consensus motifs, other potential related motifs are excluded if they are not identified from any single genome in which they theoretically occur, despite exhaustive screening at a complexity which would be predicted to cover all of the potential domains encoded by the genome/s. Accordingly, the aligned sequences from the screen of pooled genomes are useful for designing optimal peptides that mimic the consensus motifs identified in the biological screen, while lacking alternative residues of structurally related peptides that were included in the exhaustive screen of a single genome.

### EXAMPLE 15

### Peptide synthesis

38-mer peptides are designed in the form of Cys(Penetratin 16-mer)(interacting 20-mer)Cys. The sequence of the penetrating motif is provided by Derossi (1994). The peptides (synthesized by Chiron Mimotopes, Australia) are cyclized by oxidation of the flanking cysteine residues using 10 mM K3Fe(CN)6 at pH 8.4 and purified by reverse-phase HPLC according to Koivunen (1994).

### REFERENCES

1. Akiyama, *et al.* (1989) *Proc. Natl Acad. Sci (USA)* 86:3949-3952.
2. Amann and Brosius (1985) Gene 40:183.
3. Ausubel, F. M., Brent, R., Kingston, RE, Moore, D.D., Seidman, J.G., Smith, J.A., and Struhl, K. (1987-1997) *In*: Current Protocols in Molecular Biology. Wiley Interscience (ISBN 047150338).
4. Chalfie, M. *et al* (1994) *Science* 263:802-805.
5. Colas, P. *et al*. (1996) *Nature* 380:548.
6. Condorelli, G.L. *et al.* (1996) *Cancer Research* 56:5113.
7. Cormack, B. *et al* (1996) Gene (in press).
8. Derossi, D. *et al.* (1994) *J*. *Biol. Chem*. 269:10444.
9. Elgar, G. (1996a). *Human Mol. Genet.* 5:1437-42.
10. Elgar, G., *et al*. (1996b). *Trends in Genetics* 12(4): 145-50.
11. Fahraeus, E., *et al.* (1996) *Current Biology 6*: 84-91.
12. Feilotter, et *al.* (1994). *Nucl. Acids Res.* 22 (8): 1502-3.
13. Fijalowska, I.J., and Schaaper, R.M. (1995) *J. Bacteriol.* 177:5979-5986.
14. Finley, Jr, R.L., *et al.* (1994) *Proc. Natl. Acad. Sci. (USA)* 91:12980.
15. Frick, *et al.* (1995) *J. Biol. Chem.* 270: 24086-24091.
16. Giebel, L.B. *et al.,* (1995) *Biochemistry* 34:15430.
17. Golemis, E., and Brent, R. (1992) *Mol. Biol.* 12: 3006-3014,
18. Green, A. R., DeLuca, E., and Begley, C. G. (1991b). *EMBO J* 10: 4153-4158.
19. Green, A.R. *et al.,* (1991a) *Oncogene* 6:475.
20. Gyuris, J.E. *et al.* (1993) *Cell* 75:791-803.
21. Haas, J. *et al* (1996) *Curr. Biol.* 6:315-324.
22. Hsu, H.L. *et al.* (1991) *Mol. Cell Biol.* 11:3037.
23. Huang, J. and Schreiber, SL (1997). *Proc. Natl. Acad. Sci.,* 94:13396-13401.
24. Inouye, S., and Tsuji, F.I. (1994) *FEBS Letts* 341:277-280.
25. Kees, U. R., *et al.* (1987). *Leukemia Res*.11(5): 489-498.
26. Kees, U. R., *et al.* (1989). Blood 74(1): 369-373.
27. Kioveunen, E. *et al.* (1994) *J*. *Cell Biol*. 124:373.
28. Kozak, M. (1987) *Nucleic Acids Res.* 15: 8125-8148.
29. Larson, R.C. *et al.,* (1996) *EMBO J.15* (5):1021.
30. Lu, T-W., and Cillo (1995) *J*. *Biol. Chem.* 270:23582-23588.
31. Mahajan, M.A. *et al.* (1996) *Oncogene* 12:2343.
32. Maki and Sekiguchi (1992) *Nature* 355: 273-275.
33. Marcello, A. et *al.* (1994) *Proc. Natl. Acad. Sci. USA* 91:8994.
34. Matz, MV *et al.,* (1999) *Nature Biotechnology* 17: 969-973.
35. Miller, J.H. (1992) A *Short Course in Bacterial Genetics*. Cold Springs Harbor Laboratory Press, Cold Spring Harbor, N.Y.
36. Miller, J.H., and Michaels, M. (1996) Gene 179:129-132.
37. Moriya and Gollman (1992) *Mol. Gen. Genetics* 239: 72-76.
38. Osada, *et al.* (1995) *Proc. Natl. Acad. Sci. USA* 92:9585.
39. Ow, D.W.; *et al.* (1986) Science 234:856-859.
40. Phelan, *et al* (1998). *Nature Biotechnology* 16(5): 440-443.
41. Plasterk, R. H. A. (1999). *Nature genetics* 21: 63-64.
42. Prasher, D.C. *et al* (1992) *Gene* 111:229-233
43. Rouquet, N *et al.,* (1996) *Current Biology* 6:1192-1195
44. Sambrook, J.; Fritsch, E.F.; and Maniatis, T. (1989) Molecular cloning. A Laboratory Manual. Second Edition. Cold Spring Harbor Laboratory, Cold Spring Harbor, N.Y.
45. Schaaper, R.M., and Comacchio, R. (1992) *J*. *Bacteriol.* 174:1974-1982.
46. Schneider, J. a. G., L. (1991). *In:* Vectors for expression of cloned genes in yeast: Regulation Overproduction and underproduction, Vol.194, (C. Guthrie, Ed) San Diego: Academic Press.
47. Serebriiskii *et* al (1999)*J*. *Biol. Chem*. 274(24):17080-17087.
48. Shimatake and Rosenberg (1981) *Nature* 292:128.
49. Short, J. (1997). *Nature Biotechnology* 15:1322-1323.
50. Sikorski, R.S. *et al.* (1989) *Genetics.* 12219-27.
51. Slupska, M.M., *et al* (1996) *Proc. Natl. Acad. Sci. (USA)* 93: 4380-4385.
52. Studier and Moffat (1986) *J. Mol. Biol. 189*:113.
53. Tajiri, *et al.* (1995) *Mutation Res.* 336: 257-267.
54. Thompson, J.F.; Hayes, L.S.; and Lloyd, D.B. (1991) *Gene 103*:171-177.
55. Tiozzo, E., *et al.* (1998). *Biochem. Biophys. Res. Comm*. 249: 202-206.
56. Vidal, M. *et al.* (1996b) *Proc. Natl. Acad. Sci. USA* 93:10315.
57. Vidal, M., P. *et al.* (1996a) *Proc. Natl*. *Acad. Sci. USA* 93:10321.
58. Wadman, I., J. *et al.* (1994) *EMBO J* 13 (20):4831.
59. Warbrick, E., *et al.,* (1996) *Current Biology* 5:275.
60. Wildt, S and Deuschle, U (1999) *Nature Biotechnology* 17: 1175-1178
61. Young, K., et *al.* (1998). *Nature Biotechnology* 16: 946-950.

## Claims

1. A method of identifying a peptide that partially or completely inhibits a target interaction between two or more binding partners in a host cell but does not inhibit a non-target interaction between some but not all of said binding partners, said method comprising:
(i) expressing in a cellular host: (a) the binding partners of said target interaction such that they operably control the expression of one or more reporter genes in said cellular host, wherein said expression is partially or completely inhibited by disruption of said target interaction; (b) the binding partners of said non-target interaction such that they operably control the expression of one or more reporter genes in said cellular host, wherein said expression is partially or completely inhibited by disruption of said non-target interaction and wherein said reporter gene is distinct from the reporter gene(s) expressed under control of the target interaction; and (c) a candidate peptide; wherein one or more reporter genes operably under the control of the target interaction or the non-target interaction is a counter selectable reporter gene that confers conditional lethality on the cellular host, and wherein, to determine the minimum concentration of binding partner expression required for the counter-selectable reporter gene to be expressed at a level that causes cell death, the expression of one or more of said binding partners is titrated in response to galactose or copper by placing said expression under the control of a promoter comprising regulatory elements of the *GAL1* or *CUP1* promoters;
(ii) growing the cellular host under conditions sufficient to distinguish the expression of each reporter gene(s) at (a) from expression of the reporter gene(s) at (b); and
(iii) detecting those host cells wherein expression of the reporter gene(s) operably under control of the target interaction is(are) partially or completely inhibited and expression of the reporter gene(s) operably under control of the non-target interaction is(are) not inhibited, said detected cells expressing a peptide that partially or completely inhibits the target interaction.

2. The method of claim 1, wherein: (i) the one or more counter selectable reporter genes are CYH2 and URA3; (ii) expression of one or more interacting binding partners is under the control of the GAL1 promoter to facilitate determination of the minimum concentration of binding partner expression required for a counter selectable reporter gene to be expressed at a level that causes cell death at a predetermined concentration of 5-FOA or cycloheximide.

3. The method of claim 1 or 2 wherein expression of one or more of said interacting binding partners is under the control of a promoter comprising regulatory elements of the *GAL1* promoter.

4. The method of claim 2 or 3 wherein expression of the interacting binding partners is varied by modifying the level of galactose in the media between no galactose and 2% (w/v) galactose and the concentration of raffinose is maintained at 2% (w/v) concentration.

5. The method of any one of claims 1 to 4, wherein:
(i)the target interaction involves two or more proteinaceous binding partners wherein one of said partners binds to nucleic acid comprising a *cis*-acting sequence and the other of said partners activates transcription of the reporter gene(s) of the target interaction when the target interaction occurs in the host cell; and
(ii) the non-target interaction involves two or more proteinaceous binding partners wherein one of said partners binds to nucleic acid comprising a *cis-*acting sequence and the other of said partners activates transcription of the reporter gene(s) of the non-target interaction when the non-target interaction occurs in the host cell.

6. The method of claim 5 wherein the target interaction is between two proteinaceous binding partners that each comprise an RNA-binding domain and a hybrid RNA molecule capable of binding to said RNA-binding domains, wherein one of said proteinaceous binding partners binds to nucleic acid comprising a *cis*-acting sequence and the other of said proteinaceous binding partners activates transcription of the counter selectable reporter gene(s) of the target interaction when the target interaction occurs in the host cell.

7. The method of any one of claims 1 to 6, further comprising the first step of introducing into the cellular host one or more nucleic adds that comprise a sequence selected from the group consisting of:
(i)a sequence encoding a binding partner of the target interaction in an expressible format;
(ii) a sequence encoding a binding partner of the non-target interaction in an expressible format;
(iii) a sequence encoding an activation domain of the target interaction in an expressible format;
(iv) a sequence encoding an activation domain of the non-target interaction in an expressible format;
(v) a sequence encoding a DNA binding domain of the target interaction in an expressible format;
(vi) a sequence encoding a DNA binding domain of the non-target interaction in an expressible format;
(vii) a sequence encoding the candidate peptide in an expressible format;
(viii) a sequence comprising a *cis*-acting sequence and a reporter gene in an expressible format; and
(ix) a sequence comprising a *cis*-acting sequence and a counter selectable reporter gene in an expressible format.

8. The method of claim 7 further comprising mating those cells having one or more of said nucleic acids so as to combine sufficient nucleic acids into a single cell to select those host cells that grow or survive when the counter selectable reporter genes operably under control of the target interaction are expressed.

9. The method of claim 7, wherein nucleic acid encoding a binding partner or a candidate peptide also encodes a nuclear localization signal (NLS) to facilitate nuclear localization of said binding partner or said candidate peptide.

10. The method of any one of claims 7 to 9, wherein one or more of said nucleic acids is placed operably under the control of a promoter selected from the group consisting of: *MYC, GAL1, CUP1, PGK1, ADH1, ADH2, PHO4, PHO5, HIS4, HIS5, TEF1, PRB1, GUT1, SPO13, CMV, SV40, LAC, EM7, SV40,* and T7.

11. The method of any one of claims 7 to 10, wherein one or more of said nucleic acids is contained within a vector selected from the group consisting of: pBLOCK-3.0 (SEQ ID NO: 1); pBLOCK-3.2 (SEQ ID NO: 2); pBLOCK-3.4 (SEQ ID NO: 3); pBLOCK-3.6 (SEQ ID NO: 4); pBLOCK-3.8 (SEQ ID NO: 5); pBLOCK-3.9 (SEQ ID NO: 6); pBLOCK-3.10 (SEQ ID NO: 7); pBLOCK-3.11 (SEQ ID NO: 8); pBLOCK-4.0 (SEQ ID NO: 9); and pRT2 (SEQ ID NO: 10).

12. The method of any one of claims 1 to 11 wherein the cellular host is a yeast cell.

13. The method according to claim 12 wherein the yeast cell has the genotype *MATα, ura3, trp1, his3, cyh2*^{*R*}*, lexAop-URA3, lexi4op-CYH2, ade2* or *MAT-a, cyh2*^{*R*}*, his3, trp1, lexAop-LEU2, lexAop-CYH2, ade2::ZEO*^{*R*}*, lexAop-URA3, his5::G418*^{*R*}*, clop-LYS.*

14. The method of claim 12 or 13 comprising:
(i)transforming a yeast cell with a vector selected from the group consisting of: pBLOCK-3.0 (SEQ ID NO: 1); pBLOCK-3.2 (SEQ ID NO: 2); pBLOCK-3.4 (SEQ ID NO: 3); pBLOCK-3.6 (SEQ ID NO: 4); pBLOCK-3.8 (SEQ ID NO: 5); pBLOCK-3.9 (SEQ ID NO: 6); pBLOCK-3.10 (SEQ ID NO: 7); pBLOCK-3.11 (SEQ ID NO: 8); and pBLOCK-4.0 (SEQ ID NO: 9), wherein said vector further comprises nucleic acid encoding a candidate peptide being tested for inhibitory activity;
(ii) introducing to said transformed yeast cell nucleic acid encoding: (a) the binding partners of said target interaction such that they operably control the expression of one or more counter selectable reporter genes or fluorescent protein-encoding reporter genes in said cellular host, wherein said expression is partially or completely inhibited by disruption of said target interaction; and (b) the binding partners of said non-target interaction such that they operably control the expression of one or more counter selectable reporter genes or fluorescent protein-encoding reporter genes in said cellular host, wherein said expression is partially or completely inhibited by disruption of said non-target interaction and wherein said reporter gene is distinct from the reporter gene(s) expressed under control of the target interaction;
(iii) selecting the recombinants;
(iv) growing the recombinants under conditions sufficient to distinguish the expression of each reporter gene(s) at (a) from expression of the reporter gene(s) at (b); and
(v) detecting those host cells wherein expression of the reporter gene(s) operably under control of the target interaction is(are) partially or completely inhibited and expression of the reporter gene(s) operably under control of the non-target interaction is(are) not inhibited, said detected cells expressing a peptide that partially or completely inhibits the target interaction.

15. The method of claim 14 wherein nucleic acid is introduced at (ii) by means of transformation and the recombinants selected at (iii) are the transformants produced by said transformation, or wherein nucleic acid is introduced at (ii) by means of cell mating and the recombinants selected at (iii) are diploids arising from said cell mating.

16. The method of claim 14 or 15, wherein the fluorescent protein-encoding reporter genes are introduced to the cell by transforming the cell with the vector pRT2 (SEQ ID NO: 11) or by mating the cell with a yeast cell containing said vector.

17. The method of claim 14 wherein nucleic acid encoding one or more of the binding partners of the target interaction and/or the non-target interaction are introduced to the cell by transforming the cell with a derivative of a vector selected from the group consisting of: pBLOCK-3.0 (SEQ ID NO: 1); pBLOCK-3.2 (SEQ ID NO: 2); pBLOCK-3.4 (SEQ ID NO: 3); pBLOCK-3.6 (SEQ ID NO: 4); pBLOCK-3.8 (SEQ ID NO: 5); pBLOCK-3.9 (SEQ ID NO: 6); pBLOCK-3.10 (SEQ ID NO: 7); pBLOCK-3.11 (SEQ ID NO: 8); and pBLOCK-4.0 (SEQ ID NO: 9), wherein said derivative includes a nucleotide sequence encoding said binding partner.

18. The method of claim 1, wherein the binding partners of said target interaction operably and simultaneously control the expression of two distinct reporter genes in said cellular host.

19. The method of any one of claims 1 to 18, wherein one or more of the binding partners of the non-target interaction is the same as a binding partner of the target interaction.

20. The method of claim 5, wherein:
(i)the proteinaceous binding partners of the target interaction consist of two fusion proteins, wherein (a) one fusion protein comprises the DNA binding domain of a transcription factor and an amino acid sequence that dimerizes with the other fusion protein of the target interaction; and (b) one fusion protein comprises the transcriptional activator domain of a transcription factor and an amino acid sequence that dimerizes with the other fusion protein of the target interaction, and wherein dimerization between said fusion proteins produces a protein complex that binds to the *cis*-acting sequence and activates expression of the reporter gene(s) when the target interaction occurs in the host cell; and
(ii) the proteinaceous binding partners of the non-target interaction consist of two fusion proteins, wherein (a) one fusion protein comprises the DNA binding domain of a transcription factor and an amino acid sequence that dimerizes with the other fusion protein of the non-target interaction; and (b) one fusion protein comprises the transcriptional adivator domain of a transcription factor and an amino acid sequence that dimerizes with the other fusion protein of the non-target interaction, and wherein dimerization between said fusion proteins produces a protein complex that binds to the *cis*-acting sequence and adivates expression of the reporter gene(s) when the non-target interaction occurs in the host cell.

21. The method of claim 20 wherein:
(i) the fusion proteins of the target interaction and the non-target interaction that comprise a transcription activator domain are the same;
(ii) the fusion proteins of the target and non-target interactions that comprise a DNA binding domain are different; and
(iii) the *cis*-acting sequences of the target and non-target interactions are different.

22. The method of claim 20 wherein:
(i)the fusion proteins of the target and non-target interactions that comprise a DNA binding domain are the same; and
(ii) the *cis*-acting sequences of the target and non-target interactions are the same; and
(iii) the fusion proteins of the target interaction and the non-target interaction that comprise a transcription activator domain are different.

23. The method of claim 20 wherein:
(i)the fusion proteins of the target and non-target interactions that comprise a transcription activator domain are the same; and
(ii) the *cis*-acting sequences of the target and non-target interactions are different; and
(iii) the proteins of the target interaction and the non-target interaction that are fused to a DNA binding domain are the same and each of said proteins is fused to a different DNA binding domain.

24. The method of any one of claims 20 to 23, wherein the *cis*-acting sequence of the target interaction or the non-target interaction is selected from the group consisting of: LexA operator, GAL4 binding site, and *cl* operator and wherein the DNA binding domain of the target interaction and/or the non-target interaction is selected from the group consisting of: LexA operator binding domain, GAL4 DNA binding domain; and *cl* operator binding domain.

25. The method according to any one of claims 19 to 24, wherein the transcriptional activator domain is the GAL4 activator domain.

26. The method of claim 24 or 25, wherein the fusion proteins of the target interaction and the non-target interaction that comprise a transcription activator domain also comprise a dimerization region of the SCL protein and wherein the fusion proteins of the target and non-target interactions that comprise DNA binding domains each comprise a dimerization region of a distinct protein selected from the group consisting of: LM01, LMO2, DRG, mSin3A, and E47.

27. The method of claim 24 wherein the fusion protein of the target interaction comprises the dimerization region of LMO2 and wherein the fusion protein of the non-target interaction comprises the dimerization region of E47, or mSin3A, or wherein the fusion protein of the target interaction comprises the dimerization region of E47 and wherein the fusion protein of the non-target interaction comprises the dimerization region of LMO2 or mSin3A.

28. The method of claim 24 or 25, wherein the fusion proteins of the target interaction and the non-target interaction that comprises a DNA binding domain also comprise a dimerization region of the SCL protein and wherein the fusion proteins of the target and non-target interactions that comprise transcription activator domains each comprise a dimerization region of the distinct protein selected from the group consisting of: LMO1, LMO2, DRG, mSin3A, and E47.

29. The method of claim 28 wherein the fusion protein of the target interaction comprises the dimerization region of LMO2 and wherein the fusion protein of the non-target interaction comprises the dimerization region of E47.

30. The method according to any one of claims claim 1 to 29, wherein a reporter gene operably under the control of the target interaction or the non-target interaction either (i) encodes a fluorescent protein and wherein said detecting comprises identifying those cells that do not fluoresce or have reduced fluorescence when said reporter gene is not expressed compared to when said reporter gene is expressed, or (ii) is a counter selectable reporter gene that encodes a polypeptide capable of converting a non-toxic substrate to a toxic product and wherein said detecting comprises identifying those cells that grow or survive when said counter selectable reporter gene is not expressed.

31. The method of claim 30 wherein the reporter gene is the *GFP* gene or *cobA* gene or a variant or fragment of said *GFP* gene or said *cobA* gene that encodes a fluorescent protein, or is a counter selectable reporter gene selected from the group consisting of: URA3, CYH2, and LYS2.

32. The method of claim 30 wherein a counter selectable reporter gene operably under control of the non-target interaction is LYS2, or LEU2.

33. The method of claim 1 wherein two counter selectable reporter genes consisting of URA3 and CYH, URA3 and LYS2, or LYS2 and CYH2 are operably under the control of the target interaction.

34. The method of any one of claims 1 to 33, wherein multiple reporter genes are operably under the control of the target interaction and wherein said reporter genes comprise at least one counter selectable reporter gene and at least one gene encoding a fluorescent protein such that said detecting comprises identifying those cells grow or survive and do not fluoresce or have reduced fluorescence when said reporter gene is not expressed compared to when said reporter gene is expressed.

35. The method of any one of claims 1 to 34, wherein multiple reporter genes are operably under the control of the non-target interaction and wherein said reporter genes comprise at least one counter selectable reporter gene and at least one gene encoding a fluorescent protein.

36. The method of any one of claims 1 to 35, wherein the candidate peptide is expressed in a conformationally constrained form within a Trx polypeptide loop or comprising oxidized flanking cysteine residues.

37. The method of any one of claims 1 to 36, further comprising selecting and growing the detected cells.

38. Use of a yeast shuttle vector capable of expressing a candidate peptide inhibitor in the method of any one of claims 1 to 37 wherein said vector comprises site specific recombinase sites flanking a sequence encoding the candidate peptide.

39. Use of a vector comprising a nucleotide sequence as set forth in a sequence selected from the group consisting of SEQ ID NOs: 1 to 10 in the method of any one of claims 1 to 38.

40. Use of a vector for expressing red and green fluorescent proteins in yeast in the method of any one of claims 1 to 37 said vector comprising:
(i)a green fluorescent protein expression cassette comprising the *gfp* gene operably under control of a chimeric yeast operable *LexA*/*GAL1* promoter having multiple *Lex*A operator sites; and
(ii) a red fluorescent protein expression cassette comprising the *cobA* gene operably under control of a chimeric *cl*/*GAL1* promoter having multiple *cl* operator sites.

41. The use of claim 40, wherein the vector has the structural characteristics for expression of the fluorescent proteins as contained in vector pRT2 (SEQ ID NO: 10).

42. The method according to any one of claims 1 to 41 additionally comprising:
(iv) isolating a nucleotide sequence encoding a peptide inhibitor that partially or oompletely inhibits the target interaction;
(v) transfecting said eukaryotic cell with the isolated nucleic acid; and
(vi) comparing the phenotype or expression pattern of the transfected eukaryotic cell to the phenotype or expression pattern of an otherwise isogenic non-transfected cell, wherein a different phenotype or expression pattern indicates that the peptide has an effect on the cell.

43. The method of claim 42 wherein the eukaryotic cell is a mammalian cell.

44. The method of claim 42 or claim 43 wherein comparing the expression pattern of the transfected eukaryotic cell to the expression pattern of an otherwise isogenic non-transfected cell is performed by producing an array of protein or nucleic acid expressed by the transfected and non-transfected cells and comparing said protein or nucleic acid.

45. The method of claim 44 or claim 45 wherein the phenotype of the transfected cell is compared to the phenotype of the non-transfected cell.

46. A process for identifying a peptide for the prophylactic or therapeutic treatment of a mammal comprising performing the method according to any one of claims 44 to 45 on a diseased cell and selecting a peptide that reverts the phenotype or expression profile of the transfected cell.

47. A process for identifying a binding partner or drug target comprising performing the method of claim 44 and identifying the nucleic acid or protein that is modified in the transfected cell.

## Patentansprüche

1. Verfahren zur Identifikation eines Peptids, das eine Zielinteraktion zwischen zwei oder mehr Bindungspartnern in einer Wirtszelle teilweise oder vollständig inhibiert, aber eine Nicht-Ziel-Interaktion zwischen einigen, aber nicht allen dieser Bindungspartner nicht inhibiert, umfassend:
(I) Expression in einem zellulären Wirt:
(A) der Bindungspartner der Zielinteraktion, so dass sie auf funktionsfähige Weise die Expression eines oder mehrerer Reportergene in dem zellulären Wirt kontrollieren, wobei diese Expression durch eine Störung der Zielinteraktion teilweise oder vollständig inhibiert wird;
(B) der Bindungspartner der Nicht-Ziel-Interaktion, so dass sie auf funktionsfähige Weise die Expression eines oder mehrerer Reportergene In dem zellulären Wirt kontrollieren, wobei diese Expression durch eine Störung der Zielinteraktion teilweise oder vollständig inhibiert wird und das Reportergen von dem/den unter Kontrolle der Zielinteraktion exprimierten Reportergen/ Reportergenen verschieden ist; und
(C) eines Kandidatenpeptids;
wobei eines oder mehrere der auf funktionsfähige Weise unter der Kontrolle der Zielinteraktion oder der Nicht-Ziel-Interaktion stehenden Reportergene gegenselektierbare Reportergene sind, die dem Wirt konditionale Letalität vermitteln, und wobei zur Bestimmung der minimalen Konzentration der Bindungspartnerexpression, die erforderlich ist, damit das gegenselektierbare Reportergen auf einem den Zelltod bewirkenden Niveau exprimiert wird, die Expression von einem oder mehreren der Bindungspartner in Abhängigkeit von Galaktose oder Kupfer titriert wird, indem die Expression unter die Kontrolle eines Promotors gestellt wird, der regulatorische Elemente der GAL1- oder CUP1-Promotoren umfasst;
(II) Wachstum des zellulären Wirts unter Bedingungen, die ausreichend sind, um die Expression jedes Reportergens bzw. aller Reportergene in (A) von der Expression des Reportergens bzw. der Reportergene in (B) zu unterscheiden; und
(III) Detektion derjenigen Wirtszellen, in denen die Expression des bzw. der funktionsfähig unter der Kontrolle der Zielinteraktion stehende(n) Reportergens/Reportergene teilweise oder vollständig inhibiert wird und die Expression des bzw. der funktionsfähig unter der Kontrolle der Nicht-Ziel-Interaktion stehenden Reportergens/Reportergene nicht inhibiert wird, wobei die detektierten Zellen ein Peptid exprimieren, das die Zielinteraktion teilweise oder vollständig inhibiert.

2. Verfahren nach Anspruch 1, wobei:
(I) das eine oder die mehreren gegenselektierbaren Reportergene CYH2 und URA3 sind;
(II) die Expression von einem oder mehreren Bindungspartnern unter der Kontrolle des GAL1-Promotors steht, um die Bestimmung der minimalen Konzentration der Bindungspartnerexpression, die erforderlich ist, damit das gegenselektierbare Reportergen auf einem bei einer vorher festgelegten Konzentration von 5-FOA oder Cycloheximid den Zelltod bewirkenden Niveau exprimiert wird, zu erleichtern.

3. Verfahren nach Anspruch 1 oder 2, wobei die Expression eines oder mehrerer interagierender Bindungspartner unter der Kontrolle eines Promotors steht, der regulatorische Elemente des GAL1-Promotors umfasst.

4. Verfahren nach Anspruch 2 oder 3, wobei die Expression der interagierenden Bindungspartner verändert wird, indem man die Galaktosekonzentration im Medium zwischen keiner Galaktose und 2% (Gewicht/Volumen) Galaktose variiert und die Raffinosekonzentration bei 2% (Gewicht/Volumen) hält.

5. Verfahren nach einem der Ansprüche 1 bis 4, wobei:
(I) an der Zielinteraktion zwei oder mehr proteinöse Bindungspartner beteiligt sind, wobei einer dieser Partner an eine Nukleinsäure bindet, die eine cis-wirksame Sequenz umfasst, und der andere dieser Partner die Transkription des Reportergens bzw. der Reportergene der Zielinteraktion aktiviert, wenn die Zielinteraktion in der Wirtszelle stattfindet;
(II) an der Nicht-Ziel-Interaktion zwei oder mehr proteinöse Bindungspartner beteiligt sind, wobei einer dieser Partner an eine Nukleinsäure bindet, die eine cis-wirksame Sequenz umfasst, und der andere dieser Partner die Transkription des Reportergens bzw. der Reportergene der Nicht-Ziel-Interaktion aktiviert, wenn die Nicht-Ziel-Interaktion in der Wirtszelle stattfindet.

6. Verfahren nach Anspruch 5, wobei die Zietinteraktion zwischen zwei proteinösen Bindungspartnern erfolgt, deren jeder eine RNA-Bindungsdomäne und ein hybrides RNA-Molekül, das an diese RNA-Bindungsdomäne zu binden vermag, umfasst, wobei einer der proteinösen Bindungspartner an Nukleinsäuren bindet, die eine cis-wirksame Sequenz umfassen, und der andere der proteinösen Bindungspartner die Transkription des gegenselektierbaren Reportergens bzw. der gegenselektierbaren Reportergene der Zielinteraktion aktiviert, wenn die Zielinteraktion in der Wirtszelle stattfindet.

7. Verfahren nach einem der Ansprüche 1 bis 6, **dadurch gekennzeichnet, dass** es weiterhin als ersten Schritt die Einführung einer oder mehrerer Nukleinsäuren in den zellulären Wirt umfasst, die eine Sequenz enthalten, die ausgewählt ist unter:
(I) einer Sequenz, die einen Bindungspartner der Zielinteraktion in exprimierbarer Form codiert;
(II) einer Sequenz, die einen Bindungspartner der Nicht-Ziel-Interaktion in exprimierbarer Form codiert;
(III) einer Sequenz, die eine Aktivierungsdomäne der Zielinteraktion in exprimierbarer Form codiert;
(IV) einer Sequenz, die eine Aktivierungsdomäne der Nicht-Ziel-Interaktion in exprimierbarer Form codiert;
(V) einer Sequenz, die eine DNA-Bindungsdomäne der Zielinteraktion in exprimierbarer Form codiert;
(VI) einer Sequenz, die eine DNA-Bindungsdomäne der Nicht-Ziet-Interaktion in exprimierbarer Form codiert;
(VII) einer Sequenz, die das Kandidatenpeptid in exprimierbarer Form codiert;
(VIII) einer Sequenz, die eine cis-wirksame Sequenz und ein Reportergen in exprimierbarer Form codiert; und
(IX) einer Sequenz, die eine cis-wirksame Sequenz und ein gegenselektierbares Reportergen in exprimierbarer Form codiert.

8. Verfahren nach Anspruch 7, **dadurch gekennzeichnet, dass** es weiterhin eine Paarung derjenigen Zellen, die eine oder mehrere der Nukleinsäuren enthalten, umfasst, so dass auseichend Nukleinsäuren in einer einzelnen Zelle kombiniert werden, um diejenigen Wirtszellen zu selektieren, die wachsen oder überleben, wenn die auf funktionsfähige Weise unter der Kontrolle der Zielinteraktion stehenden gegenselektierbaren Reportergene exprimiert werden.

9. Verfahren nach Anspruch 7, wobei eine Nukleinsäure, die einen Bindungspartner oder ein Kandidatenpeptid codiert, auch ein Kernlokalisationssignal (KLS) codiert, um die Kernlokalisation des Bindungspartners oder des Kandidatenpeptids zu erleichtem.

10. Verfahren nach einem der Ansprüche 7 bis 9, wobei eine oder mehrere der Nukleinsäuren auf funktionsfähige Weise unter der Kontrolle eines Promotors stehen, der ausgewählt ist unter MYC, GAL1, CUP1, PGK1, ADH1, ADH2, PHO4, PHO5, HIS4, HIS5, TEF1, PRB1, GUT1, SPO13 CMV, SV40, LAC, EM7, SV40 und T7.

11. Verfahren nach einem der Ansprüche 7 bis 10, wobei eine oder mehrere der Nukleinsäuren in einem Vektor enthalten sind, der ausgewählt ist unter: pBLOCK-3.0 (SEQ ID NO:1); pBLOCK-3.2 (SEQ ID NO:2); pBLOCK-3.4 (SEQ ID NO:3); pBLOCK-3.6 (SEQ ID NO:4); pBLOCK-3.8 (SEQ ID NO:5); pBLOCK-3.9 (SEQ ID NO:6); pBLOCK-3.10 (SEQ ID NO:7); pBLOCK-3.11 (SEQ ID NO:8); pBLOCK-4.0 (SEQ ID NO:9); und pRT2 (SEQ ID N0:10).

12. Verfahren nach einem der Ansprüche 1 bis 11, wobei der zelluläre Wirt eine Hefezelle ist.

13. Verfahren nach Anspruch 12, wobei die Hefezelle den Genotyp MATα, ura3, trp1, his3, cyh2^{R}, lexAop-URA3, lexAop-CYH2, ade2 oder den Genotyp MAT-a, cyh2^{R}, his3, trp1, lexAop-LEU2, lexAop-CYH2, ade2::ZEO^{R}, lexAop-URA3, his5::G418^{R}, clop-LYS besitzt.

14. Verfahren nach Anspruch 12 oder 13, umfassend:
(I) Transformation einer Hefezelle mit einem Vektor, ausgewählt unter:
pBLOCK-3.0 (SEQ ID NO:1); pBLOCK-3.2 (SEQ ID NO:2); pBLOCK-3.4 (SEQ ID NO:3); pBLOCK-3.6 (SEQ ID NO:4); pBLOCK-3.8 (SEQ ID NO:5); pBLOCK-3.9 (SEQ ID NO:6); pBLOCK-3.10 (SEQ ID NO:7); pBLOCK-3.11 (SEQ ID NO:8); und pBLOCK-4.0 (SEQ ID NO:9), wobei dieser Vektor weiterhin eine Nukleinsäure umfasst, die ein Kandidatenpeptid codiert, das auf inhibitorische Wirkung getestet wird;
(II) Einführung einer Nukleinsäure, codierend:
(A) die Bindungspartner der Zielinteraktion, so dass sie auf funktionsfähige Weise die Expression eines oder mehrerer gegenselektierbarer Reportergene oder fluoreszenzproteincodierender Reportergene in dem zellulären Wirt kontrollieren, wobei die Expression teilweise oder vollständig durch Störung der Zielinteraktion behindert wird; und
(B) die Bindungspartner der Nicht-Ziel-Interaktion, so dass sie auf funktionsfähige Weise die Expression eines oder mehrerer gegenselektierbarer Reportergene oder fluoreszenzproteincodierender Reportergene in dem zellulären Wirt kontrollieren, wobei die Expression teilweise oder vollständig durch Störung der Nicht-Ziel-Interaktion behindert wird und dieses Reportergen sich von dem/den unter Kontrolle der Zielinteraktion exprimierten Reportergen/Reportergenen unterscheidet;
in diese transformierte Hefezelle;
(III) Selektion der Rekombinanten;
(IV) Wachstum der Rekombinanten unter Bedingungen, die ausreichend sind, um die Expression jedes Reportergens bzw. aller Reportergene in (A) von der Expression des Reportergens bzw. der Reportergene in (B) zu unterscheiden; und
(V) Detektion derjenigen Wirtszellen, in denen die Expression der auf funktionsfähige Weise unter der Kontrolle der Zielinteraktion stehenden Reportergene teilweise oder vollständig inhibiert wird und die Expression der auf funktionsfähige Weise unter der Kontrolle der Nicht-Ziel-Interaktion stehenden Reportergene nicht inhibiert wird, wobei die Zellen ein Peptid exprimieren, das die Zielinteraktion teilweise oder vollständig inhibiert.

15. Verfahren nach Anspruch 14, wobei die Nukleinsäure in (II) durch Transformation eingeführt wird und die in (III) selektierten Rekombinanten die durch diese Transformation hervorgebrachten Transformanten sind, oder wobei die Nukleinsäure in (II) durch zelluläre Paarung eingeführt wird und die in (III) selektierten Rekombinanten die aus dieser Zellpaarung entstehenden Diploiden sind.

16. Verfahren nach Anspruch 14 oder 15, wobei die fluoreszenzproteincodierenden Reportergene durch Transformation mit dem Vektor pRT2 (SEQ ID NO:11) oder durch Paarung der Zelle mit einer Hefezelle, die diesen Vektor enthält, eingeführt werden.

17. Das Verfahren nach Anspruch 14, wobei die einen oder mehrere der Bindungspartner der Zielinteraktion und/oder der Nicht-Ziel-Interaktion codierende Nukleinsäure in die Zelle eingeführt wird, indem man die Zelle mit einem Derivat eines Vektors transformiert, der ausgewählt ist unter pBLOCK-3.0 (SEQ ID NO: 1); pBLOCK-3.2 (SEQ ID NO:2); pBLOCK-3.4 (SEQ ID NO:3); pBLOCK-3.6 (SEQ ID NO:4); pBLOCK-3.8 (SEQ ID NO:5); pBLOCK-3.9 (SEQ ID NO:6); pBLOCK-3.10 (SEQ ID NO:7); pBLOCK-3.11 (SEQ ID NO:8), und pBLOCK-4.0 (SEQ ID NO:9), wobei das Derivat eine Nukleotidsequenz enthält, die diesen Bindungspartner codiert.

18. Verfahren nach Anspruch 1, wobei die Bindungspartner der Zielinteraktion auf funktionsfähige Weise und gleichzeitig die Expression zweier verschiedener Reportergene in dem zellulären Wirt kontrollieren.

19. Verfahren nach einem der Ansprüche 1 bis 18, wobei einer oder mehrere der Bindungspartner der Nicht-Ziel-Interaktion mit einem Bindungspartner der Zielinteraktion identisch sind.

20. Verfahren nach Anspruch 5, wobei:
(I) die proteintisen Bindungspartner der Zielinteraktion aus zwei Fusionsproteinen bestehen, wobei
(A) ein Fusionsprotein die DNA-Bindungsdomäne eines Transkriptionsfaktors und eine Aminosäurensequenz, die mit dem anderen Fusionsprotein der Zielinteraktion dimerisiert, umfasst; und
(B) ein Fusionsprotein die Transkriptionsaktivatordomäne eines Transkriptionsfaktors und eine Aminosäurensequenz, die mit dem anderen Fusionsprotein der Zielinteraktion dimerislert, umfasst,
und wobei die Dimerisierung zwischen diesen Fusionsproteinen zur Bildung eines Proteinkomplexes führt, der an die cis-wirksame Sequenz bindet und die Expression des Reportergens oder der Reportergene aktiviert, wenn die Zielinteraktion in der Wirtszelle stattfindet; und
(II) die proteinösen Bindungspartner der Nicht-Ziel-Interaktion aus zwei Fusionsproteinen bestehen, wobei
(A) ein Fusionsprotein die DNA-Bindungsdomäne eines Transkriptionsfaktors und eine Aminosäurensequenz, die mit dem anderen Fusionsprotein der Nicht-Ziel-Interaktion dimerisiert, umfasst; und
(B) ein Fusionsprotein die Transkriptionsaktivatordomäne eines Transkriptionsfaktors und eine Aminosäurensequenz, die mit dem anderen Fusionsprotein der Nicht-Ziel-Interaktion dimerisiert, umfasst,
und wobei die Dimerisierung zwischen diesen Fusionsproteinen zur Bildung eines Proteinkomplexes führt, der an die cis-wirksame Sequenz bindet und die Expression des Reportergens oder der Reportergene aktiviert, wenn die Nicht-Ziel-Interaktion in der Wirtszelle stattfindet.

21. Verfahren nach Anspruch 20, wobei:
(I) die Fusionsproteine der Zielinteraktion und die der Nicht-Ziel-Interaktion, die eine Transkriptionsaktivatordomäne umfassen, identisch sind;
(II) die Fusionsproteine der Zielinteraktion und die der Nicht-Ziel-Interaktion, die eine DNA-Bindungsdomäne umfassen, verschieden sind; und
(lll) die cis-wirksamen Sequenzen der Ziel- und Nicht-Ziel-Interaktionen verschieden sind.

22. Verfahren nach Anspruch 20, wobei:
(I) die Fusionsproteine der Zielinteraktion und die der Nicht-Ziel-Interaktion, die eine DNA-Bindungsdomäne umfassen, identisch sind;
(II) die cis-wirksamen Sequenzen der Ziel- und Nicht-Ziel-Interaktionen identisch sind; und
(III) die Fusionsproteine der Zielinteraktion und die der Nicht-Ziel-Interaktion, die eine Transkriptionsaktivatordomäne umfassen, verschieden sind.

23. Verfahren nach Anspruch 20, wobei:
(I) die Fusionsproteine der Zielinteraktion und die der Nicht-Ziel-Interaktion, die eine Transkriptionsaktivatordomäne umfassen, identisch sind;
(II) die cis-wirksamen Sequenzen der Ziel- und Nicht-Ziel-Interaktionen verschieden sind; und
(III) die Proteine der Zielinteraktion und der Nicht-Ziel-Interaktion, die mit einer DNA-Bindungsdomäne fusioniert sind, identisch sind und jedes dieser Proteine mit einer anderen DNA-Bindungsdomäne fusioniert ist.

24. Verfahren nach einem der Ansprüche 20 bis 23, wobei die cis-wirksame Sequenz der Zielinteraktion oder der Nicht-Ziel-Interaktion ausgewählt ist unter: LexA-Operator, GAL4-Bindungsstelle und cl-Operator, und wobei die DNA-Bindungsdomäne der Zielinteraktion und/oder der Nicht-Ziel-Interaktion ausgewählt ist unter: LexA-Operator-Bindungsdomäne, GAL4-DNA-Bindungsdomäne und cl-Operator-Bindungsdomäne.

25. Verfahren nach einem der Ansprüche 19 bis 24, wobei die Transkriptionsaktivatordomäne die GAL4-Aktivatordomäne ist.

26. Verfahren nach Anspruch 24 oder 25, wobei die Fusionsproteine der Zielinteraktion und die der Nicht-Ziel-Interaktion, die eine Transkriptionsaktivatordomäne umfassen, auch eine Dimerisierungsregion des SCL-Proteins umfassen, und wobei die Fusionsproteine der Zielinteraktion und die der Nicht-Ziel-Interaktion, die DNA-Bindungsdomänen umfassen, jeweils eine Dimerisierungsregion eines unter LMO1 LM02, DRG, mSin3A und E47 ausgewählten anderen Proteins umfassen.

27. Verfahren nach Anspruch 24, wobei das Fusionsprotein der Zielinteraktion die Dimerisierungsregion von LM02 umfasst und das Fusionsprotein der Nicht-Ziel-Interaktion die Dimerisierungsregion von E47 oder mSin3A umfasst, oder wobei das Fusionsprotein der Zielinteraktion die Dimerisierungsregion von E47 umfasst und das Fusionsprotein der Nicht-Ziel-Interaktion die Dimerisierungsregion von LM02 oder mSin3A umfasst.

28. Verfahren nach Anspruch 24 oder 25, wobei die Fusionsproteine der Zielinteraktion und der Nicht-Ziel-Interaktion, die eine DNA-Bindungsdomäne umfassen, auch eine Dimerisierungsregion des SCL-Proteins umfassen, und wobei die Fusionsproteine der Zielinteraktion und der Nicht-Ziel-Interaktion, die Transkriptionsaktivatordomänen umfassen, jeweils eine Dimerisierungsregion eines unter LM01, LM02, DRG, mSin3A und E47 ausgewählten anderen Proteins umfassen.

29. Verfahren nach Anspruch 28, wobei das Fusionsprotein der Zielinteraktion die Dimerisierungsregion von LM02 umfasst, und wobei das Fusionsprotein der Nicht-Ziel-Interaktion die Dimerisierungsregion von E47 umfasst.

30. Verfahren nach einem der Ansprüche 1 bis 29, wobei ein auf funktionsfähige Weise unter der Kontrolle der Zielinteraktion oder der Nicht-Ziel-Interaktion stehendes Reportergen entweder
(I) ein Fluoreszenzprotein codiert und die Detektion die Identifikation derjenigen Zellen umfasst, die nicht fluoreszieren oder eine verringerte Fluoreszenz zeigen, wenn das Reportergen nicht exprimiert wird, verglichen zur Situation bei Expression des Reportergens; oder
(II) ein gegenselektierbares Reportergen ist, das ein Polypeptid codiert, das im Stande ist, ein ungiftiges Substrat in ein giftiges Produkt umzuwandeln, und die Detektion die Identifikation derjenigen Zellen umfasst, die überleben oder wachsen, wenn das Reportergen nicht exprimiert wird.

31. Verfahren nach Anspruch 30, wobei das Reportergen das GFP-Gen oder das cobA-Gen oder eine Variante oder ein Fragment des GFP-Gens oder des cobA-Gens ist, das bzw. die ein Fluoreszenzprotein codiert, oder ein unter URA3, CYH2 und LYS2 ausgewähltes gegenselektierbares Reportergen ist.

32. Verfahren nach Anspruch 30, wobei ein gegenselektierbares Reportergen, das auf funktionsfähige Weise unter der Kontrolle der Nlcht-Ziel-Interaktion steht, LYS2 oder LEU2 ist.

33. Verfahren nach Anspruch 1, wobei zwei gegenselektierbare Reportergene, bestehend aus URA3 und CYH, aus URA3 und LYS2, oder aus LYS2 und CYH2, in funktionsfähiger Weise unter der Kontrolle der Zielinteraktion stehen.

34. Verfahren nach einem der Ansprüche 1 bis 33, wobei mehrfache Reportergene in funktionsfähiger Weise unter der Kontrolle der Zielinteraktion stehen und mindestens ein gegenselektierbares Reportergen und mindestens ein ein Fluoreszenzprotein codierendes Gen umfassen, so dass die Detektion die Identifikation derjenigen Zellen umfasst, die überleben oder wachsen und nicht fluoreszieren oder eine verringerte Fluoreszenz zeigen, wenn das Reportergen nicht exprimiert wird, verglichen zur Situation bei Expression des Reportergens.

35. Verfahren nach einem der Ansprüche 1 bis 34, wobei mehrfache Reportergene in funktionsfähiger Weise unter der Kontrolle der Nicht-Ziel-Interaktion stehen und wobei die Reportergene mindestens ein gegenselektierbares Reportergen und mindestens ein ein Fluoreszenzprotein codierendes Gen umfassen.

36. Verfahren nach einem der Ansprüche 1 bis 35, wobei das Kandidatenpeptid in einer konformationell gespannten Form innerhalb einer Trx-Polypeptidschleife exprimiert wird oder oxydierte flankierende Cysteinreste umfasst.

37. Verfahren nach einem der Ansprüche 1 bis 36, weiterhin umfassend Selektion und Wachstum der detektierten Zellen.

38. Verwendung eines Hefe-Shuttlevektors, der im Stande ist, einen Kandidaten für ein inhibitorisches Peptid im Verfahren nach einem der Ansprüche 1 bis 37 zu exprimieren, wobei der Vektor ortsspezifische Rekombinasestellen umfasst, die eine das Kandidatenpeptid codierende Sequenz flankieren.

39. Verwendung eines Vektors, der eine wie in einer unter SEQ ID NO:1 bis SEQ ID NO:10 ausgewählten Sequenz angegebene Nukleotidsequenz umfasst, im Verfahren nach einem der Ansprüche 1 bis 38.

40. Verwendung eines Vektors zur Expression roter und grüner Fluoreszenzproteine in Hefe im Verfahren nach einem der Ansprüche 1 bis 37, wobei dieser Vektor
(I) eine ein grünes Fluoreszenzprotein exprimierende Kassette, umfassend das gfp-Gen, auf funktionsfähige Weise unter der Kontrolle eines in Hefe funktionsfähigen chimären LexA/GAL1-Promotors mit mehrfachen LexA-Operatorstellen stehend; und
(II) eine ein rotes Fluoreszenzprotein exprimierende Kassette, umfassend das cobA-Gen, auf funktionsfähige Weise unter der Kontrolle eines in Hefe funktionsfähigen chimären cl/GAL1-Promotors mit mehrfachen cl-Operatorstellen stehend,
umfasst.

41. Verwendung nach Anspruch 40, wobei der Vektor die Strukturmerkmale zur Expression des im Vektor pRT2 (SEQ ID NO:10) enthaltenen Fluoreszenzproteins besitzt.

42. Verfahren nach einem der Ansprüche 1 bis 41, zusätzlich umfassend:
(IV) Isolierung einer Nukleotidsequenz, die einen Peptidinhibitor codiert, der teilweise oder vollständig die Zielinteraktion inhibiert;
(V) Transfektion der eukaryontischen Zelle mit der isolierten Nukleinsäure; und
(VI) Vergleich des Phänotyps oder Expressionsmusters der transfizierten eukaryontischen Zelle mit dem Phänotyp oder Expressionsmuster einer ansonsten isogenen untransfizierten Zelle, wobei ein unterschiedlicher Phänotyp oder ein unterschiedliches Expressionsmuster anzeigt, dass das Peptid eine Wirkung auf die Zelle hat.

43. Verfahren nach Anspruch 42, wobei die eukaryontische Zelle eine Säugerzelle ist.

44. Verfahren nach Anspruch 42 oder 43, wobei der Vergleich des Expressionsmusters der transfizierten eukaryontischen Zelle mit dem Expressionsmuster der ansonsten isogenen untransfizierten Zelle durchgeführt wird, indem man ein Array der von den transfizierten und der von den untransfizierten Zellen gebildeten Proteine oder Nukleinsäuren herstellt und diese Proteine oder Nukleinsäuren vergleicht.

45. Verfahren nach Anspruch 44 oder 45, wobei der Phänotyp der transfizierten Zelle mit dem Phänotyp der untransfizierten Zelle verglichen wird.

46. Verfahren zur Identifikation eines Peptids zur vorbeugenden oder therapeutischen Behandlung eine Säugers, wobei man das Verfahren nach einem der Ansprüche 44 bis 45 an einer erkrankten Zelle durchführt und ein Peptid selektiert, das den Phänotyp oder das Expressionsprofil der transfizierten Zelle revertiert.

47. Verfahren zur Identifikation eines Bindungspartners oder pharmakologischen Ziels, wobei man das Verfahren nach Anspruch 44 durchführt und die Nukleinsäure oder das Protein, die oder das in der transfizierten Zelle verändert ist, identifiziert.

## Revendications

1. Procédé d'identification d'un peptide qui inhibe partiellement ou complètement une interaction cible entre deux partenaires de liaison ou plus dans une cellule hôte, mais qui n'inhibe pas une interaction non cible entre certains, mais pas tous, desdits partenaires de liaison, ledit procédé comprenant :
(i) l'expression dans un hôte cellulaire : (a) des partenaires de liaison de ladite interaction cible de sorte qu'ils contrôlent de manière fonctionnelle l'expression d'un ou de plusieurs gènes rapporteurs dans ledit hôte cellulaire, où ladite expression est partiellement ou complètement inhibée par la rupture de ladite interaction cible ; (b) des partenaires de liaison de ladite interaction non cible de sorte qu'ils contrôlent de manière fonctionnelle l'expression d'un ou de plusieurs gènes rapporteurs dans ledit hôte cellulaire, où ladite expression est partiellement ou complètement inhibée par la rupture de ladite interaction non cible et où ledit gène rapporteur est distinct du ou des gènes rapporteurs exprimés sous le contrôle de l'interaction cible ; et (c) d'un peptide candidat ; où un ou plusieurs gènes rapporteurs placés fonctionnellement sous le contrôle de l'interaction cible ou de l'interaction non cible est un gène rapporteur contre-sélectionnable qui confère une létalité conditionnelle à l'hôte cellulaire et où, pour déterminer la concentration minimale de l'expression du partenaire de liaison requise pour que le gène rapporteur contre-sélectionnable soit exprimé à un niveau qui provoque la mort cellulaire, l'expression d'un ou de plusieurs partenaires de liaison est titrée en réponse au galactose ou au cuivre en plaçant ladite expression sous le contrôle d'un promoteur comprenant des éléments régulateurs des promoteurs *GAL1* et *CUP1 ;*
(ii) la croissance de l'hôte cellulaire dans des conditions suffisantes pour distinguer l'expression de chaque gène rapporteur au niveau (a) à partir de l'expression du gène rapporteur au niveau (b) ; et
(iii) la détection des cellules hôtes dans lesquelles l'expression du ou des gènes rapporteurs placés de manière fonctionnelle sous le contrôle de l'interaction cible est partiellement ou complètement inhibée et l'expression du ou des gènes placés de manière fonctionnelle sous le contrôle de l'interaction non cible n'est pas inhibée, lesdites cellules détectées exprimant un peptide qui inhibe partiellement ou complètement l'interaction.cible.

2. Procédé selon la revendication 1, dans lequel : (i) les un ou plusieurs gènes rapporteurs contre-sélectionnables sont CYH2 et URA3 ; (ii) l'expression d'un ou de plusieurs partenaires de liaison interagissant est sous le contrôle du promoteur GAL1 pour faciliter la détermination de la concentration minimale de l'expression du partenaire de liaison requise pour qu'un gène rapporteur contre-sélectionnable soit exprimé à un niveau qui provoque la mort cellulaire à une concentration prédéterminée de 5-FOA ou de cycloheximide.

3. Procédé selon la revendication 1 ou 2, dans lequel l'expression d'un ou de plusieurs desdits partenaires de liaison interagissant est sous le contrôle d'un promoteur comprenant des éléments régulateurs du promoteur *GAL1.*

4. Procédé selon la revendication 2 ou 3, dans lequel l'expression des partenaires de liaison interagissant est variée en modulant le taux de galactose dans les milieux entre un pas de galactose et 2 % (p/v) de galactose et la concentration en raffinose est maintenue à une concentration de 2 % (p/v).

5. Procédé selon l'une quelconque des revendications 1 à 4, dans lequel :
(i) l'interaction cible implique deux ou plusieurs partenaires de liaison protéiques, où l'un desdits partenaires se lie à l'acide nucléique comprenant une séquence agissant en *cis* et l'autre desdits partenaires active la transcription du gène ou des gènes rapporteurs de l'interaction cible quand l'interaction cible se produit dans la cellule hôte ; et
(ii) l'interaction non cible implique deux partenaires de liaison protéiques ou plus, où l'un desdits partenaires se lie à l'acide nucléique comprenant une séquence agissant en *cis* et l'autre desdits partenaires active la transcription du ou des gènes rapporteurs de l'interaction non cible quand l'interaction non cible se produit dans la cellule hôte.

6. Procédé selon la revendication 5, dans lequel l'interaction cible est entre deux partenaires de liaison protéiques, comprenant chacun un domaine de liaison à l'ARN et une molécule d'ARN hybride capable de se lier aux dits domaines de liaison à l'ARN, où un desdits partenaires de liaison protéiques se lie à l'acide nucléique comprenant une séquence agissant en *cis* et l'autre desdits partenaires de liaison protéiques active la transcription du ou des gènes rapporteurs contre-sélectionnables de l'interaction cible, quand l'interaction cible se produit dans la cellule hôte.

7. Procédé selon l'une quelconque des revendications 1 à 6, comprenant en outre la première étape consistant à introduire dans l'hôte cellulaire un ou plusieurs acides nucléiques qui comprennent une séquence choisie dans le groupe constitué de :
(i) une séquence codant un partenaire de liaison de l'interaction cible dans un format exprimable ;
(ii) une séquence codant un partenaire de liaison de l'interaction non cible dans un format exprimable ;
(iii) une séquence codant un domaine d'activation de l'interaction cible dans un format exprimable ;
(iv) une séquence codant un domaine d'activation de l'interaction non cible dans un format exprimable ;
(v) une séquence codant un domaine de liaison à l'ADN de l'interaction cible dans un format exprimable ;
(vi) une séquence codant un domaine de liaison à l'ADN de l'interaction non cible dans un format exprimable ;
(vii) une séquence codant le peptide candidat dans un format exprimable ;
(viii) une séquence comprenant une séquence agissant en *cis* et un gène rapporteur dans un format exprimable ; et
(ix) une séquence comprenant une séquence agissant en *cis* et un gène rapporteur contre-sélectionnable dans un format exprimable.

8. Procédé selon la revendication 7, comprenant en outre la conjugaison des cellules ayant un ou plusieurs desdits acides nucléiques de manière à combiner suffisamment d'acides nucléiques dans une unique cellule pour sélectionner les cellules hôtes qui croissent ou survivent quand les gènes rapporteurs contre-sélectionnables placés de manière fonctionnelle sous le contrôle de l'interaction cible sont exprimés.

9. Procédé selon la revendication 7, dans lequel l'acide nucléique codant un partenaire de liaison ou un peptide candidat code aussi un signal de localisation nucléaire (SLN) pour faciliter la localisation nucléaire dudit partenaire de liaison ou dudit peptide candidat.

10. Procédé selon l'une quelconque des revendications 7 à 9, dans lequel un ou plusieurs desdits acides nucléiques sont placés de manière fonctionnelle sous le contrôle d'un promoteur choisi dans le groupe constitué par : *MYC, GAL1, CUP1, PGK1, ADH1, ADH2, PHO4, PHO5, HIS4, HIS5, TEF1 , PRB1, GUT1, SPO13, CMV, SV40, LAC, EM7, SV40* et *T7.*

11. Procédé selon l'une quelconque des revendications 7 à 10, dans lequel un ou plusieurs desdits acides nucléiques sont contenus dans un vecteur choisi dans le groupe constitué de pBLOCK-3.0 (SEQ ID N°1); pBLOCK-3.2 (SEQ ID N°2); pBLOCK-3.4 (SEQ ID N°3); pBLOCK-3.6 (SEQ ID N°4); pBLOCK-3.8 (SEQ ID N°5); pBLOCK-3.9 (SEQ ID N°6); pBLOCK-3.10 (SEQ ID N°7); pBLOCK-3.11 (SEQ ID N°8); pBLOCK-4.0 (SEQ ID N°9); et pRT2 (SEQ ID N°10).

12. Procédé selon l'une quelconque des revendications 1 à 11, dans lequel l'hôte cellulaire est une cellule de levure.

13. Procédé selon la revendication 12 dans lequel la cellule de levure possède le génotype *MATα, ura3, trp1, his3, cyh2*^{*R*}*, lexAop- URA3, lexAop-CYH2, ade2* ou *MATa, cyh2*^{*R*}*, his3, trp1, 1exAop-LEU2, lexAop-CYH2, ade2::ZEO*^{*R*}*, 1exAop-URA3, his5: :G418*^{*R*}*, clop-LYS.*

14. Procédé selon la revendication 12 ou 13, comprenant .
(i) la transformation d'une cellule de levure avec un vecteur sélectionné dans le groupe constitué de : pBLOCK-3.0 (SEQ ID N°1); pBLOCK-3.2 (SEQ ID N°2); pBLOCK-3.4 (SEQ ID N°3); pBLOCK-3.6 (SEQ ID N°4); pBLOCK-3.8 (SEQ ID N°5); pBLOCK-3.9 (SEQ ID N°6); pBLOCK-3.10 (SEQ ID N°7); pBLOCK-3.11 (SEQ ID N°8) et pBLOCK-4.0 (SEQ ID N°9), où ledit vecteur comprend en outre un acide nucléique codant un peptide candidat testé pour son activité inhibitrice ;
(ii) l'introduction dans ladite cellule de levure transformée d'acide nucléique codant :
(a) les partenaires de liaison de ladite interaction cible de sorte qu'ils contrôlent de manière fonctionnelle l'expression d'un ou de plusieurs gènes rapporteurs contre-sélectionnables ou gènes rapporteurs codant une protéine fluorescente dans ledit hôte cellulaire, où ladite expression est partiellement ou complètement inhibée par la rupture de ladite interaction cible ; et
(b) les partenaires de liaison de ladite interaction non cible de sorte qu'ils contrôlent de manière fonctionnelle l'expression d'un ou de plusieurs gènes rapporteurs contre-sélectionnables ou de gènes rapporteurs codant une protéine fluorescente dans ledit hôte cellulaire, où ladite expression est partiellement ou complètement inhibée par la rupture de ladite interaction non cible et où ledit gène rapporteur est distinct du ou des gènes rapporteurs exprimés sous le contrôle de l'interaction cible ;
(iii) la sélection des recombinants ;
(iv) la croissance des recombinants dans des conditions suffisantes pour distinguer l'expression de chaque gène rapporteur au niveau (a) de l'expression du ou des gènes rapporteurs au niveau (b) ; et
(v) la détection des cellules hôtes dans lesquelles l'expression du ou des gènes rapporteurs placés de manière fonctionnelle sous le contrôle de l'interaction cible est partiellement ou complètement inhibée et l'expression du ou des gènes rapporteurs placés de manière fonctionnelle sous le contrôle de l'interaction non cible n'est pas inhibée, lesdites cellules détectées exprimant un peptide qui inhibe partiellement ou complètement l'interaction cible.

15. Procédé selon la revendication 14, dans lequel l'acide nucléique est introduit à l'étape (ii) par transformation et les recombinants choisis à l'étape (iii) sont les transformants produits par ladite transformation, ou dans lequel l'acide nucléique est introduit à l'étape (ii) par conjugaison cellulaire et les recombinants choisis à l'étape (iii) sont des diploïdes provenant de ladite conjugaison cellulaire.

16. Procédé selon la revendication 14 ou 15, dans lequel les gènes rapporteurs codant une protéine fluorescente sont introduits dans la cellule par transformation de la cellule avec le vecteur pRT2 (SEQ ID N°11) ou par conjugaison de la cellule avec une cellule de levure contenant ledit vecteur.

17. Procédé selon la revendication 14, dans lequel l'acide nucléique codant un ou plusieurs des partenaires de liaison de l'interaction cible et/ou de l'interaction non cible est introduit dans la cellule par transformation de la cellule avec un dérivé d'un vecteur sélectionné dans le groupe constitué de pBLOCK-3.0 (SEQ ID N°1); pBLOCK-3.2 (SEQ ID N°2); pBLOCK-3.4 (SEQ ID N°3); pBLOCK-3.6 (SEQ ID N°4); pBLOCK-3.8 (SEQ ID N°5); pBLOCK-3.9 (SEQ ID N°6); pBLOCK-3.10 (SEQ ID N°7); pBLOCK-3.11 (SEQ ID N°8) et pBLOCK-4.0 (SEQ ID N°9), où ledit dérivé comprend une séquence de nucléotides codant ledit partenaire de liaison.

18. Procédé selon la revendication 1, dans lequel les partenaires de liaison de ladite interaction cible contrôlent de manière fonctionnelle et simultanée l'expression de deux gènes rapporteurs distincts dans ledit hôte cellulaire.

19. Procédé selon l'une quelconque des revendications 1 à 18, dans lequel un ou plusieurs des partenaires de liaison de l'interaction non cible sont les mêmes qu'un partenaire de liaison de l'interaction cible.

20. Procédé selon la revendication 5, dans lequel :
(i) les partenaires de liaison protéiques de l'interaction cible se composent de deux protéines de fusion, où (a) une protéine de fusion comprend le domaine de liaison à l'ADN d'un facteur de transcription et une séquence d'acides aminés qui se dimérise avec l'autre protéine de fusion de l'interaction cible ; et (b) une protéine de fusion comprend le domaine activateur de transcription d'un facteur de transcription et une séquence d'acides aminés qui se dimérise avec l'autre protéine de fusion de l'interaction cible, et où la dimérisation entre lesdites protéines de fusion produit un complexe de protéine qui se lie à la séquence agissant en *cis* et active l'expression du ou des gènes rapporteurs quand l'interaction cible se produit dans la cellule hôte ; et
(ii) les partenaires de liaison protéiques de l'interaction non cible se composent de deux protéines de fusion, où (a) une protéine de fusion comprend le domaine de liaison à l'ADN d'un facteur de transcription et une séquence d'acides aminés qui se dimérise avec l'autre protéine de fusion de l'interaction non cible ; et (b) une protéine de fusion comprend le domaine activateur de transcription d'un facteur de transcription et une séquence d'acides aminés qui se dimérise avec l'autre protéine de fusion de l'interaction non cible, et où la dimérisation entre lesdites protéines de fusion produit un complexe de protéine qui se lie à la séquence agissant en *cis* et active l'expression du ou des gènes rapporteurs quand l'interaction non cible se produit dans la cellule hôte.

21. Procédé selon la revendication 20, dans lequel :
(i) les protéines de fusion de l'interaction cible et de l'interaction non cible qui comprennent un domaine activateur de transcription sont les mêmes ;
(ii) les protéines de fusion des interactions cibles et non cibles qui comprennent un domaine de liaison à l'ADN sont différentes ; et
(iii) les séquences agissant en *cis* des interactions cibles et non cibles sont différentes.

22. Procédé selon la revendication 20, dans lequel :
(i) les protéines de fusion des interactions cibles et non cibles qui comprennent un domaine de liaison à l'ADN sont les mêmes ; et
(ii) les séquences agissant en *cis* des interactions cibles et non cibles sont les mêmes ; et
(iii) les protéines de fusion de l'interaction cible et de l'interaction non cible qui comprennent un domaine activateur de transcription sont différentes.

23. Procédé selon la revendication 20, dans lequel :
(i) les protéines de fusion des interactions cibles et non cibles qui comprennent un domaine activateur de transcription sont les mêmes ; et
(ii) les séquences agissant en *cis* des interactions cibles et non cibles sont différentes ; et
(iii) les protéines de l'interaction cible et de l'interaction non cible qui sont fusionnées à un domaine de liaison à l'ADN sont les mêmes et chacune desdites protéines est fusionnée à un domaine de liaison à l'ADN différent.

24. Procédé selon l'une quelconque des revendications 20 à 23, dans lequel la séquence agissant en *cis* de l'interaction cible ou de l'interaction non cible est sélectionnée dans le groupe constitué de : l'opérateur LexA, du site de liaison GAL4, et de l'opérateur *c1* et dans lequel le domaine de liaison à l'ADN de l'interaction cible et/ou de l'interaction non cible est sélectionné dans le groupe constitué du : domaine de liaison de l'opérateur LexA, domaine de liaison à l'ADN GAL4 ; et domaine de liaison à l'opérateur *cl*.

25. Procédé selon l'une quelconque des revendications 19 à 24, dans lequel le domaine activateur de transcription est le domaine activateur GAL4.

26. Procédé selon la revendication 24 ou 25, dans lequel les protéines de fusion de l'interaction cible et de l'interaction non cible qui comprennent un domaine activateur de transcription comprennent aussi une région de dimérisation de la protéine SCL et dans lequel les protéines de fusion des interactions cibles et non cibles qui comprennent des domaines de liaison à l'ADN comprennent chacune une région de dimérisation d'une protéine distincte choisie dans le groupe constitué de : LMO1, LMO2, DRG, mSin3A et E47.

27. Procédé selon la revendication 24, dans lequel la protéine de fusion de l'interaction cible comprend la région de dimérisation de LMO2 et dans lequel la protéine de fusion de l'interaction non cible comprend la région de dimérisation de E47 ou mSin3A, ou dans lequel la protéine de fusion de l'interaction cible comprend la région de dimérisation de E47 et dans lequel la protéine de fusion de l'interaction non cible comprend la région de dimérisation de LMO2 ou mSin3A.

28. Protéine selon la revendication 24 ou 25, dans laquelle les protéines de fusion de l'interaction cible et de l'interaction non cible qui comprennent un domaine de liaison à l'ADN comprennent aussi une région de dimérisation de la protéine SCL et dans lequel les protéines de fusion des interactions cibles et non cibles qui comprennent des domaines activateurs de transcription comprennent chacune une région de dimérisation de la protéine distincte choisie dans le groupe constitué de LMO1, LMO2, DRG, mSin3A et E47.

29. Procédé selon la revendication 28, dans lequel la protéine de fusion de l'interaction cible comprend la région de dimérisation de LMO2 et dans lequel la protéine de fusion de l'interaction non cible comprend la région de dimérisation de E47.

30. Procédé selon l'une quelconque des revendications 1 à 29, dans lequel un gène rapporteur placé de manière fonctionnelle sous le contrôle de l'interaction cible ou de l'interaction non cible soit (i) code une protéine fluorescente et où ladite détection comprend l'identification des cellules qui n'émettent pas de fluorescence ou ont une fluorescence réduite quand ledit gène rapporteur n'est pas exprimé par rapport quand le gène rapporteur est exprimé, soit (ii) est un gène rapporteur contre-sélectionnable qui code un polypeptide capable de convertir un substrat non toxique en un produit toxique et où ladite détection comprend l'identification des cellules qui croissent ou survivent quand ledit gène rapporteur contre-sélectionnable n'est pas exprimé.

31. Procédé selon la revendication 30, dans lequel le gène rapporteur est le gène *GFP* ou le gène *cobA* ou un variant ou un fragment dudit gène *GFP* ou dudit gène *cobA* qui code une protéine fluorescente, ou est un gène rapporteur contre-sélectionnable choisi dans le groupe constitué de : URA3, CYH2 et LYS2.

32. Procédé selon la revendication 30, dans lequel un gène rapporteur contre-sélectionnable placé de manière fonctionnelle sous le contrôle de l'interaction non cible est LYS2 ou LEU2.

33. Procédé selon la revendication 1 dans lequel deux gènes rapporteurs contre-sélectionnables consistant en URA3 et CYH, URA3 et LYS2, ou LYS2 et CYH2 sont de manière fonctionnelle sous le contrôle de l'interaction cible.

34. Procédé selon l'une quelconque des revendications 1 à 33, dans lequel de multiples gènes rapporteurs sont de manière fonctionnelle sous le contrôle de l'interaction cible et dans lequel lesdits gènes rapporteurs comprennent au moins un gène rapporteur contre-sélectionnable et au moins un gène codant une protéine fluorescente de sorte que la détection comprenne l'identification des cellules qui croissent ou survivent et n'émettent pas de fluorescence ou ont une fluorescence réduite quand ledit gène rapporteur n'est pas exprimé par rapport quand ledit gène rapporteur est exprimé.

35. Procédé selon l'une quelconque des revendications 1 à 34, dans lequel de multiples gènes rapporteurs sont de manière fonctionnelle sous le contrôle de l'interaction non cible et dans lequel lesdits gènes rapporteurs comprennent au moins un gène rapporteur contre-sélectionnable et au moins un gène codant une protéine fluorescente.

36. Procédé selon l'une quelconque des revendications 1 à 35, dans lequel le peptide candidat est exprimé dans une forme conformationnellement contrainte à l'intérieur d'une boucle de polypeptide Trx ou comprenant des résidus de cystéine flanquants oxydés.

37. Procédé selon l'une quelconque des revendications 1 à 36, comprenant en outre la sélection et la croissance des cellules détectées.

38. Utilisation d'un vecteur navette de levure capable d'exprimer un peptide inhibiteur candidat dans le procédé selon l'une quelconque des revendications 1 à 37 dans laquelle le vecteur comprend des sites de recombinase spécifiques à un site, flanquant une séquence codant le peptide candidat.

39. Utilisation d'un vecteur comprenant une séquence nucléotidique telle qu'elle est exposée dans une séquence choisie dans le groupe constitué des SEQ ID N°1 à 10 dans le procédé selon l'une quelconque des revendications 1 à 38.

40. Utilisation d'un vecteur pour l'expression de protéines fluorescentes rouges et vertes dans la levure dans le procédé selon l'une quelconque des revendications 1 à 37, ledit vecteur comprenant :
(i) une cassette d'expression d'une protéine fluorescente verte comprenant le gène *gfp* placé de manière fonctionnelle sous le contrôle d'un promoteur *LexA*/*GAL1* chimérique fonctionnel chez la levure ayant des sites opérateurs *LexA* multiples ; et
(ii) une cassette d'expression d'une protéine rouge fluorescent comprenant le gène *cobA* placé de manière fonctionnelle sous le contrôle d'un promoteur chimérique *cl*/*GAL1* ayant des sites opérateurs cl multiples.

41. Utilisation selon la revendication 40, dans laquelle le vecteur possède les caractéristiques structurelles pour l'expression de protéines fluorescentes telles qu'elles sont présentes dans le vecteur pRT2 (SEQ ID N°10).

42. Procédé selon l'une quelconque des revendications 1 à 41 comprenant en plus :
(iv) l'isolement d'une séquence de nucléotides codant un peptide inhibiteur qui inhibe partiellement ou complètement l'interaction cible ;
(v) la transfection de ladite cellule eucaryote avec l'acide nucléique isolé ; et
(vi) la comparaison du phénotype ou du mode d'expression de la cellule eucaryote transfectée avec le phénotype ou le mode d'expression d'une cellule non transfectée sinon isogénique, où un phénotype ou un mode d'expression différent indique que le peptide possède un effet sur la cellule.

43. Procédé selon la revendication 42, dans lequel la cellule eucaryote est une cellule de mammifère.

44. Procédé selon la revendication 42 ou 43, dans lequel la comparaison du mode d'expression de la cellule eucaryote transfectée avec le mode d'expression d'une cellule non transfectée sinon isogénique est réalisée en produisant une cartographie des protéines ou des acides nucléiques exprimés par les cellules transfectées et non transfectées et en comparant lesdites protéines acides nucléiques.

45. Procédé selon la revendication 44 ou la revendication 45, dans lequel le phénotype de la cellule transfectée est comparé au phénotype de la cellule non transfectée.

46. Procédé pour l'identification d'un peptide destiné au traitement prophylactique ou thérapeutique d'un mammifère comprenant la réalisation du procédé selon l'une quelconque des revendications 44 à 45 sur une cellule malade et la sélection d'un peptide qui renverse le phénotype ou le profil d'expression de la cellule transfectée.

47. Procédé pour l'identification d'un partenaire de liaison ou d'une cible de médicament comprenant la réalisation du procédé selon la revendication 44 et l'identification de l'acide nucléique ou de la protéine qui est modifié dans la cellule transfectée.


### SEQUENCE LISTING

<110> TVW Telethon Institute for Child Health Research
<120> Improved Reverse N-Hybrid Screening Method
<130> multiple reporter pct.doc
<160> 10
<170> PatentIn version 3.0
<210> 1
   <211> 6766
   <212> DNA
   <213> synthetic
<400> 1
   gacgaaaggg cctcgtgata cgcctatttt tataggttaa tgtcatgata ataatggttt      60
   cttagacgtc aggtggcact tttcggggaa atgtgcgcgg aacccctatt tgtttatttt     120
   tctaaataca ttcaaatatg tatccgctca tgagacaata accctgataa atgcttcaat     180
   aatattgaaa aaggaagagt atgagtattc aacatttccg tgtcgccctt attccctttt     240
   ttgcggcatt ttgccttcct gtttttgctc acccagaaac gctggtgaaa gtaaaagatg     300
   ctgaagatca gttgggtgca cgagtgggtt acatcgaact ggatctcaac agcggtaaga     360
   tccttgagag ttttcgcccc gaagaacgtt ttccaatgat gagcactttt aaagttctgc     420
   tatgtggcgc ggtattatcc cgtattgacg ccgggcaaga gcaactcggt cgccgcatac     480
   actattctca gaatgacttg gttgagtact caccagtcac agaaaagcat cttacggatg     540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat ttttttattt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcacc atgggtaagc ctatccctaa ccctctcctc ggtctcgatt 5100
   ctacacaagc tatgggtgct cctccaaaaa agaagagaaa ggtagctgaa ttcgagctca 5160
   gatctcagct gggcccggta ccaattgatg catcgatacc ggtactagtc ggaccgcata 5220
   tgcccgggcg taccgcggcc gctcgagtcg acctgcagcc aagctaattc cgggcgaatt 5280
   tcttatgatt tatgattttt attattaaat aagttataaa aaaaataagt gtatacaaat 5340
   tttaaagtga ctcttaggtt ttaaaacgaa aattcttgtt cttgagtaac tctttcctgt   5400
   aggtcaggtt gctttctcag gtatagcatg aggtcgctct tattgaccac acctctaccg   5460
   gcatgccgag caggggatcc tctagagtcg acctgcataa taaaactagt aataaagaag   5520
   gcgcgccgtg tgttggtttt ttgtgtgatg caggcatgca agcttggcac tggccgtcgt   5580
   tttacaacgt cgtgactggg aaaaccctgg cgttacccaa cttaatcgcc ttgcagcaca   5640
   tccccctttc gccagctggc gtaatagcga agaggcccgc accgatcgcc cttcccaaca   5700
   gttgcgcagc ctgaatggcg aatcgttcaa ttgccttaat taacagtgtg ctgcaattcg   5760
   cccttgagag ccgggagcgc taatttttca aacaaagaat ctgagctgca tttttacaga   5820
   acagaaatgc aacgcgagag cgctatttta ccaacaaaga atctatactt cttttttgtt   5880
   ctacaaaaat gcatcccgag agcgctattt ttctaacaaa gcatcttaga ttactttttt   5940
   tctcctttgt gcgctctata atgcagtctc ttgataactt tttgcactgt aggtccgtta   6000
   aggttagaag aaggctactt tggtgtctat tttctcttcc ataaaaaaag cctgactcca   6060
   cttcccgcgt ttactgatta ctagcgaagc tgcgggtgca ttttttcaag ataaaggcat   6120
   ccccgattat attctatacc gatgtggatt gcgcatactt tgtgaacaga aagtgatagc   6180
   gttgatgatt cttcattggt cagaaaatta tgaacggttt cttctatttt gtctctatat   6240
   actacgtata ggaaatgttt acattttcgt attgttttcg attcactcta tgaatagttc   6300
   ttactacaat ttttttgtct aaagagtaat actagagata aacataaaaa atgtagaggt   6360
   cgagtttaga tgcaagttca aggagcgaaa ggtggatggg taggttatat agggatatag   6420
   cacagagata tatagcaaag agatactttt gagcaatgtt tgtggaagcg gtattcgcaa   6480
   tattcgcgcc tctcaagggc gaattgtgca gatttcttaa ttaaaacgcc tgatgcggta   6540
   ttttctcctt acgcatctgt gcggtatttc acaccgcata tatggtgcac tctcagtaca   6600
   atctgctctg atgccgcata gttaagccag ccccgacacc cgccaacacc cgctgacgcg   6660
   ccctgacggg cttgtctgct cccggcatcc gcttacagac aagctgtgac cgtctccggg   6720
   agctgcatgt gtcagaggtt ttcaccgtca tcaccgaaac gcgcga                  6766
<210> 2
   <211> 6735
   <212> DNA
   <213> synthetic
<400> 2
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtyaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcacc atgggtaagc ctatccctaa ccctctcctc ggtctcgatt 5100
   ctacacaagc tatgggtgct cctccaaaaa agaagagaaa ggtagctgaa ttgactttgt 5160
   acaaaaaagc aggctgaatt cgagctcaga tctcagctgg gcccggtacc aattgatgca 5220
   tcgataccgg tactagtcgg accgcatatg cccggtaccc agctttcttg tacaaagccc 5280
   gggcgtaccg cggccgctcg agtcgacctg cagccaagct aattccgggc gaatttctta 5340
   tgatttatga tttttattat taaataagtt ataaaaaaaa taagtgtata caaattttaa 5400
   agtgactctt aggttttaaa acgaaaattc ttgttcttga gtaactcttt cctgtaggtc 5460
   aggttgcttt ctcaggtata gcatgaggtc gctcttattg accacacctc taccggcatg 5520
   ccgagcaggg gatcctctag agtcgacctg cataataaaa ctagtaataa agaaggcgcg 5580
   ccgtgtgttg gttttttgtg tgatgcaggc atgcaagctt ggcactggcc gtcgttttac 5640
   aacgtcgtga ctgggaaaac cctggcgtta cccaacttaa tcgccttgca gcacatcccc 5700
   ctttcgccag ctggcgtaat agcgaagagg cccgcaccga tcgcccttcc caacagttgc 5760
   gcagcctgaa tggcgaatgg cgggagcgct aatttttcaa acaaagaatc tgagctgcat 5820
   ttttacagaa cagaaatgca acgcgagagc gctattttac caacaaagaa tctatacttc 5880
   ttttttgttc tacaaaaatg catcccgaga gcgctatttt tctaacaaag catcttagat 5940
   tacttttttt ctcctttgtg cgctctataa tgcagtctct tgataacttt ttgcactgta 6000
   ggtccgttaa ggttagaaga aggctacttt ggtgtctatt ttctcttcca taaaaaaagc 6060
   ctgactccac ttcccgcgtt tactgattac tagcgaagct gcgggtgcat tttttcaaga 6120
   taaaggcatc cccgattata ttctataccg atgtggattg cgcatacttt gtgaacagaa 6180
   agtgatagcg ttgatgattc ttcattggtc agaaaattat gaacggtttc ttctattttg 6240
   tctctatata ctacgtatag gaaatgttta cattttcgta ttgttttcga ttcactctat 6300
   gaatagttct tactacaatt tttttgtcta aagagtaata ctagagataa acataaaaaa 6360
   tgtagaggtc gagtttagat gcaagttcaa ggagcgaaag gtggatgggt aggttatata 6420
   gggatatagc acagagatat atagcaaaga gatacttttg agcaatgttt gtggaagcgg 6480
   tattcgcaat attggcgcct gatgcggtat tttctcctta cgcatctgtg cggtatttca 6540
   caccgcatat atggtgcact ctcagtacaa tctgctctga tgccgcatag ttaagccagc 6600
   cccgacaccc gccaacaccc gctgacgcgc cctgacgggc ttgtctgctc ccggcatccg 6660
   cttacagaca agctgtgacc gtctccggga gctgcatgtg tcagaggttt tcaccgtcat 6720
   caccgaaacg cgcga 6735
<210> 3
   <211> 7130
   <212> DNA
   <213> synthetic
<400> 3
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcacc atgggtaagc ctatccctaa ccctctcctc ggtctcgatt 5100
   ctacacaagc tatgggtgct cctccaaaaa agaagagaaa ggtagctgaa ttggcgcaac 5160
   gcaattaatg tgagttagct cactcattag gcaccccagg ctttacactt tatgcttccg 5220
   gctcgtatgt tgtgtggaat tgtgagcgga taacaatttc acacaggaaa cagctatgga 5280
   attcgagctc agatctcagc tgggcccggt accaattgat gcatcgatac cggtactagt 5340
   cggaccgcat atgcccggtt acgtacggca gtttaaggtt tacacctata aaagagagag 5400
   ccgttatcgt ctgtttgtgg atgtacagag tgatattatt gacacgccgg ggcgacggat 5460
   ggtgatcccc ctggccagtg cacgtctgct gtcagataaa gtctcccgtg aactttaccc 5520
   ggtggtgcat atcggggatg aaagctggcg catgatgacc accgatatgg ccagtgtgcc 5580
   ggtctccgtt atcggggaag aagtggctga tctcagccac cgcgaaaatg acatcaaaaa 5640
   cgccattaac ctgatgttct ggggaatata agcccgggcg taccgcggcc gctcgagtcg 5700
   acctgcagcc aagctaattc cgggcgaatt tcttatgatt tatgattttt attattaaat 5760
   aagttataaa aaaaataagt gtatacaaat tttaaagtga ctcttaggtt ttaaaacgaa 5820
   aattcttgtt cttgagtaac tctttcctgt aggtcaggtt gctttctcag gtatagcatg 5880
   aggtcgctct tattgaccac acctctaccg gcatgccgag caggggatcc tctagagtcg 5940
   acctgcataa taaaactagt aataaagaag gcgcgccgtg tgttggtttt ttgtgtgatg 6000
   caggcatgca agcttggcac tggccgtcgt tttacaacgt cgtgactggg aaaaccctgg 6060
   cgttacccaa cttaatcgcc ttgcagcaca tccccctttc gccagctggc gtaatagcga 6120
   agaggcccgc accgatcgcc cttcccaaca gttgcgcagc ctgaatggcg aatggcggga 6180
   gcgctaattt ttcaaacaaa gaatctgagc tgcattttta cagaacagaa atgcaacgcg 6240
   agagcgctat tttaccaaca aagaatctat acttcttttt tgttctacaa aaatgcatcc 6300
   cgagagcgct atttttctaa caaagcatct tagattactt tttttctcct ttgtgcgctc 6360
   tataatgcag tctcttgata actttttgca ctgtaggtcc gttaaggtta gaagaaggct 6420
   actttggtgt ctattttctc ttccataaaa aaagcctgac tccacttccc gcgtttactg 6480
   attactagcg aagctgcggg tgcatttttt caagataaag gcatccccga ttatattcta 6540
   taccgatgtg gattgcgcat actttgtgaa cagaaagtga tagcgttgat gattcttcat 6600
   tggtcagaaa attatgaacg gtttcttcta ttttgtctct atatactacg tataggaaat 6660
   gtttacattt tcgtattgtt ttcgattcac tctatgaata gttcttacta caattttttt 6720
   gtctaaagag taatactaga gataaacata aaaaatgtag aggtcgagtt tagatgcaag 6780
   ttcaaggagc gaaaggtgga tgggtaggtt atatagggat atagcacaga gatatatagc 6840
   aaagagatac ttttgagcaa tgtttgtgga agcggtattc gcaatattgg cgcctgatgc 6900
   ggtattttct ccttacgcat ctgtgcggta tttcacaccg catatatggt gcactctcag   6960
   tacaatctgc tctgatgccg catagttaag ccagccccga cacccgccaa cacccgctga   7020
   cgcgccctga cgggcttgtc tgctcccggc atccgcttac agacaagctg tgaccgtctc   7080
   cgggagctgc atgtgtcaga ggttttcacc gtcatcaccg aaacgcgcga              7130
<210> 4
   <211> 7109
   <212> DNA
   <213> synthetic
<400> 4
   gacgaaaggg cctcgtgata cgcctatttt tataggttaa tgtcatgata ataatggttt     60
   cttagacgtc aggtggcact tttcggggaa atgtgcgcgg aacccctatt tgtttatttt    120
   tctaaataca ttcaaatatg tatccgctca tgagacaata accctgataa atgcttcaat    180
   aatattgaaa aaggaagagt atgagtattc aacatttccg tgtcgccctt attccctttt    240
   ttgcggcatt ttgccttcct gtttttgctc acccagaaac gctggtgaaa gtaaaagatg    300
   ctgaagatca gttgggtgca cgagtgggtt acatcgaact ggatctcaac agcggtaaga    360
   tccttgagag ttttcgcccc gaagaacgtt ttccaatgat gagcactttt aaagttctgc    420
   tatgtggcgc ggtattatcc cgtattgacg ccgggcaaga gcaactcggt cgccgcatac    480
   actattctca gaatgacttg gttgagtact caccagtcac agaaaagcat cttacggatg    540
   gcatgacagt aagagaatta tgcagtgctg ccataaccat gagtgataac actgcggcca    600
   acttacttct gacaacgatc ggaggaccga aggagctaac cgcttttttg cacaacatgg    660
   gggatcatgt aactcgcctt gatcgttggg aaccggagct gaatgaagcc ataccaaacg    720
   acgagcgtga caccacgatg cctgtagcaa tggcaacaac gttgcgcaaa ctattaactg    780
   gcgaactact tactctagct tcccggcaac aattaataga ctggatggag gcggataaag    840
   ttgcaggacc acttctgcgc tcggcccttc cggctggctg gtttattgct gataaatctg    900
   gagccggtga gcgtgggtct cgcggtatca ttgcagcact ggggccagat ggtaagccct    960
   cccgtatcgt agttatctac acgacgggga gtcaggcaac tatggatgaa cgaaatagac   1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcacc atgggtaagc ctatccctaa ccctctcctc ggtctcgatt 5100
   ctacacaagc tatgggtgct cctccaaaaa agaagagaaa ggtagctgcc ggtacccagc 5160
   tttcttgtac aaagcaattg gcgcaacgca atgcatgtga gttagctcac tcattaggca 5220
   ccccaggctt tacactttat gcttccggct cgtatgttgt gtggaattgt gagcggataa 5280
   caatttcaca gaggaaacag ctgatggaat tctacgtacg gcagtttaag gtttacacct 5340
   ataaaagaga gagccgttat cgtctgtttg tggatgtaca gagtgatatt attgacacgc 5400
   cggggcgacg gatggtgatc cccctggcca gtgcacgtct gctgtcagat aaagtctccc 5460
   gtgaacttta cccggtggtg catatcgggg atgaaagctg gcgcatgatg accaccgata 5520
   tggccagtgt gccggtctcc gttatcgggg aagaagtggc tgatctcagc caccgcgaaa 5580
   atgacatcaa aaacgccatt aacctgatgt tctggggaat ataacccggt acccagcttt 5640
   cttgtacaaa gcccgggcgt accgcggccg ctcgagtcga cctgcagcca agctaattcc 5700
   gggcgaattt cttatgattt atgattttta ttattaaata agttataaaa aaaataagtg 5760
   tatacaaatt ttaaagtgac tcttaggttt taaaacgaaa attcttgttc ttgagtaact 5820
   ctttcctgta ggtcaggttg ctttctcagg tatagcatga ggtcgctctt attgaccaca 5880
   cctctaccgg catgccgagc aggggatcct ctagagtcga cctgcataat aaaactagta 5940
   ataaagaagg cgcgccgtgt gttggttttt tgtgtgatgc aggcatgcaa gcttggcact 6000
   ggccgtcgtt ttacaacgtc gtgactggga aaaccctggc gttacccaac ttaatcgcct 6060
   tgcagcacat ccccctttcg ccagctggcg taatagcgaa gaggcccgca ccgatcgccc 6120
   ttcccaacag ttgcgcagcc tgaatggcga atggcgggag cgctaatttt tcaaacaaag 6180
   aatctgagct gcatttttac agaacagaaa tgcaacgcga gagcgctatt ttaccaacaa 6240
   agaatctata cttctttttt gttctacaaa aatgcatccc gagagcgcta tttttctaac 6300
   aaagcatctt agattacttt ttttctcctt tgtgcgctct ataatgcagt ctcttgataa 6360
   ctttttgcac tgtaggtccg ttaaggttag aagaaggcta ctttggtgtc tattttctct 6420
   tccataaaaa aagcctgact ccacttcccg cgtttactga ttactagcga agctgcgggt 6480
   gcattttttc aagataaagg catccccgat tatattctat accgatgtgg attgcgcata 6540
   ctttgtgaac agaaagtgat agcgttgatg attcttcatt ggtcagaaaa ttatgaacgg 6600
   tttcttctat tttgtctcta tatactacgt ataggaaatg tttacatttt cgtattgttt 6660
   tcgattcact ctatgaatag ttcttactac aatttttttg tctaaagagt aatactagag 6720
   ataaacataa aaaatgtaga ggtcgagttt agatgcaagt tcaaggagcg aaaggtggat 6780
   gggtaggtta tatagggata tagcacagag atatatagca aagagatact tttgagcaat 6840
   gtttgtggaa gcggtattcg caatattggc gcctgatgcg gtattttctc cttacgcatc 6900
   tgtgcggtat ttcacaccgc atatatggtg cactctcagt acaatctgct ctgatgccgc 6960
   atagttaagc cagccccgac acccgccaac acccgctgac gcgccctgac gggcttgtct 7020
   gctcccggca tccgcttaca gacaagctgt gaccgtctcc gggagctgca tgtgtcagag 7080
   gttttcaccg tcatcaccga aacgcgcga 7109
<210> 5
   <211> 8902
   <212> DNA
   <213> synthetic
<220>
   <221> misc_feature
   <223> N is any nucleotide residue
<400> 5
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttatggag acagacacac tcctgctatg ggtactgctg ctctgggttc 2760
   caggttccac tggtgactat ccatatgatg ttccagatta tgctggggcc cagccggcca 2820
   tggccgaggt caagctgcag gagtcagggg gaggcttagt gcagcctgga gggtcccgga 2880
   aactctcctg tgcagcctct ggattcactt tcagtagctt tggaatgcac tgggttcgtc 2940
   aggctccaga gaaggggctg gagtgggtcg catatattag tagtggcagt agtaccatct 3000
   actatgcaga cacagtgaag ggacgattca ccatctccag agacaatccc aagaacaccc 3060
   tgttcctgca aatgaccagt ctaaggtctg aggacacggn catgtattac tgtgcaagag 3120
   attacggggc ttattggggc caagggacca cggncaccgt ctcctcaggt ggaggcggct 3180
   caggcggagg tggctctggc ggtggcggat cggacattga gctcacccag tctccagcaa 3240
   tcatgtctgc atctccaggg gagagggtca ccatgacctg cagtgccagt tcaagtgtaa 3300
   ggtacatgaa ctggttccaa cagaagtcag gcacctcccc caaaagatgg atttatgaca 3360
   catccaaact gtcttctgga gtccctgctc gcttcagtgg cagtgggtct gggacctctt 3420
   actctctcac aatcagcagc atggaggctg aagatgctgc cacttactac tgccagcagt 3480
   ggagtagtaa cccactcacg ttcggtgctg ggaccaagct ggagctgaaa cgggcggccg 3540
   cagaacaaaa actcatctca gaagaggatc tgaatggggc cgtcgacgaa caaaaactca 3600
   tctcagaaga ggatctgaat gctgtgggcc aggacacgca ggaggtcatc gtggtgccac 3660
   actccttgcc ctttaaggtg gtggtgatct cagccatcct ggccctggtg gtgctcacca 3720
   tcatctccct tatcatcctc atcatgcttt ggcagaagaa gccacgttag ggccagatct 3780
   gagctcgcgg ccgcgatatt aataaaacta gtaataaaga aggcgcgccg tgtgttggtt 3840
   ttttgtgtga tgcacgctag ctcgagccca cacaccatag cttcaaaatg tttctactcc 3900
   ttttttactc ttccagattt tctcggactc cgcgcatcgc cgtaccactt caaaacaccc 3960
   aagcacagca tactaaattt cccctctttc ttcctctagg gtgtcgttaa ttacccgtac 4020
   taaaggtttg gaaaagaaaa aagagaccgc ctcgtttctt tttcttcgtc gaaaaaggca 4080
   ataaaaattt ttatcacgtt tctttttctt gaaaattttt ttttttagtt tttttctctt 4140
   tcgatgacct cccattgata tttaagttaa taaacggtct tcaatttctc aagtttcagt 4200
   ttcatttttc ttgttctatt acaacttttt ttacttcttg ctcattagaa agaaagcata 4260
   gcaatctaat ctaagggcgg cgttgacaat taatcatcgg catagtatat cggcatagta 4320
   taatacgaca aggtgaggaa ctaaaccctc ccagttcaat tacagctctt aaggctagag 4380
   tacttaatac gactcactat aggctagcct cgagaattca cgcgtcgagc atgcatctag 4440
   ggcggccaat tccgcccctc tccctccccc ccccctaacg ttactggccg aagccgcttg 4500
   gaataaggcc ggtgtgcgtt tgtctatatg tgattttcca ccatattgcc gtcttttggc 4560
   aatgtgaggg cccggaaacc tggccctgtc ttcttgacga gcattcctag gggtctttcc 4620
   cctctcgcca aaggaatgca aggtctgttg aatgtcgtga aggaagcagt tcctctggaa 4680
   gcttcttgaa gacaaacaac gtctgtagcg accctttgca ggcagcggaa ccccccacct 4740
   ggcgacaggt gcctctgcgg ccaaaagcca cgtgtataag atacacctgc aaaggcggca 4800
   caaccccagt gccacgttgt gagttggata gttgtggaaa gagtcaaatg gctctcctca 4860
   agcgtattca acaaggggct gaaggatgcc cagaaggtac cccattgtat gggatctgat 4920
   ctggggcctc ggtgcacatg ctttacatgt gtttagtcga ggttaaaaaa acgtctaggc 4980
   cccccgaacc acggggacgt ggttttcctt tgaaaaacac gatgataaat ggccaagcct 5040
   ttgtctcaag aagaatccac cctcattgaa agagcaacgg ctacaatcaa cagcatcccc 5100
   atctctgaag actacagcgt cgccagcgca gctctctcta gcgacggccg catcttcact 5160
   ggtgtcaatg tatatcattt tactggggga ccttgtgcag aactcgtggt gctgggcact 5220
   gctgctgctg cggcagctgg caacctgact tgtatcgtcg cgatcggaaa tgagaacagg 5280
   ggcatcttga gcccctgcgg acggtgccga caggtgcttc tcgatctgca tcctgggatc 5340
   aaagccatag tgaaggacag tgatggacag ccgacggcag ttgggattcg tgaattgctg 5400
   ccctctggtt atgtgtggga gggctaagca cttcgtggcc gaggagcagg actgacacgt 5460
   ccgacgcggc ccgacgggtc cgaggcctcg gagatccgtc ccccttttcc tttgtcgata 5520
   tcatgtaatt agttatgtca cgcttacatt cacgccctcc ccccacatcc gctctaaccg 5580
   aaaaggaagg agttagacaa cctgaagtct aggtccctat ttattttttt atagttatgt 5640
   tagtattaag aacgttattt atatttcaaa tttttctttt ttttctgtac agacgcgtgt 5700
   acgcatgtaa cattatactg aaaaccttgc ttgagaaggt tttgggacgc tcgaaggctt 5760
   taatttgcaa gctgaattgt tacataactt acggtaaatg gcccgcctgg ctgaccgccc 5820
   aacgaccccc gcccattgac gtcaataatg acgtatgttc ccatagtaac gccaataggg 5880
   actttccatt gacgtcaatg ggtggagtat ttacggtaaa ctgcccactt ggcagtacat 5940
   caagtgtatc atatgccaag tacgccccct attgacgtca atgacggtaa atggcccgcc 6000
   tggcattatg cccagtacat gaccttatgg gactttccta cttggcagta catctacgta 6060
   ttagtcatcg ctattaccat ggtgatgcgg ttttggcagt acatcaatgg gcgtggatag 6120
   cggtttgact cacggggatt tccaagtctc caccccattg acgtcaatgg gagtttgttt 6180
   tggcaccaaa atcaacggga ctttccaaaa tgtcgtaaca actccgcccc attgacgcaa 6240
   atgggcggta ggcgtgtacg gtgggaggtc tatataagca gagctcgttt agtgaaccgt 6300
   cagatcgcct ggagacgcca tccacgctgt tttgacctcc atagaagaca attaattccc 6360
   aagggatttt ggtcatgaga tccaacttct ttcttttttt tttcttttct ctctcccccg 6420
   ttgttgtctc accatatccg caatgacaaa aaaaatgatg gaagacacta aaggaaaaaa 6480
   ttaacgacaa agacagcacc aacagatgtc gttgttccag agctgatgag gggtatcttc 6540
   gaacacacga aactttttcc ttccttcatt cacgcacact actctctaat gagcaacggt 6600
   atacggcctt ccttccagtt acttgaattt gaaataaaaa aagtttgccg ctttgctatc 6660
   aagtataaat agacctgcaa ttattaatct tttgtttcct cgtcattgtt ctcgttccct 6720
   ttcttccttg tttctttttc tgcacaatat ttcaagctat accaagcata caatcaactc 6780
   caagcttccc cggatcggac tactagcagc tgtaatacga ctcactatag ggaatattaa 6840
   gctaagctca ccatgggtaa gcctatccct aaccctctcc tcggtctcga ttctacacaa 6900
   gctatgggtg ctcctccaaa aaagaagaga aaggtagctg aattgacttt gtacaaaaaa 6960
   gcaggctgaa ttggcgcaac gcaatgcatg tgagttagct cactcattag gcaccccagg 7020
   ctttacactt tatgcttccg gctcgtatgt tgtgtggaat tgtgagcgga taacaatttc 7080
   acagaggaaa cagctgatgg aattctacgt acggcagttt aaggtttaca cctataaaag 7140
   agagagccgt tatcgtctgt ttgtggatgt acagagtgat attattgaca cgccggggcg 7200
   acggatggtg atccccctgg ccagtgcacg tctgctgtca gataaagtct cccgtgaact 7260
   ttacccggtg gtgcatatcg gggatgaaag ctggcgcatg atgaccaccg atatggccag 7320
   tgtgccggtc tccgttatcg gggaagaagt ggctgatctc agccaccgcg aaaatgacat 7380
   caaaaacgcc attaacctga tgttctgggg aatataaccc ggtacccagc tttcttgtac 7440
   aaagcccggg cgtaccgcgg ccgctcgagt cgacctgcag ccaagctaat tccgggcgaa 7500
   tttcttatga tttatgattt ttattattaa ataagttata aaaaaaataa gtgtatacaa 7560
   attttaaagt gactcttagg ttttaaaacg aaaattcttg ttcttgagta actctttcct 7620
   gtaggtcagg ttgctttctc aggtatagca tgaggtcgct cttattgacc acacctctac 7680
   cggcatgccg agcaggggat cctctagagt cgacctgcat aataaaacta gtaataaaga 7740
   aggcgcgccg tgtgttggtt ttttgtgtga tgcaggcatg caagcttggc actggccgtc 7800
   gttttacaac gtcgtgactg ggaaaaccct ggcgttaccc aacttaatcg ccttgcagca 7860
   catccccctt tcgccagctg gcgtaatagc gaagaggccc gcaccgatcg cccttcccaa 7920
   cagttgcgca gcctgaatgg cgaatggcgg gagcgctaat ttttcaaaca aagaatctga 7980
   gctgcatttt tacagaacag aaatgcaacg cgagagcgct attttaccaa caaagaatct 8040
   atacttcttt tttgttctac aaaaatgcat cccgagagcg ctatttttct aacaaagcat 8100
   cttagattac tttttttctc ctttgtgcgc tctataatgc agtctcttga taactttttg 8160
   cactgtaggt ccgttaaggt tagaagaagg ctactttggt gtctattttc tcttccataa 8220
   aaaaagcctg actccacttc ccgcgtttac tgattactag cgaagctgcg ggtgcatttt 8280
   ttcaagataa aggcatcccc gattatattc tataccgatg tggattgcgc atactttgtg 8340
   aacagaaagt gatagcgttg atgattcttc attggtcaga aaattatgaa cggtttcttc 8400
   tattttgtct ctatatacta cgtataggaa atgtttacat tttcgtattg ttttcgattc 8460
   actctatgaa tagttcttac tacaattttt ttgtctaaag agtaatacta gagataaaca 8520
   taaaaaatgt agaggtcgag tttagatgca agttcaagga gcgaaaggtg gatgggtagg 8580
   ttatataggg atatagcaca gagatatata gcaaagagat acttttgagc aatgtttgtg 8640
   gaagcggtat tcgcaatatt ggcgcctgat gcggtatttt ctccttacgc atctgtgcgg 8700
   tatttcacac cgcatatatg gtgcactctc agtacaatct gctctgatgc cgcatagtta 8760
   agccagcccc gacacccgcc aacacccgct gacgcgccct gacgggcttg tctgctcccg 8820
   gcatccgctt acagacaagc tgtgaccgtc tccgggagct gcatgtgtca gaggttttca 8880
   ccgtcatcac cgaaacgcgc ga 8902
<210> 6
   <211> 7193
   <212> DNA
   <213> synthetic
<400> 6
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagg ataatgtatg ctatacgaag ttatgctaag ctcaccatgg gtaagcctat 5100
   ccctaaccct ctcctcggtc tcgattctac acaagctatg ggtgctcctc caaaaaagaa 5160
   gagaaaggta gctgaattgg cgcaacgcaa tgcatgtgag ttagctcact cattaggcac 5220
   cccaggcttt acactttatg cttccggctc gtatgttgtg tggaattgtg agcggataac 5280
   aatttcacag aggaaacagc tgatggaatt ctacgtacgg cagtttaagg tttacaccta 5340
   taaaagagag agccgttatc gtctgtttgt ggatgtacag agtgatatta ttgacacgcc 5400
   ggggcgacgg atggtgatcc ccctggccag tgcacgtctg ctgtcagata aagtctcccg 5460
   tgaactttac ccggtggtgc atatcgggga tgaaagctgg cgcatgatga ccaccgatat 5520
   ggccagtgtg ccggtctccg ttatcgggga agaagtggct gatctcagcc accgcgaaaa 5580
   tgacatcaaa aacgccatta acctgatgtt ctggggaata taacgataat gtatgctata 5640
   cgaagttatg ccgggcgtac cgcggccgct cgagtcgacc tgcagccaag ctaattccgg 5700
   gcgaatttct tatgatttat gatttttatt attaaataag ttataaaaaa aataagtgta 5760
   tacaaatttt aaagtgactc ttaggtttta aaacgaaaat tcttgttctt gagtaactct 5820
   ttcctgtagg tcaggttgct ttctcaggta tagcatgagg tcgctcttat tgaccacacc 5880
   tctaccggca tgccgagcag gggatcctct agagtcgacc tgcataataa aactagtaat 5940
   aaagaaggcg cgccgtgtgt tggttttttg tgtgatgcag gcatgcaagc ttggcactgg 6000
   ccgtcgtttt acaacgtcgt gactgggaaa accctggcgt tacccaactt aatcgccttg 6060
   cagcacatcc ccctttcgcc agctggcgta atagcgaaga ggcccgcacc gatcgccctt 6120
   cccaacagtt gcgcagcctg aatggcgaat cgttcaattg ccttaattaa cagtgtgctg 6180
   caattcgccc ttgagagccg ggagcgctaa tttttcaaac aaagaatctg agctgcattt 6240
   ttacagaaca gaaatgcaac gcgagagcgc tattttacca acaaagaatc tatacttctt 6300
   ttttgttcta caaaaatgca tcccgagagc gctatttttc taacaaagca tcttagatta 6360
   ctttttttct cctttgtgcg ctctataatg cagtctcttg ataacttttt gcactgtagg 6420
   tccgttaagg ttagaagaag gctactttgg tgtctatttt ctcttccata aaaaaagcct 6480
   gactccactt cccgcgttta ctgattacta gcgaagctgc gggtgcattt tttcaagata 6540
   aaggcatccc cgattatatt ctataccgat gtggattgcg catactttgt gaacagaaag 6600
   tgatagcgtt gatgattctt cattggtcag aaaattatga acggtttctt ctattttgtc 6660
   tctatatact acgtatagga aatgtttaca ttttcgtatt gttttcgatt cactctatga 6720
   atagttctta ctacaatttt tttgtctaaa gagtaatact agagataaac ataaaaaatg 6780
   tagaggtcga gtttagatgc aagttcaagg agcgaaaggt ggatgggtag gttatatagg 6840
   gatatagcac agagatatat agcaaagaga tacttttgag caatgtttgt ggaagcggta 6900
   ttcgcaatat tcgcgcctct caagggcgaa ttgtgcagat ttcttaatta aaacgcctga 6960
   tgcggtattt tctccttacg catctgtgcg gtatttcaca ccgcatatat ggtgcactct 7020
   cagtacaatc tgctctgatg ccgcatagtt aagccagccc cgacacccgc caacacccgc 7080
   tgacgcgccc tgacgggctt gtctgctccc ggcatccgct tacagacaag ctgtgaccgt 7140
   ctccgggagc tgcatgtgtc agaggttttc accgtcatca ccgaaacgcg cga 7193
<210> 7
   <211> 7473
   <212> DNA
   <213> synthetic
<400> 7
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcacc atgggtaagc ctatccctaa ccctctcctc ggtctcgatt 5100
   ctacacaagc tatgggtgct cctccaaaaa agaagagaaa ggtagctgaa ttggcgcaac 5160
   gcaatttatg tgagttagct cactcattag gcaccccagg ctttacactt tatgcttccg 5220
   gctcgtatgt tgtgtggaat tgtgagcgga taacaatttc acacaggaaa cagctatgac 5280
   catgattacg ccaagctatt taggtgacac tatagaatac tcaagctatg catcaagctt 5340
   ggtaccgagc tcggatccac tagtaacggc cgccagtgtg ctggaattct gcagatatcc 5400
   atcacactgg cggccgctcg agcatgcatc tagagggccc aattcgccct atagtgagtc 5460
   gtattacaat tcactggccg tcgttttaca acgtcgtgac tgggaaaacc ctggcgttac 5520
   ccaacttaat cgccttgcag cacatccccc tttcgccagc tggcgtaata gcgaagaggc 5580
   ccgcaccgat cgcccttccc aacagttgcg cagcctatac gtacggcagt ttaaggttta 5640
   cacctataaa agagagagcc gttatcgtct gtttgtggat gtacagagtg atattattga 5700
   cacgccgggg cgacggatgg tgatccccct ggccagtgca cgtctgctgt cagataaagt 5760
   ctcccgtgaa ctttacccgg tggtgcatat cggggatgaa agctggcgca tgatgaccac 5820
   cgatatggcc agtgtgccgg tctccgttat cggggaagaa gtggctgatc tcagccaccg 5880
   cgaaaatgac atcaaaaacg ccattaacct gatgttctgg ggaatataac ccgggcgtac 5940
   cgcggccgct cgagtcgacc tgcagccaag ctaattccgg gcgaatttct tatgatttat 6000
   gatttttatt attaaataag ttataaaaaa aataagtgta tacaaatttt aaagtgactc 6060
   ttaggtttta aaacgaaaat tcttgttctt gagtaactct ttcctgtagg tcaggttgct 6120
   ttctcaggta tagcatgagg tcgctcttat tgaccacacc tctaccggca tgccgagcag 6180
   gggatcctct agagtcgacc tgcataataa aactagtaat aaagaaggcg cgccgtgtgt 6240
   tggtttttgg tgtgatgcag gcatgcaagc ttggcactgg ccgtcgtttt acaacgtcgt 6300
   gactgggaaa accctggcgt tacccaactt aatcgccttg cagcacatcc ccctttcgcc 6360
   agctggcgta atagcgaaga ggcccgcacc gatcgccctt cccaacagtt gcgcagcctg 6420
   aatggcgaat cgttcaattg ccttaattaa cagtgtgctg caattcgccc ttgagagccg 6480
   ggagcgctaa tttttcaaac aaagaatctg agctgcattt ttacagaaca gaaatgcaac 6540
   gcgagagcgc tattttacca acaaagaatc tatacttctt ttttgttcta caaaaatgca 6600
   tcccgagagc gctatttttc taacaaagca tcttagatta ctttttttct cctttgtgcg 6660
   ctctataatg cagtctcttg ataacttttt gcactgtagg tccgttaagg ttagaagaag 6720
   gctactttgg tgtctatttt ctcttccata aaaaaagcct gactccactt cccgcgttta 6780
   ctgattacta gcgaagctgc gggtgcattt tttcaagata aaggcatccc cgattatatt 6840
   ctataccgat gtggattgcg catactttgt gaacagaaag tgatagcgtt gatgattctt 6900
   cattggtcag aaaattatga acggtttctt ctattttgtc tctatatact acgtatagga 6960
   aatgtttaca ttttcgtatt gttttcgatt cactctatga atagttctta ctacaatttt 7020
   tttgtctaaa gagtaatact agagataaac ataaaaaatg tagaggtcga gtttagatgc 7080
   aagttcaagg agcgaaaggt ggatgggtag gttatatagg gatatagcac agagatatat 7140
   agcaaagaga tacttttgag caatgtttgt ggaagcggta ttcgcaatat tcgcgcctct 7200
   caagggcgaa ttgtgcagat ttcttaatta aaacgcctga tgcggtattt tctccttacg 7260
   catctgtgcg gtatttcaca ccgcatatat ggtgcactct cagtacaatc tgctctgatg 7320
   ccgcatagtt aagccagccc cgacacccgc caacacccgc tgacgcgccc tgacgggctt 7380
   gtctgctccc ggcatccgct tacagacaag ctgtgaccgt ctccgggagc tgcatgtgtc 7440
   agaggttttc accgtcatca ccgaaacgcg cga 7473
<210> 8
   <211> 6864
   <212> DNA
   <213> synthetic
<400> 8
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   ccaagccttt gtctcaagaa gaatccaccc tcattgaaag agcaacggct acaatcaaca 3300
   gcatccccat ctctgaagac tacagcgtcg ccagcgcagc tctctctagc gacggccgca 3360
   tcttcactgg tgtcaatgta tatcatttta ctgggggacc ttgtgcagaa ctcgtggtgc 3420
   tgggcactgc tgctgctgcg gcagctggca acctgacttg tatcgtcgcg atcggaaatg 3480
   agaacagggg catcttgagc ccctgcggac ggtgccgaca ggtgcttctc gatctgcatc 3540
   ctgggatcaa agccatagtg aaggacagtg atggacagcc gacggcagtt gggattcgtg 3600
   aattgctgcc ctctggttat gtgtgggagg gctaagcact tcgtggccga ggagcaggac 3660
   tgacacgtcc gacgcggccc gacgggtccg aggcctcgga gatccgtccc ccttttcctt 3720
   tgtcgatatc atgtaattag ttatgtcacg cttacattca cgccctcccc ccacatccgc 3780
   tctaaccgaa aaggaaggag ttagacaacc tgaagtctag gtccctattt atttttttat 3840
   agttatgtta gtattaagaa cgttatttat atttcaaatt tttctttttt ttctgtacag 3900
   acgcgtgtac gcatgtaaca ttatactgaa aaccttgctt gagaaggttt tgggacgctc 3960
   gaaggcttta atttgcaagc tgaattgtta cataacttac ggtaaatggc ccgcctggct 4020
   gaccgcccaa cgacccccgc ccattgacgt caataatgac gtatgttccc atagtaacgc 4080
   caatagggac tttccattga cgtcaatggg tggagtattt acggtaaact gcccacttgg 4140
   cagtacatca agtgtatcat atgccaagta cgccccctat tgacgtcaat gacggtaaat 4200
   ggcccgcctg gcattatgcc cagtacatga ccttatggga ctttcctact tggcagtaca 4260
   tctacgtatt agtcatcgct attaccatgg tgatgcggtt ttggcagtac atcaatgggc 4320
   gtggatagcg gtttgactca cggggatttc caagtctcca ccccattgac gtcaatggga 4380
   gtttgttttg gcaccaaaat caacgggact ttccaaaatg tcgtaacaac tccgccccat 4440
   tgacgcaaat gggcggtagg cgtgtacggt gggaggtcta tataagcaga gctcgtttag 4500
   tgaaccgtca gatcgcctgg agacgccatc cacgctgttt tgacctccat agaagacaat 4560
   taattcccaa gggattttgg tcatgagatc caacttcttt tctttttttt tcttttctct 4620
   ctcccccgtt gttgtctcac catatccgca atgacaaaaa aaatgatgga agacactaaa 4680
   ggaaaaaatt aacgacaaag acagcaccaa cagatgtcgt tgttccagag ctgatgaggg 4740
   gtatcttcga acacacgaaa ctttttcctt ccttcattca cgcacactac tctctaatga 4800
   gcaacggtat acggccttcc ttccagttac ttgaatttga aataaaaaaa gtttgccgct 4860
   ttgctatcaa gtataaatag acctgcaatt attaatcttt tgtttcctcg tcattgttct 4920
   cgttcccttt cttccttgtt tctttttctg cacaatattt caagctatac caagcataca 4980
   atcaactcca agcttccccg gatcggacta ctagcagctg taatacgact cactataggg 5040
   aatattaagc taagctcagg atggcagaac aaaaactcat ctcagaagag gatctgcctc 5100
   caaaaaagaa gagaaaggta gctgatggca cctccaaaaa agaagagaaa ggtagctcat 5160
   gggcaagcct atcccaaacc ctctcctcgg tctcgattct acacaagcta tgggtgctcc 5220
   tccaaaaaag aagagaaagg cagctgaatt cgagctcaga tctcagctgg gcccggtacc 5280
   aattgatgca tcgataccgg tactagtcgg accgcatatg cccgggcgta ccgcggccgc 5340
   tcgagtcgac ctgcagccaa gctaattccg ggcgaatttc ttatgattta tgatttttat 5400
   tattaaataa gttataaaaa aaataagtgt atacaaattt taaagtgact cttaggtttt 5460
   aaaacgaaaa ttcttgttct tgagtaactc tttcctgtag gtcaggttgc tttctcaggt 5520
   atagcatgag gtcgctctta ttgaccacac ctctaccggc atgccgagca ggggatcctc 5580
   tagagtcgac ctgcataata aaactagtaa taaagaaggc gcgccgtgtg ttggtttttt 5640
   gtgtgatgca ggcatgcaag cttggcactg gccgtcgttt tacaacgtcg tgactgggaa 5700
   aaccctggcg ttacccaact taatcgcctt gcagcacatc cccctttcgc cagctggcgt 5760
   aatagcgaag aggcccgcac cgatcgccct tcccaacagt tgcgcagcct gaatggcgaa 5820
   tcgttcaatt gccttaatta acagtgtgct gcaattcgcc cttgagagcc gggagcgcta 5880
   atttttcaaa caaagaatct gagctgcatt tttacagaac agaaatgcaa cgcgagagcg 5940
   ctattttacc aacaaagaat ctatacttct tttttgttct acaaaaatgc atcccgagag 6000
   cgctattttt ctaacaaagc atcttagatt actttttttc tcctttgtgc gctctataat 6060
   gcagtctctt gataactttt tgcactgtag gtccgttaag gttagaagaa ggctactttg 6120
   gtgtctattt tctcttccat aaaaaaagcc tgactccact tcccgcgttt actgattact 6180
   agcgaagctg cgggtgcatt ttttcaagat aaaggcatcc ccgattatat tctataccga 6240
   tgtggattgc gcatactttg tgaacagaaa gtgatagcgt tgatgattct tcattggtca 6300
   gaaaattatg aacggtttct tctattttgt ctctatatac tacgtatagg aaatgtttac 6360
   attttcgtat tgttttcgat tcactctatg aatagttctt actacaattt ttttgtctaa 6420
   agagtaatac tagagataaa cataaaaaat gtagaggtcg agtttagatg caagttcaag 6480
   gagcgaaagg tggatgggta ggttatatag ggatatagca cagagatata tagcaaagag 6540
   atacttttga gcaatgtttg tggaagcggt attcgcaata ttcgcgcctc tcaagggcga 6600
   attgtgcaga tttcttaatt aaaacgcctg atgcggtatt ttctccttac gcatctgtgc 6660
   ggtatttcac accgcatata tggtgcactc tcagtacaat ctgctctgat gccgcatagt 6720
   taagccagcc ccgacacccg ccaacacccg ctgacgcgcc ctgacgggct tgtctgctcc 6780
   cggcatccgc ttacagacaa gctgtgaccg tctccgggag ctgcatgtgt cagaggtttt 6840
   caccgtcatc accgaaacgc gcga 6864
<210> 9
   <211> 7198
   <212> DNA
   <213> synthetic
<400> 9
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgtcc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ctgggcccag ccaaaaaggc caaaaaggcc tggtgaccgg 1920
   atccgctgtg gaatgtgtgt cagttagggt gtggaaagtc cccaggctcc ccagcaggca 1980
   gaagtatgca aagcatgcat ctcaattagt cagcaaccag gtgtggaaag tccccaggct 2040
   ccccagcagg cagaagtatg caaagcatgc atctcaatta gtcagcaacc atagtcccgc 2100
   ccctaactcc gcccatcccg cccctaactc cgcccagttc cgcccattct ccgccccatg 2160
   gctgactaat tttttttatt tatgcagagg ccgaggccgc ctcggcctct gagctattcc 2220
   agaagtagtg aggaggcttt tttggaggcc taggcttttg caaaaagctg gctcgatctc 2280
   tccttcacgc gcccgccgcc ctacctgagg ccgccatcca cgccggtgag tcgcgttctg 2340
   ccgcctcccg cctgtggtgc ctcctgaact gcgtccgccg tctaggtaag tttagagctc 2400
   aggtcgagac cgggcctttg tccggcgctc ccttggagcc tacctagact cagccggctc 2460
   tccacgcttt gcctgaccct gcttgctcaa ctctacgtct ttgtttcgtt ttctgttctg 2520
   cgccgttaca acttcaaggt atgcgctggg acctggcagg cggcatctgg gacccctagg 2580
   aagggcttgg gggtcctcgt gcccaaggca gggaacatag tggtcccagg aaggggagca 2640
   gaggcatcag ggtgtccact ttgtctccgc agctcctgag cctgcagcac gtgttgggcc 2700
   tttttggcct ttttggccag atctgagctc gcggccgcga tatcgctagc tcgagcccac 2760
   acaccatagc ttcaaaatgt ttctactcct tttttactct tccagatttt ctcggactcc 2820
   gcgcatcgcc gtaccacttc aaaacaccca agcacagcat actaaatttc ccctctttct 2880
   tcctctaggg tgtcgttaat tacccgtact aaaggtttgg aaaagaaaaa agagaccgcc 2940
   tcgtttcttt ttcttcgtcg aaaaaggcaa taaaaatttt tatcacgttt ctttttcttg 3000
   aaaatttttt tttttagttt ttttctcttt cgatgacctc ccattgatat ttaagttaat 3060
   aaacggtctt caatttctca agtttcagtt tcatttttct tgttctatta caactttttt 3120
   tacttcttgc tcattagaaa gaaagcatag caatctaatc taagggcggc gttgacaatt 3180
   aatcatcggc atagtatatc ggcatagtat aatacgacaa ggtgaggaac taaaccatgg 3240
   atggtgaaga tgttcaagct ggatcgtttc gcatgattga acaagatgga ttgcacgcag 3300
   gttctccggc cgcttgggtg gagaggctat tcggctatga ctgggcacaa cagacaatcg 3360
   gctgctctga tgccgccgtg ttccggctgt cagcgcaggg gcgcccggtt ctttttgtca 3420
   agaccgacct gtccggtgcc ctgaatgaac tgcaggacga ggcagcgcgg ctatcgtggc 3480
   tggccacgac gggcgttcct tgcgcagctg tgctcgacgt tgtcactgaa gcgggaaggg 3540
   actggctgct attgggcgaa gtgccggggc aggatctcct gtcatctcac cttgctcctg 3600
   ccgagaaagt atccatcatg gctgatgcaa tgcggcggct gcatacgctt gatccggcta 3660
   cctgcccatt cgaccaccaa gcgaaacatc gcatcgagcg agcacgtact cggatggaag 3720
   ccggtcttgt cgatcaggat gatctggacg aagagcatca ggggctcgcg ccagccgaac 3780
   tgttcgccag gctcaaggcg cgcatgcccg acggcgagga tctcgtcgtg actcatggcg 3840
   atgcctgctt gccgaatatc atggtggaaa atggccgctt ttctggattc atcgactgtg 3900
   gccggctggg tgtggcggac cgctatcagg acatagcgtt ggctacccgt gatattgctg 3960
   aagagcttgg cggcgaatgg gctgaccgct tcctcgtgct ttacggtatc gccgctcccg 4020
   attcgcagcg catcgccttc tatcgccttc ttgacgagtt cttctgagca cttcgtggcc 4080
   gaggagcagg actgacacgt ccgacgcggc ccgacgggtc cgaggcctcg gagatccgtc 4140
   ccccttttcc tttgtcgata tcatgtaatt agttatgtca cgcttacatt cacgccctcc 4200
   ccccacatcc gctctaaccg aaaaggaagg agttagacaa cctgaagtct aggtccctat 4260
   ttattttttt atagttatgt tagtattaag aacgttattt atatttcaaa tttttctttt 4320
   ttttctgtac agacgcgtgt acgcatgtaa cattatactg aaaaccttgc ttgagaaggt 4380
   tttgggacgc tcgaaggctt taatttgcaa gctgaattgt tacataactt acggtaaatg 4440
   gcccgcctgg ctgaccgccc aacgaccccc gcccattgac gtcaataatg acgtatgttc 4500
   ccatagtaac gccaataggg actttccatt gacgtcaatg ggtggagtat ttacggtaaa 4560
   ctgcccactt ggcagtacat caagtgtatc atatgccaag tacgccccct attgacgtca 4620
   atgacggtaa atggcccgcc tggcattatg cccagtacat gaccttatgg gactttccta 4680
   cttggcagta catctacgta ttagtcatcg ctattaccat ggtgatgcgg ttttggcagt 4740
   acatcaatgg gcgtggatag cggtttgact cacggggatt tccaagtctc caccccattg 4800
   acgtcaatgg gagtttgttt tggcaccaaa atcaacggga ctttccaaaa tgtcgtaaca 4860
   actccgcccc attgacgcaa atgggcggta ggcgtgtacg gtgggaggtc tatataagca 4920
   gagctcgttt agtgaaccgt cagatcgcct ggagacgcca tccacgctgt tttgacctcc 4980
   atagaagaca attaattccc aagggatttt ggtcatgaga tccaacttct ttcttttttt 5040
   tttcttttct ctctcccccg ttgttgtctc accatatccg caatgacaaa aaaaatgatg 5100
   gaagacacta aaggaaaaaa ttaacgacaa agacagcacc aacagatgtc gttgttccag 5160
   agctgatgag gggtatcttc gaacacacga aactttttcc ttccttcatt cacgcacact 5220
   actctctaat gagcaacggt atacggcctt ccttccagtt acttgaattt gaaataaaaa 5280
   aagtttgccg ctttgctatc aagtataaat agacctgcaa ttattaatct tttgtttcct 5340
   cgtcattgtt ctcgttccct ttcttccttg tttctttttc tgcacaatat ttcaagctat 5400
   accaagcata caatcaactc caagcttccc cggatcggac tactagcagc tgtaatacga 5460
   ctcactatag ggaatattaa gctaagctca ccatgggtaa gcctatccct aaccctctcc 5520
   tcggtctcga ttctacacaa gctatgggtg ctcctccaaa aaagaagaga aaggtagctg 5580
   aattcgagct cagatctcag ctgggcccgg taccaattga tgcatcgata ccggtactag 5640
   tcggaccgca tatgcccggg cgtaccgcgg ccgctcgagt cgacctgcag ccaagctaat 5700
   tccgggcgaa tttcttatga tttatgattt ttattattaa ataagttata aaaaaaataa 5760
   gtgtatacaa attttaaagt gactcttagg ttttaaaacg aaaattcttg ttcttgagta 5820
   actctttcct gtaggtcagg ttgctttctc aggtatagca tgaggtcgct cttattgacc 5880
   acacctctac cggcatgccg agcaggggat cctctagagt cgacctgcat aataaaacta 5940
   gtaataaaga aggcgcgccg tgtgttggtt ttttgtgtga tgcaggcatg caagcttggc 6000
   actggccgtc gttttacaac gtcgtgactg ggaaaaccct ggcgttaccc aacttaatcg 6060
   ccttgcagca catccccctt tcgccagctg gcgtaatagc gaagaggccc gcaccgatcg 6120
   cccttcccaa cagttgcgca gcctgaatgg cgaatcgttc aattgcctta attaacagtg 6180
   tgctgcaatt cgcccttgag agccgggagc gctaattttt caaacaaaga atctgagctg 6240
   catttttaca gaacagaaat gcaacgcgag agcgctattt taccaacaaa gaatctatac 6300
   ttcttttttg ttctacaaaa atgcatcccg agagcgctat ttttctaaca aagcatctta 6360
   gattactttt tttctccttt gtgcgctcta taatgcagtc tcttgataac tttttgcact 6420
   gtaggtccgt taaggttaga agaaggctac tttggtgtct attttctctt ccataaaaaa 6480
   agcctgactc cacttcccgc gtttactgat tactagcgaa gctgcgggtg cattttttca 6540
   agataaaggc atccccgatt atattctata ccgatgtgga ttgcgcatac tttgtgaaca 6600
   gaaagtgata gcgttgatga ttcttcattg gtcagaaaat tatgaacggt ttcttctatt 6660
   ttgtctctat atactacgta taggaaatgt ttacattttc gtattgtttt cgattcactc 6720
   tatgaatagt tcttactaca atttttttgt ctaaagagta atactagaga taaacataaa 6780
   aaatgtagag gtcgagttta gatgcaagtt caaggagcga aaggtggatg ggtaggttat 6840
   atagggatat agcacagaga tatatagcaa agagatactt ttgagcaatg tttgtggaag 6900
   cggtattcgc aatattcgcg cctctcaagg gcgaattgtg cagatttctt aattaaaacg 6960
   cctgatgcgg tattttctcc ttacgcatct gtgcggtatt tcacaccgca tatatggtgc 7020
   actctcagta caatctgctc tgatgccgca tagttaagcc agccccgaca cccgccaaca 7080
   cccgctgacg cgccctgacg ggcttgtctg ctcccggcat ccgcttacag acaagctgtg 7140
   accgtctccg ggagctgcat gtgtcagagg ttttcaccgt catcaccgaa acgcgcga 7198
<210> 10
   <211> 9167
   <212> DNA
   <213> synthetic
<400> 10
   taccttttga tgcggaattg actttttcgt gaataataca taacttttct gaaaagaatc 60
   aaagacagat aaaatttaag agatattaaa cattagtgag aagccgagaa ttttgtaaca 120
   ccaacataac actgacatct ttaacaactt ttaattatga taaatttctt acgtcatgat 180
   tgattattac agctatgctg acaaatgact cttgttgcag ggctacgaac cgggtaatat 240
   taagtgattg actcttgctg accttttatt aagaactaaa tggacaatat tatggagcat 300
   ttcatgtata aattggtgcg taaaatcgtt ggatctctct tctaagtaca tcctactata 360
   acaatcaaga aaaacaagaa aaccggacaa aacaatcaag tatggattct agaacagttg 420
   gtatattggg agggggacaa ttgggacgta tgattgttga ggcagctaac aggctcaaca 480
   ttaagacggt aatactagat gctgaaaatt ctcctgccaa acaaataagc aactccaatg 540
   accacgttaa tggctccttt tccaatcctc ttgatatcga aaaactagct gaaaaatgtg 600
   atgtgctaac gattgagatt gagcatgttg atgttcctac actaaagaat cttcaagtaa     660
   aacatcccaa attaaaaatt tacccttctc cagaaacaat cggattgata caagacaaat     720
   atattcaaaa agagcattta atcaaaaatg gtatagcagt tacccaaagt gtccctgtgg 780
   aacaagccag tgagacgtcc ctattgaatg ttggaagaga tttgggtttt ccattcgtct 840
   tgaagtcgag gactttggca tacgatggaa gaggtaactt cgttgtaaag aataaggaaa 900
   tgattccgga agctttggaa gtactgaagg atcgtccttt gtacgccgaa aaatggggac 960
   catttactaa agaattagca gtcatgattg tgagatctgt taacggttta gtgttttttt 1020
   acccaattgt agagactatc cacaaggaca atatttgtga cttatgttat gcgcctgcta 1080
   gagttccgga ctccgttcaa cttaaggcga agttgttggc gaaaatgcaa tcaaactttt 1140
   cccggttgtg gtatattggt gtggaaatgt tctatttaga aacaggggaa ttgcttatta 1200
   acgaaattgc cccaaggcct cacaactctg gacattatac cattgatgct tgcgtcactt 1260
   ctcaatttga agctcatttg agatcaatat tggatttgcc aatgccaaag aatttcacat 1320
   ctttctccac cattacaacg aacgccatta tgctaaatgt tcttggagac aaacatacaa 1380
   aagataaaga gctagaaact tgcgaaagag cattggcgac tccaggttcc tcagtgtact 1440
   tatatggaaa agagtctaga cctaacagaa aagtaggtca cataaatatt attgcctcca 1500
   gtatggcgga atgtgaacaa aggctgaact acattacagg gagaactgat attccactca 1560
   aaatctctgt cgctcaaaag ttggacttgg aagcaatggt caaaccattg gttggagtca 1620
   tcatgggatc agactctgac ttgccggtaa tgtctgccgc atgtgcggtt ttaaaagatt 1680
   ttggcgttac atttgaattg acaatagtct ctgctcatag aactccacat aggatgtcag 1740
   catatgctat ttccgcaagc aagcgtggaa ttaaaacaat tatcgctgga gctggtgggg 1800
   ctgctcactt gccaggtatg gtggctgcaa tgacaccact tcctgtcatc ggtgtgcccg 1860
   taaaaggttc ttgtctagat ggagtagatt ctttacattc aaccgtgcaa atgcctagag 1920
   gtgttccagt agctaccgtc gctattaata atagtacgaa cgctgcgctg ttggctgtca 1980
   gactgcttgg cgcttatgat tcaagttata caacaaaaat ggaacagttt ttattaaagc 2040
   aggaagaaga agttcttgtc aaagcacaaa agttagaaac tgtcggttac gaagcttatc 2100
   tagaaaacaa gtaatatata agtttattga tatacttgca cagcaaataa tataaaatga 2160
   tatacctatt ttttaggctt tgttatgatt acatcaaatg tggacttcat acatagaaat 2220
   caacgcttac aggtgtcctt atcgatgcta gcttgcatgc ctgcagcaat tcccgaggct 2280
   gtagccgacg atggtgcgcc aggagagttg ttgatcggta ctagtcggac cgcatatgcc 2340
   cgggcgtacc gcggccgctc gagtcgacct gcagccaagc taattccggg cgaatttctt 2400
   atgatttatg atttttatta ttaaataagt tataaaaaaa ataagtgtat acaaatttta 2460
   aagtgactct taggttttaa aacgaaaatt cttgttcttg agtaactctt tcctgtaggt 2520
   caggttgctt tctcaggtat agcatgaggt cgctcttatt gaccacacct ctaccggcat 2580
   gccgagcatt atttgtagag ctcatccatg ccatgtgtaa tcccagcagc agttacaaac 2640
   tcaagaagga ccatgtggtc acgcttttcg ttgggatctt tcgaaagggc agattgtgtc 2700
   gacaggtaat ggttgtctgg taaaaggaca gggccatcgc caattggagt attttgttga 2760
   taatggtctg ctagttgaac ggatccatct tcaatgttgt ggcgaatttt gaagttagct 2820
   ttgattccat tcttttgttt gtctgccgtg atgtatacat tgtgtgagtt atagttgtac 2880
   tcgagtttgt gtccgagaat gtttccatct tctttaaaat caataccttt taactcgata 2940
   cgattaacaa gggtatcacc ttcaaacttg acttcagcac gcgtcttgta gttcccgtca 3000
   tctttgaaag atatagtgcg ttcctgtaca taaccttcgg gcatggcact cttgaaaaag   3060
   tcatgccgtt tcatatgatc cggataacgg gaaaagcatt gaacaccata agagaaagta   3120
   gtgacaagtg ttggccatgg aacaggtagt tttccagtag tgcaaataaa tttaagggta   3180
   agctttccgt atgtagcatc accttcaccc tctccactga cagaaaattt gtgcccatta   3240
   acatcaccat ctaattcaac aagaattggg acaactccag tgaaaagttc ttctcctttg   3300
   ctagccattc tagagaattc cgcacttttc ggccaatggt cttggtaatt cctttgcgct   3360
   agaattgaac tcaggtacaa tcacttcttc tgaatgagat ttagtcatta tagttttttc   3420
   tccttgacgt taaagtatag aggtatatta acaatttttt gttgatactt ttattacatt   3480
   tgaataagaa gtaatacaaa ccgaaaatgt tgaaagtatt agttaaagtg gttatgcagt   3540
   ttttgcattt atatatctgt taatagatca aaaatcatcg cttcgctgat taattacccc   3600
   agaaataagg ctaaaaaact aatcgcatta tcatccctcg acgtactgta catataacca   3660
   ctggttttat atacagcagt actgtacata taaccactgg ttttatatac agcagtcgac   3720
   gtactgtaca tataaccact ggttttatat acagcagtac tgtacatata accactggtt   3780
   ttatatacag cagtcgaggt aagattagat atggatatgt atatggatat gtatatggtg   3840
   gtaatgccat gtaatatgat tattaaactt ctttgcgtcc atccaaaaaa aaagtaagaa   3900
   tttttgaaaa ttcaatataa atgacagctc agttacaaag tgaaagtact tctaaaattg   3960
   ttttggttac aggtggtgct ggatacattg gttcacacac tgtggtagag ctaattgaga   4020
   atggatatga ctgtgttgtt gctgataacc tgtcgaattc agatccccga cctgaagtct   4080
   aggtccctat ttattttttt atagttatgt tagtattaag aacgttattt atatttcaaa   4140
   tttttctttt ttttctgtac agacgcgtgt acgaatttcg acctcgaccg ggtaccgagc   4200
   tcggatcccc ctaagaaacc attattatca tgacattaac ctataaaaat aggcgtatca   4260
   cgaggccctt tcgtctcgcg cgtttcggtg atgacggtga aaacctctga cacatgcagc   4320
   tcccggagac ggtcacagct tgtctgtaag cggatgccgg gagcagacaa gcccgtcagg   4380
   gcgcgtcagc gggtgttggc gggtgtcggg gctggcttaa ctatgcggca tcagagcaga   4440
   ttgtactgag agtgcaccat aacgcattta agcataaaca cgcactatgc cgttcttctc   4500
   atgtatatat atatacaggc aacacgcaga tataggtgcg acgtgaacag tgagctgtat   4560
   gtgcgcagct cgcgttgcat tttcggaagc gctcgttttc ggaaacgctt tgaagttcct 4620
   attccgaagt tcctattctc tagctagaaa gtataggaac ttcagagcgc ttttgaaaac 4680
   caaaagcgct ctgaagacgc actttcaaaa aaccaaaaac gcaccggact gtaacgagct 4740
   actaaaatat tgcgaatacc gcttccacaa acattgctca aaagtatctc tttgctatat 4800
   atctctgtgc tatatcccta tataacctac ccatccacct ttcgctcctt gaacttgcat 4860
   ctaaactcga cctctacatt ttttatgttt atctctagta ttactcttta gacaaaaaaa 4920
   ttgtagtaag aactattcat agagtgaatc gaaaacaata cgaaaatgta aacatttcct 4980
   atacgtagta tatagagaca aaatagaaga aaccgttcat aattttctga ccaatgaaga 5040
   atcatcaacg ctatcacttt ctgttcacaa agtatgcgca atccacatcg gtatagaata 5100
   taatcgggga tgcctttatc ttgaaaaaat gcacccgcag cttcgctagt aatcagtaaa 5160
   cgcgggaagt ggagtcaggc ttttttatgg gaagagaaaa tagacaccaa agtagccttc 5220
   ttctaacctt aacggaccta cagtgcaaaa agttatcaag agactgcatt atagagcgca 5280
   caaaggagaa aaaaagtaat ctaagatgct ttgttagaaa aatagcgctc tcgggatgca 5340
   tttttgtaga acaaaaaaga agtatagatt ctttgttggt aaaatagcgc tctcgcgttg 5400
   catttctgtt ctgtaaaaat gcagctcaga ttctttgttt gaaaaattag cgctctcgcg 5460
   ttgcattttt gttttacaaa aatgaagcac agattcttcg ttggtaaaat agcgctttcg 5520
   cgttgcattt ctgttctgta aaaatgcagc tcagattctt tgtttgaaaa attagcgctc 5580
   tcgcgttgca tttttgttct acaaaatgaa gcacagatgc ttcgttagct tgggacggat 5640
   tacaacaggt attgtcctct gaggacataa aatacacacc gagattcatc aactcattgc 5700
   tggagttagc atatctacaa ttcagaagaa ctcgtcaaga aggcgataga aggcgatgcg 5760
   ctgcgaatcg ggagcggcga taccgtaaag cacgaggaag cggtcagccc attcgccgcc 5820
   aagctcttca gcaatatcac gggtagccaa cgctatgtcc tgatagcggt ccgccacacc 5880
   cagccggcca cagtcgatga atccagaaaa gcggccattt tccaccatga tattcggcaa 5940
   gcaggcatcg ccatgggtca cgacgagatc ctcgccgtcg ggcatgctcg ccttgagcct 6000
   ggcgaacagt tcggctggcg cgagcccctg atgctcttcg tccagatcat cctgatcgac 6060
   aagaccggct tccatccgag tacgtgctcg ctcgatgcga tgtttcgctt ggtggtcgaa 6120
   tgggcaggta gccggatcaa gcgtatgcag ccgccgcatt gcatcagcca tgatggatac 6180
   tttctcggca ggagcaaggt gagatgacag gagatcctgc cccggcactt cgcccaatag 6240
   cagccagtcc cttcccgctt cagtgacaac gtcgagcaca gctgcgcaag gaacgcccgt 6300
   cgtggccagc cacgatagcc gcgctgcctc gtcttgcagt tcattcaggg caccggacag 6360
   gtcggtcttg acaaaaagaa ccgggcgccc ctgcgctgac agccggaaca cggcggcatc 6420
   agagcagccg attgtctgtt gtgcccagtc atagccgaat agcctctcca cccaagcggc 6480
   cggagaacct gcgtgcaatc catcttgttc aatcatgcga aacgatcctc atcctgtctc 6540
   ttgatcagag cttgatcccc tgcgccatca gatccttggc ggcgagaaag ccatccagtt 6600
   tactttgcag ggcttcccaa ccttaccaga gggcgcccca gctggcaatt ccggttcgct 6660
   tgctgtccat aaaaccgccc agtctagcta tcgccatgta agcccactgc aagctacctg 6720
   ctttctcttt gcgcttgcgt tttcccttgt ccagatagcc cagtagctga cattcatccg 6780
   gggtcagcac cgtttctgcg gactggcttt ctacgtgaaa aggatctagg tgaagatcct 6840
   ttttgataat ctcatgacca aaatccctta acgtgagttt tcgttccact gagcgtcaga 6900
   ccccgtagaa aagatcaaag gatcttcttg agatcctttt tttctgcgcg taatctgctg 6960
   cttgcaaaca aaaaaaccac cgctaccagc ggtggtttgt ttgccggatc aagagctacc 7020
   aactcttttt ccgaaggtaa ctggcttcag cagagcgcag ataccaaata ctgtccttct 7080
   agtgtagccg tagttaggcc accacttcaa gaactctgta gcaccgccta catacctcgc 7140
   tctgctaatc ctgttaccag tggctgctgc cagtggcgat aagtcgtgtc ttaccgggtt 7200
   ggactcaaga cgatagttac cggataaggc gcagcggtcg ggctgaacgg ggggttcgtg 7260
   cacacagccc agcttggagc gaacgaccta caccgaactg agatacctac agcgtgagct 7320
   atgagaaagc gccacgcttc ccgaagggag aaaggcggac aggtatccgg taagcggcag 7380
   ggtcggaaca ggagagcgca cgagggagct tccaggggga aacgcctggt atctttatag 7440
   tcctgtcggg tttcgccacc tctgacttga gcgtcgattt ttgtgatgct cgtcaggggg 7500
   gcggagccta tggaaaaacg ccagcaacgc ggccttttta cggttcctgg gcttttgctg 7560
   gccttttgct cacatgatat aattcaattg aagctctaat ttgtgagttt agtatacatg 7620
   catttactta taatacagtt ttttagtttt gctggccgca tcttctcaaa tatgcttccc 7680
   agcctgcttt tctgtaacgt tcaccctcta ccttagcatc ccttcccttt gcaaatagtc 7740
   ctcttccaac aataataatg tcagatcctg tagagaccga attcattcga caggttatca   7800
   gcaacaacac agtcatatcc attctcaatt agctctacca cagtgtgtga accaatgtat   7860
   ccagcaccac ctgtaaccaa aacaatttta gaagtacttt cactttgtaa ctgagctgtc   7920
   atttatattg aattttcaaa aattcttact ttttttttgg atggacgcaa agaagtttaa   7980
   taatcatatt acatggcatt accaccatat acatatccat atacatatcc atatctaatc   8040
   ttacctcgag cattatcacc gccagaggta aaatagtcaa cacgcacggt gttagatatt   8100
   tatcccttgc ggtgatagct cgagggatga taatgcgatt agttttttag ccttatttct   8160
   ggggtaatta atcagcgaag cgatgatttt tgatctatta acagatatat aaatgcaaaa   8220
   actgcataac cactttaact aatactttca acattttcgg tttgtattac ttcttattca   8280
   aatgtaataa aagtatcaac aaaaaattgt taatatacct ctatacttta acgtcaagga   8340
   gaaaaaacta taatggaatt ctgcagccga tgaggaaacc cgatgaccac cacactgttg   8400
   cccggcactg tcaccctcgt cggcgccggg cccggcgacc ctgaactcgt caccgtggcc   8460
   ggcctgcggg ccgtgcagca ggccgaggtg atcctctacg accggctcgc cccgcaggac   8520
   ctgctgtcgg aggcgtccga cgacgccgaa ctcgtgccgg tcggcaagat cccgcgcggc   8580
   cactatgtgc cccaggagga gatcaaccaa ctgctcgtcg cgcacgcccg cgagggccgc   8640
   aaggtggtgc gcctcaaggg tggcgactcg ttcgtcttcg ggcgtggcgg cgaggaatgg   8700
   caggcctgcg ccgaggccgg catcccggtg cgcgtgatcc cgggagtctc ctcggccacc   8760
   gcgggcccgg cgctggccgg catcccgctg acccatcgcc acctggtgca ggggttcacc   8820
   gtcgtgtcgg ggcatgtatc gcccagcgac gagcgctccg aggtgccatg gcgccaactc   8880
   gccaaggacc ggctcacgct ggtgatcctg atgggcgtgg cccatatgcg cgacatcgcg   8940
   ccggaattga tggccggcgg gctgcctgcc gacacccccg tgcgcgtggt gagcaatgcg   9000
   agcctggcca gccaggaatc gtggcgcacc acgctgggcg atgccgtggc cgacatggac   9060
   gcgcaccacg tgcgtccgcc cgcgctggtg gtggtgggta ccctggccgg cgtcgacctg   9120
   tcgcatcccg accatcgcgc gcccagcgac cactgagtcg cggccgc               9167